# EUROPEAN PATENT APPLICATION

(11) **EP 2 975 046 A1**
(43) Date of publication of application: **20.01.2016**
(21) Application number: 14177268.1
(22) Date of filing: 16.07.2014
(51) Int. Cl.: C07K 5/02, A61K 38/00

(54) **Novel compounds**

(71) Applicant: The Provost, Fellows, Foundation Scholars, and The Other Members of Board, of The College of The Holy and Undivided Trinity of Queen Elizabeth, Dublin 2 (IE); InvivoGen SAS, 31400 Toulouse (FR)
(72) Inventor: Mills, Kingston, Dublin, Dublin 6 (IE); Kelly, Patrick, Dublin, Dublin 6 (IE); Tiraby, Jean-Gerard, 31400 Toulouse (FR); Lioux, Thierry, 31130 Balma (FR); Perouzel, Eric, 31500 Toulouse (FR)
(74) Representative: Pezzoli, Anna

(57) **Abstract**

A compound of formula (I) or a pharmaceutically acceptable derivative thereof, 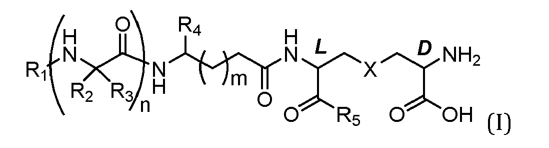 wherein R₁,R₂, R₃, R₄, R₅, X, m and n are defined in the specification; a process for preparing such compounds; a pharmaceutical composition comprising such compounds; and the use of such compounds in medicine.

## Description

### FIELD OF INVENTION

The present invention relates to novel NOD modulators, particularly to NOD1 or NOD1 and NOD2 agonists, processes for their preparation, pharmaceutical compositions comprising these modulators and their uses in the treatment of conditions for which agonism of NOD receptors is beneficial including inflammatory and/or autoimmune diseases, infectious or cancer diseases.

### BACKGROUND OF THE INVENTION

NOD1 and NOD2 are two members of the growing family of Nod-like receptors characterized by a nucleotide-oligomerization domain (NOD) and ligand-recognizing leucine-rich repeats. NOD1 and NOD2 are involved in the recognition of peptidoglycan (PGN), major surface component of Gram-positive bacteria. The innate immune receptors recognize specific molecules that are commonly found in microbes and induce host defense responses to eliminate invading pathogens (Creagh and O'Neill, 2006). These pathogen-recognizing receptors include membrane-bound Toll-like receptors (TLRs) as well as cytosolic NOD-like receptors and RIG-I family proteins (Inohara et al., 2005). These three classes of pathogen-recognizing receptors regulate innate and acquired immune responses by inducing intracellular signaling pathways that lead to the activation of p38, c-Jun NH2-terminal kinase (JNK), extracellular signal-regulated kinases (ERKs), caspase-1, and transcription factors including NF-κB and/or IRFs (Creagh and O'Neill, 2006). NOD1 is the founding member of the NOD-like receptor protein family that contains nucleotide oligomerization domain (NOD) and ligand-recognizing leucine-rich repeats. This family is comprised of more than 20 members including NOD2, cryopyrin, Ipaf, NAIP, and NALP1 (Creagh and O'Neill, 2006;(Inohara et al., 2005). Genetic studies have revealed that variations of the *NOD1* gene are associated with susceptibility to several human disorders including allergic diseases (Eder et al., 2006; Hysi et al., 2005; Weidinger et al., 2005), Crohn's disease (McGovern et al., 2005), and sarcoidosis (Tanabe et al., 2006), although the mechanism underlying these disease associations remains poorly understood. Previous studies suggested the involvement of NOD1 in the recognition of numerous bacteria including *Helicobacter pylori* (Boughan et al., 2006; Viala et al., 2004), *Listeria monocytogenes* (Opitz et al., 2006; Park et al., 2007), *Shigella flexneri* (Girardin et al., 2001), several *Bacillus* species (Hasegawa et al., 2006), and *Propionibacterium acnes* (Tanabe et al., 2006). NOD1 was expressed in multiple tissues and is thought to have an important role, particularly, in epithelium (Inohara et al., 1999). It has been suggested that NOD1 plays a role in intestinal immunity by regulating innate immune responses of epithelial cells infected with invasive enterobacteria and *Helicobacter pylori* (Kim et al., 2004; Viala et al., 2004).

Several NOD1 polymorphisms are linked to the development of inflammatory bowel disease, asthma and atopic eczema (Huebner et al., 2009; Hysi et al., 2005; McGovern et al., 2005; Weidinger et al., 2005). The NOD1 agonists are potentially useful as vaccine adjuvants and to prevent, inhibit or treat a variety of disorders including bowel diseases, asthma and cancer.

US patent application 2006/0194740 A1 and 2009/0028795 A1 provide compositions and methods for treating tumors that involve increasing the expression of NOD1 and/or the activity of NOD1. US patent 7244557 B2 provides a method fro screening modulators of NOD1 signaling. US patent 7396812 B2 provides a method for modulating NOD1 activity, use of a MTP related molecule for modulating NOD1 activity, and therapeutic applications thereof.

There is evidence that NOD1 mediates host recognition of bacteria or bacterial fractions containing soluble PGN-related molecules with the essential iE-DAP dipeptide (Girardin et al., 2001; Inohara et al., 2001). NOD1 senses the essential iE-DAP dipeptide, which is uniquely found in PGN of all Gram-negative and certain Gram-positive bacteria (Chamaillard et al., 2003; Girardin et al., 2003a; Girardin et al., 2003b; Girardin et al., 2003c; Inohara et al., 2003), whereas NOD2 recognizes the muramyl dipeptide (MDP) structure in bacterial peptidoglycan (PGN)-related molecules. Although the iE-DAP structure is found in the insoluble fraction of intact PGN, intermediates of PGN synthesis and cleaved PGN products produced during bacterial growth and PGN recycling (Girardin et al., 2003b; Holtje, 1998), the identity of the major NOD1 stimulatory molecule (s) remains unknown (18). Previous studies have shown that several iE-DAP-containing molecules including iE-DAP, D-Ala-γ-Glu-*meso*-DAP (A-iEDAP), and G]cNAc-anhMurNAc-D-Ala-γ-Glu-meso-DAP, but not intact macromolecular PGN, stimulate NOD1 (Chamaillard et al., 2003).

WO2010/072797 discloses that the NOD1 agonist analogs can have immunosuppressive activity and can act to selectively suppress Th1 and Th17 (IL-17 producing T cell) T cell mediated inflammatory autoimmune disease. It has been identified that the in-vivo administration of TriDAP involves an anti-inflammatory effect.

US patent 4,401,658 discloses tri-tetra or penta pepdide, comprising L-Ala-D-Glu residues and their use as vaccine adjuvants and immunostimulants. Particularly, they are useful to increase the hypersensitivity reactions and/or the production of circulating antibodies against the antigens with which they are administered, and they can stimulate, defence reactions against certain infections.

In light of the role NODs plays in the pathogenesis of diseases, it is desirable to prepare compounds that modulate NODs activity and hence have utility in the treatment of diseases mediated by NODs such as inflammatory and/or autoimmune diseases,infectious and cancer diseases.

### SUMMARY OF THE INVENTION

The present invention provides novel NOD modulators, specifically NOD1 or NOD1 and NOD2 agonists, processes for their preparation, pharmaceutical compositions comprising these modulators and their uses in the treatment of conditions for which agonism of NOD receptors is beneficial including inflammatory and/or autoimmune diseases, infectious and cancer diseases.

More specifically, the present invention is directed to compounds of formula (I) or a pharmaceutically acceptable salt thereof 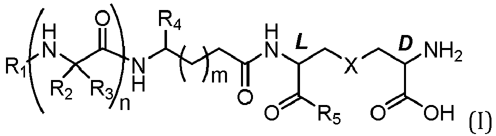 wherein
- R₁ is hydrogen, C₁-C₂₀ alkyl, C₂-C₂₀ alkenyl or C₂-C₂₀ alkynyl each of which groups are optionally substituted by one or more of C₁-C₄ alkyamino, C₁-C₄ alkoxy or R₁ is (C₁-C₂₀ alkyl)-W-(C₂-C₂₀ alkyenyl)-, wherein W is -O-, -S-, -NH-, -C(O)- or -S(O)₂-, or R₁ is a group (II)

   -Z-R₆ (II)

   in which Z is -C(O)-,-C(S)- or -S(O)₂-, R₆ is C₁-C₂₀ alkyl, C₂-C₂₀ alkenyl; C₂-C₂₀ alkynyl, C₃-C₇ cycloalkyl, phenyl or benzyl each of which may be optionally substituted by one or more of halogen, C₁-C₄ alkyl or C₁-C₄ alkoxy; or R₁ is a Muramic acid derivative of formula (III): 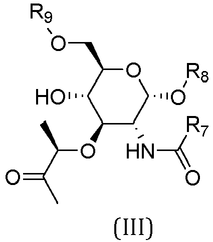 in which:
   - **R₇** is C₁-C₄ alkyl optionally substituted by hydroxyl;
   - **R₈** is hydrogen or benzyl group;
   - **R₉** is hydrogen, C₁-C₂₀ alkyl, C₁-C₂₀ alkenyl, C₁-C₂₀ alkynyl each of which may be optionally substituted by C₁-C₄ alkyamino or C₁-C₄ alkoxy; or R₉ is a group :

      -Z-R₁₀ (IV)

      in which **Z** is is -C(O)-,-C(S)- or -S(O)₂-, **R₁₀** is a group selected from C₁-C₂₀ alkyl, C₂-C₂₀ alkenyl; C₂-C₂₀ alkynyl, C₃-C₇ cycloalkyl, phenyl or benzyl; each of which may be optionally substituted by one or more of hydroxy, halogen, C₁-C₄ alkyl or C₁-C₄ alkoxy;
- **R₂ and R₃** are hydrogen or are independently selected from hydrogen, benzyl which may be optionally substituted by hydroxy or C₁-C₆ alkyl group which may be optionally substituted by one or more of hydroxy, carboxy, amino, -C(O)NH₂, -SH, -SCH₃,-NHC(=NH)NH₂ or of a heterocyclyl group;
- **R₄** is hydrogen, hydroxyl, amino, -COOR₁₁, or -CONR₁₁R₁₂ wherein R₁₁ and R₁₂ are hydrogen or are independently selected from hydrogen, or C₁-C₂₀ alkyl optionally substituted with a hydroxyl or amino group;
- **R₅** is hydroxy, amino, -O-C₁-C₂₀ alkyl, -NH-C₁-C₂₀ alkyl, optionally substituted by hydroxyl or R₅ is a group of formula (V), 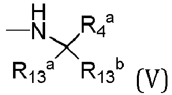 wherein
   o **R₁₃^{a}** and **R₁₃^{b}** are hydrogen or are independently selected from hydrogen, benzyl which may be optionally substituted by hydroxy or C₁-C₆ alkyl group which may be optionally substituted by one or more of hydroxy, carboxy, amino, -C(O)NH₂, -SH, -SCH₃,-NHC(=NH)NH₂ or of a heterocyclyl group;
   o **R₄^{a}** is are hydrogen, hydroxyl, amino, -COOR₁₁, or -CONR₁₁R₁₂ wherein R₁₁ and R₁₂ are hydrogen or are independently selected from hydrogen, or C₁-C₂₀ alkyl optionally substituted with hydroxyl or amino group;
- **m** is an integer from 1 to 3;
- **n** is an integer from 0 to 2;
- **X** is sulfur, oxygen or -NH-;
- **L or D** represents the absolute configuration of the chiral carbon atom;
with the proviso that when X is sulfur, m and n are 1, R₂ is hydrogen, R₃ is methyl, R₄ is -COOH or -CONH2 and R₅ is hydroxy, R₁ is not a dodecanoyl group.

In one aspect, the present invention provides a pharmaceutical composition comprising a) a compound of formula (I) or a pharmaceutically acceptable salt thereof and b) one or more pharmaceutically acceptable exicipents.

In a further aspect, the present invention provides a compound of formula (I), or a pharmaceutically acceptable salt thereof, for use in therapy.

Compounds of formula (I) and pharmaceutically acceptable salts thereof, are modulators of NOD, particularly they are agonists of NOD1 or NOD1 and NOD2 and can be useful in the treatment of inflammatory and/or autoimmune diseases mediated by NOD receptors.

Compounds of Formula (I) and pharmaceutically acceptable salts thereof can be useful in the treatment of a variety of cancers and tumors.

Furthermore the compounds of Formula (I) and pharmaceutically acceptable salts thereof can be useful in the treatment of infectious diseases particularly in preventing or treating Gram-negative bacteria infection.

In a further aspect, the present invention provides a compound of formula (I), or a pharmaceutically acceptable salt thereof, for use in treatment of conditions for which agonism of NOD1 or NODland NOD2 receptors is beneficial.

In a further aspect, the present invention provides a compound of formula (I), or a pharmaceutically acceptable salt thereof, for use in treatment of inflammatory and or autoimmune diseases, cancers and tumors and infectious diseases.

In yet a further aspect, the present invention is directed to method of treating inflammatory and/or autoimmune diseases, cancers and tumors and infectious diseases, which comprises administering to a subject in need thereof a therapeutically amount of a compound of formula (I) or a pharmaceutically acceptable salt thereof.

### BRIEF DESCRIPTION OF THE DRAWINGS

**Figure 1A** shows the concentrations of IL-10 produced by PEC after 24 h stimulation with the compounds of Example 29 (Ex 29) or of Example 15 (Ex 15).
**Figure 1B** shows absolute cell numbers of LPMs in the peritoneal cavity after injection of PBS, compounds of Example 29 (Ex 29) or of Example 15 (Ex 15).
**Figure 1C** shows Experimental Autoimmune Encephalomyelitis (EAE) clinical scores and percentage weight loss.
**Figure 2** shows the role of IL-10 in protection against EAE induced by NOD1 activation.
**Figure 3** shows the treatment of mice with the compound of Example 29 (Ex 29) protects mice against DSS-induced colitis.
**Figure 4** shows the treatment of mice with the compound of Example 29 (Ex 29) protects mice against DSS-induced inflammation in the colon.

### DETAILED DESCRIPTION OF THE INVENTION

In a first apect, the present invention is directed to compounds of formula (I) or a pharmaceutically acceptable salt thereof 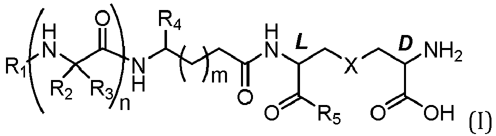 wherein
- **R₁** is hydrogen, C₁-C₂₀ alkyl, C₂-C₂₀ alkenyl or C₂-C₂₀ alkynyl each of which groups are optionally substituted by one or more of C₁-C₄ alkyamino, C₁-C₄ alkoxy or R₁ is (C₁-C₂₀ alkyl)-W-(C₂-C₂₀ alkyenyl)-, wherein W is -O-, -S-, -NH-, -C(O)- or -S(O)₂-, or R₁ is a group (II)

   -Z-R₆ (II)

   in which Z is -C(O)-,-C(S)- or -S(O)₂-, R₆ is C₁-C₂₀ alkyl, C₂-C₂₀ alkenyl; C₂-C₂₀ alkynyl, C₃-C₇ cycloalkyl, phenyl or benzyl each of which may be optionally substituted by one or more of halogen, C₁-C₄ alkyl or C₁-C₄ alkoxy; or R₁ is a Muramic acid derivative of formula (III): 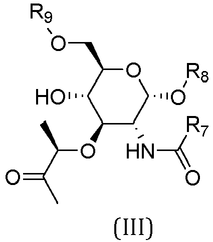 in which:
   - **R₇** is C₁-C₄ alkyl optionally substituted by hydroxyl;
   - **R₈** is hydrogen or benzyl group;
   - **R₉** is hydrogen, C₁-C₂₀ alkyl, C₁-C₂₀ alkenyl, C₁-C₂₀ alkynyl each of which may be optionally substituted by C₁-C₄ alkyamino or C₁-C₄ alkoxy; or R₉ is a group :

      -Z-R₁₀ (IV)

      in which **Z** is is -C(O)-,-C(S)- or -S(O)₂-, **R₁₀** is a group selected from C₁-C₂₀ alkyl, C₂-C₂₀ alkenyl; C₂-C₂₀ alkynyl, C₃-C₇ cycloalkyl, phenyl or benzyl; each of which may be optionally substituted by one or more of hydroxy, halogen, C₁-C₄ alkyl or C₁-C₄ alkoxy;
- **R₂ and R₃** are hydrogen or are independently selected from hydrogen, benzyl which may be optionally substituted by hydroxy or C₁-C₆ alkyl group which may be optionally substituted by one or more of hydroxy, carboxy, amino, -C(O)NH₂, -SH, -SCH₃,-NHC(=NH)NH₂ or of a heterocyclyl group;
- **R₄** is hydrogen, hydroxyl, amino, -COOR₁₁, or -CONR₁₁R₁₂ wherein R₁₁ and R₁₂ are hydrogen or are independently selected from hydrogen, or C₁-C₂₀ alkyl optionally substituted with hydroxyl or amino group;
- **R₅** is hydroxy, amino, -O-C₁-C₂₀ alkyl, -NH-C₁-C₂₀ alkyl, optionally substituted by hydroxyl or R₅ is a group of formula (V), 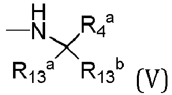 wherein
   o **R₁₃^{a} and R₁₃^{b}** are hydrogen or are independently selected from hydrogen, benzyl which may be optionally substituted by hydroxy or C₁-C₆ alkyl group which may be optionally substituted by one or more of hydroxy, carboxy, amino, -C(O)NH₂, -SH, -SCH₃,-NHC(=NH)NH₂ or of a heterocyclyl group;
   o **R₄^{a}** is are hydrogen, hydroxyl, amino, -COOR₁₁, or -CONR₁₁R₁₂ wherein R₁₁ and R₁₂ are hydrogen or are independently selected from hydrogen, or C₁-C₂₀ alkyl optionally substituted with hydroxyl or amino group;
- **m** is an integer from 1 to 3;
- **n** is an integer from 0 to 2;
- **X** is sulfur, oxygen or -NH-;
- **L or D** represents the absolute configuration of the chiral carbon atom;
with the proviso that when X is sulfur, m and n are 1, R₂ is hydrogen, R₃ is methyl, R₄ is -COOH or -CONH2 and R₅ is hydroxy, R₁ is not a dodecanoyl group.

Compounds according to formula (I) contain a basic functional group and are therefore capable of forming pharmaceutically acceptable acid addition salts by treatment with a suitable acid.

Suitable acids include pharmaceutically acceptable inorganic acids and pharmaceutically acceptable organic acids.

Representative pharmaceutically acceptable acid addition salts include hydrochloride, hydrobromide, nitrate, methylnitrate, sulfate, bisulfate, sulfamate, phosphate, acetate, hydroxyacetate, phenylacetate, propionate, butyrate, isobutyrate, valerate, maleate, hydroxymaleate, acrylate, fumarate, malate, tartrate, citrate, salicylate, p-aminosalicyclate, glycollate, lactate, heptanoate, phthalate, oxalate, succinate, benzoate, o-acetoxybenzoate, chlorobenzoate, methylbenzoate, dinitrobenzoate, hydroxybenzoate, methoxybenzoate, naphthoate, hydroxynaphthoate, mandelate, tannate, formate, stearate, ascorbate, palmitate, oleate, pyruvate, pamoate, malonate, laurate, glutarate, glutamate, estolate, methanesulfonate (mesylate), ethanesulfonate (esylate), 2- hydroxyethanesulfonate, benzenesulfonate (besylate), p-aminobenzenesulfonate, p- toluenesulfonate (tosylate), and napthalene-2-sulfonate.

In certain embodiments, compounds according to formula (I) may contain an acidic functional group. Suitable pharmaceutically acceptable salts include salts of such acidic functional groups.

Representative salts include pharmaceutically acceptable metal salts such as sodium, potassium, lithium, calcium, magnesium, aluminum, and zinc salts; carbonates and bicarbonates of a pharmaceutically acceptable metal cation such as sodium, potassium, lithium, calcium, magnesium, aluminum, and zinc; pharmaceutically acceptable organic primary, secondary, and tertiary amines including aliphatic amines, aromatic amines, aliphatic diamines, and hydroxy alkylamines such as methylamine, ethylamine, 2-hydroxyethylamine, diethylamine, Triethylamine" ethylenediamine, ethanolamine, diethanolamine, and cyclohexylamine.

For reviews on suitable pharmaceutical salts see Berge et al, J. Pharm, Sci., 66, 1-19, 1977; P L Gould, International Journal of Pharmaceutics, 33 (1986), 201-217; and Bighley et al, Encyclopaedia of Pharmaceutical Technology, Marcel Dekker Inc, New York 1996, Volume 13, page 453-497.

Other salts that are not deemed pharmaceutically acceptable may be useful in the preparation of compounds of formula (I) and are included within the scope of the invention, such as those formed with ammonia and trifluoroacetic acid.

The present invention encompasses all possible stoichiometric and non-stoichiometric forms of the salts of the compounds of formula (I).

These pharmaceutically acceptable salts may be prepared in situ during the final isolation and purification of the compound, or by separately reacting the purified compound in its free acid or free base form with a suitable base or acid, respectively

The person skilled in the art will appreciate that many organic compounds can form complexes with solvents in which they are reacted or from which they are precipitated or crystallised. These complexes are known as "solvates". Where the solvent is water the complex is known as a "hydrate".

The present invention encompasses all solvates of the compounds of formula (I). In addition, prodrugs are also included within the context of this invention.

Prodrugs are any covalently bonded carriers that release a compound of formula (I) in vivo when such prodrug is administered to a patient. Prodrugs are generally prepared by modifying functional groups in a way such that the modification is cleaved, either by routine manipulation or in vivo, yielding the parent compound. Prodrugs include, for example, compounds of this invention wherein hydroxy, amine or sulfhydryl groups are bonded to any group that, when administered to a patient, cleaves to form the hydroxy, amine or sulfhydryl groups. Thus, representative examples of prodrugs include (but are not limited to) acetate, formate and benzoate derivatives of alcohol, sulfhydryl and amine functional groups of the compounds of formula (I). Further, in the case of a carboxylic acid (-COOH), esters may be employed, such as methyl esters, ethyl esters, and the like. Esters may be active in their own right and /or be hydrolysable under in vivo conditions in the human body. Suitable pharmaceutically acceptable in vivo hydrolysable ester groups include those which break down readily in the human body to leave the parent acid or its salt.

The compounds of formula (I) and pharmaceutically acceptable salts thereof contain at least three asymmetric centers (also referred to as a chiral center) and may, therefore, exist as individual enantiomers, diastereomers, or other stereoisomeric forms, or as mixtures thereof.

Chiral centers, such as chiral carbon atoms, may also be present in a substituent such as an alkyl group. Where the stereochemistry of a chiral center present in a compound of formula (I), or in any chemical structure illustrated herein, is not specified the structure is intended to encompass all individual stereoisomers and all mixtures thereof. Thus, compounds of formula (I) and pharmaceutically acceptable salts thereof may be used as racemic mixtures, enantiomerically enriched mixtures, or as enantiomerically pure individual stereoisomers.

In one embodiment, n is 0.

In one embodiment, n is 0 and X is sulphur.

In one embodiment, n is 0 and m is 1 and X is sulphur.

In one embodiment, n is 1 and X is sulphur.

In one embodiment, n and m are 1 and X is sulphur.

In one embodiment, X is oxygen.

In one embodiment, n is 0 and m is 1, X is sulphur and R₅ is hydroxy, a D-alanyl or a glycyl residue.

In one embodiment, n and m are 1, X is sulphur and R₅ is hydroxy, a D-alanyl or a glycyl residue.

In one embodiment, m is 1, X is Oxygen and R₅ is hydroxyl, a D-alanyl or a glycyl residue.

In one embodiment, the present invention provides a subset of compounds of formula (I), having formula (1a) or a pharmaceutically acceptable salt thereof, 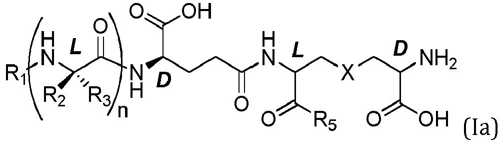 wherein
- **R₁** is hydrogen, an acyl group selected from a heptanoyl, dodecanoyl tetradecanoyl or 2-hydroxypropanoyl group or R₁ is a Muramic acid derivative of formula (III): 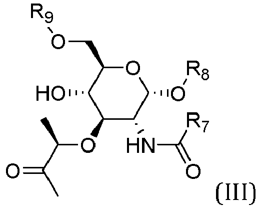 in which:
   - **R₇** is C₁-C₄ alkyl;
   - **R₈** is hydrogen or benzyl group;
   - **R₉** is hydrogen,
- **R₂ and R₃** are independently selected from hydrogen, benzyl which may be optionally substituted by hydroxy or C₁-C₆ alkyl group which may be optionally substituted by one or more of hydroxy, carboxy, amino, -C(O)NH₂, -SH, -SCH₃,-NHC(=NH)NH₂ or of a heterocyclyl group;
- **R₅** is hydroxyl, a D-alanyl or a glycyl residue;
- **n** is an integer from 0 or 1;
- **X** is sulphur or oxygen;
- **L or D** represents the absolute configuation of the chiral carbon atom;
   with the proviso that when X is sulfur, m and n are 1, R₂ is hydrogen, R₃ is methyl, and R₅ is hydroxyl, R₁ is not a dodecanoyl group.

In a further embodiment, the present invention provides a subset of compounds of formula (I), of formula (lb) or a pharmaceutically acceptable salt thereof, 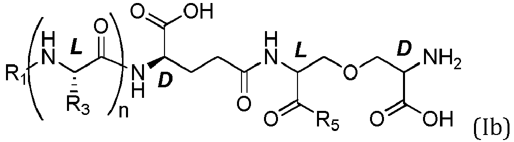 wherein
- R1 is hydrogen, an acyl group selected from a heptanoyl, dodecanoyl tetradecanoyl or-2-hydroxypropanoyl or R₁ is a Muramic acid derivative of formula (III) 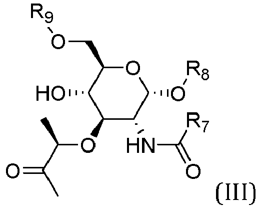 in which:
   - **R₇** is C₁-C₄ alkyl;
   - **R₈** is hydrogen or benzyl group;
   - **R₉** is hydrogen,
- **R₃** is hydrogen, benzyl which may be optionally substituted by hydroxy or C₁-C₆ alkyl group which may be optionally substituted by one or more of hydroxy, carboxy or amino;
- **R₅** is hydroxyl, D-alanyl or a glycyl residue;
- **n** is an integer from 0 or 1;
- **L or D** represents the absolute configuation of the chiral carbon atom.

In one embodiment, the present invention provides a subset of compounds of formula (I), of formula (lc) or a pharmaceutically acceptable salt thereof, 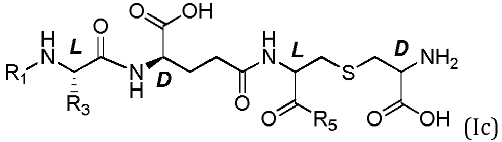 wherein
- **R₁** is hydrogen, an acyl group selected from a heptanoyl, dodecanoyl tetradecanoyl or-2-hydroxypropanoyl or R₁ is a Muramic acid derivative of formula (III): 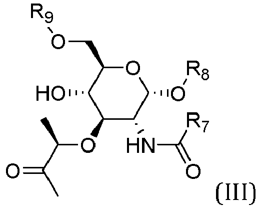 in which:
   - **R₇** is C₁-C₄ alkyl;
   - **R₈** is hydrogen or benzyl group;
   - **R₉** is hydrogen,
- **R₃** is hydrogen, benzyl which may be optionally substituted by hydroxy or C₁-C₆ alkyl group which may be optionally substituted by one or more of hydroxy, carboxy or amino;
- **R₅** is hydroxyl, a D-alanyl or a glycyl residue;
- **L or D** represents the absolute configuation of the chiral carbon atom;
   with the proviso that when R₃ is methyl and R₅ is hydroxy, R₁ is not a dodecanoyl group.

In one embodiment, the present invention provides a subset of compounds of formula (I), of formula (ld) or a pharmaceutically acceptable salt thereof, 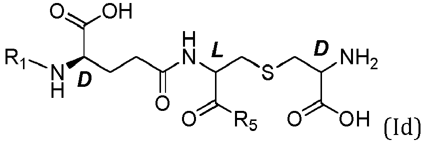 wherein
- **R₁** is hydrogen or an acyl group selected from a heptanoyl, dodecanoyl tetradecanoyl or -2-hydroxypropanoyl or R₁ is a Muramic acid derivative of formula (III): 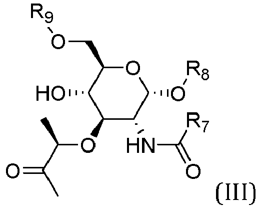 in which:
   - **R₇** is C₁-C₄ alkyl;
   - **R₈** is hydrogen or benzyl group;
   - **R₉** is hydrogen,
- **R₃** is hydrogen, benzyl which may be optionally substituted by hydroxy or C₁-C₆ alkyl group which may be optionally substituted by one or more of hydroxy, carboxy, or amino;
- **R₅** is hydroxy or D-alanyl or a glycyl residue;
- **L or D** represents the absolute configuation of the chiral carbon atom.

It is understood that the present invention covers all combinations of substituent groups referred to herein above.

In one embodiment, compounds of the invention include:
*N*⁵-((*S*)-2-(((*R*)-2-amino-2-carboxyethyl)thio)-1-carboxyethyl)-*D*-glutamine;
*N*⁵-((*S*)-2-(((*R*)-2-amino-2-carboxyethyl)thio)-1-carboxyethyl)-*N*²-heptanoyl-*D-*glutamine;
*N*⁵-((*S*)-2-(((*R*)-2-amino-2-carboxyethyl)thio)-1-carboxyethyl)-*N*²-dodecanoyl-*D-*glutamine;
*N*⁵-((*S*)-2-(((*R*)-2-amino-2-carboxyethyl)thio)-1-carboxyethyl)-*N*²-tetradecanoyl-*D-*glutamine;
*N*²-(*L*-alanyl)-*N*⁵-((*S*)-2-(((*R*)-2-amino-2-carboxyethyl)thio)-1-carboxyethyl)-*D-*glutamine;
*N*²-(*L*-leucyl)-*N*⁵-((*S*)-2-(((*R*)-2-amino-2-carboxyethyl)thio)-1-carboxyethyl)-*D-*glutamine;
*N*²-(*L*-alloisoleucyl)-*N*⁵-((*S*)-2-(((*R*)-2-amino-2-carboxyethyl)thio)-1-carboxyethyl)-D-glutamine;
*N*²-(*L*-valyl)-*N*⁵-((*S*)-2-(((*R*)-2-amino-2-carboxyethyl)thio)-1-carboxyethyl)-*D-*glutamine;
*N*²-(*L*-phenylalanyl)-*N*⁵-((*S*)-2-(((*R*)-2-amino-2-carboxyethyl)thio)-1-carboxyethyl)-*D*-glutamine;
*N*⁵-((*S*)-2-(((*R*)-2-amino-2-carboxyethyl)thio)-1-carboxyethyl)-*N*²-(tetradecanoyl-*L-*leucyl)-*D*-glutamine;
*N*⁵-((*S*)-2-(((*R*)-2-amino-2-carboxyethyl)thio)-1-carboxyethyl)-*N*²-(tetradecanoyl-*L-*alloisoleucyl)-*D*-glutamine;
*N*⁵-((*S*)-2-(((*R*)-2-amino-2-carboxyethyl)thio)-1-carboxyethyl)-*N*²-(tetradecanoyl-*L-*valyl)-*D*-glutamine;
*N⁵*-((*S*)-2-(((*R*)-2-amino-2-carboxyethyl)thio)-1-carboxyethyl)-*N²*-(tetradecanoyl-*L-*phenylalanyl)-*D*-glutamine;
*N*⁵-((*S*)-3-(((*R*)-2-amino-2-carboxyethyl)thio)-1-(((*R*)-1-carboxyethyl)amino)-1-oxopropan-2-yl)-*D*-glutamine;
*N*⁵-((*S*)-3-(((*R*)-2-amino-2-carboxyethyl)thio)-1-(((*R*)-1-carboxyethyl)amino)-1-oxopropan-2-yl)-*N*²-heptanoyl-*D*-glutamine;
*N*⁵-((*S*)-3-(((*R*)-2-amino-2-carboxyethyl)thio)-1-(((*R*)-1-carboxyethyl)amino)-1-oxopropan-2-yl)-*N*²-dodecanoyl-*D*-glutamine;
*N*⁵-((*S*)-3-(((*R*)-2-amino-2-carboxyethyl)thio)-1-(((*R*)-1-carboxyethyl)amino)-1-oxopropan-2-yl)-*N*²-tetradecanoyl-*D*-glutamine;
*N*²-(*L-*alanyl)-*N*⁵-((*S*)-3-(((*R*)-2-amino-2-carboxyethyl)thio)-1-(((*R*)-1-carboxyethyl)amino)-1-oxopropan-2-yl)-*D*-glutamine;
*N*⁵-((*S*)-3-(((*R*)-2-amino-2-carboxyethyl)thio)-1-(((*R*)-1-carboxyethyl)amino)-1-oxopropan-2-yl)-*N*²-(heptanoyl-*L*-alanyl)-*D*-glutamine;
*N*⁵-((*S*)-3-(((*R*)-2-amino-2-carboxyethyl)thio)-1-(((*R*)-1-carboxyethyl)amino)-1-oxopropan-2-yl)-*N*²-(dodecanoyl-*L*-alanyl)-*D*-glutamine;
*N*⁵-((*S*)-3-(((*R*)-2-amino-2-carboxyethyl)thio)-1-(((*R*)-1-carboxyethyl)amino)-1-oxopropan-2-yl)-*N*²-(tetradecanoyl-*L*-alanyl)-*D*-glutamine;
*N*⁵-((*S*)-3-(((*R*)-2-amino-2-carboxyethyl)thio)-1-((carboxymethyl)amino)-1-oxopropan-2-yl)-*D*-glutamine;
*N*⁵-((*S*)-3-(((*R*)-2-amino-2-carboxyethyl)thio)-1-((carboxymethyl)amino)-1-oxopropan-2-yl)-*N*²-heptanoyl-*D*-glutamine;
*N*⁵-((*S*)-3-(((*R*)-2-amino-2-carboxyethyl)thio)-1-((carboxymethyl)amino)-1-oxopropan-2-yl)-*N*²-dodecanoyl-*D*-glutamine;
*N*⁵-((*S*)-3-(((*R*)-2-amino-2-carboxyethyl)thio)-1-((carboxymethyl)amino)-1-oxopropan-2-yl)-*N*²-tetradecanoyl-*D*-glutamine;
*N*²-(L-alanyl)-*N*⁵-((*S*)-3-(((*R*)-2-amino-2-carboxyethyl)thio)-1-((carboxymethyl)amino)-1-oxopropan-2-yl)-*D*-glutamine;
*N*⁵-((*S*)-3-(((*R*)-2-amino-2-carboxyethyl)thio)-1-((carboxymethyl)amino)-1-oxopropan-2-yl)-*N*²-(heptanoyl-*L*-alanyl)-*D*-glutamine;
*N*⁵-((*S*)-3-(((*R*)-2-amino-2-carboxyethyl)thio)-1-((carboxymethyl)amino)-1-oxopropan-2-yl)-*N*²-(dodecanoyl-*L*-alanyl)-*D*-glutamine;
*N*⁵-((S)-3-(((R)-2-amino-2-carboxyethyl)thio)-1-((carboxymethyl)amino)-1-oxopropan-2-yl)-*N*²-(tetradecanoyl-L-alanyl)-D-glutamine;
*N*⁵-((*S*)-3-(((*R*)-2-amino-2-carboxyethyl)thio)-1-((carboxymethyl)amino)-1-oxopropan-2-yl)-*N*²-(((*R*)-2-hydroxypropanoyl)-*L*-alanyl)-*D*-glutamine;
*N*²-(((*R*)-2-(((2*S*,3*R*,4*R*,5*S*,6*R*)-3-acetamido-2-(benzyloxy)-5-hydroxy-6-(hydroxymethyl)tetrahydro-2*H*-pyran-4-yl)oxy)propanoyl)-L-alanyl)-*N*⁵-((*S*)-2-(((*R*)-2-amino-2-carboxyethyl)thio)-1-carboxyethyl)-*D*-glutamine;
*N*²-(((*R*)-2-(((2*S*,3*R*,4*R*,5*S*,6*R*)-3-acetamido-2-(benzyloxy)-5-hydroxy-6-(hydroxymethyl)tetrahydro-2*H*-pyran-4-yl)oxy)propanoyl)-*L*-alanyl)-*N*⁵-((*S*)-3-(((R)-2-amino-2-carboxyethyl)thio)-1-((carboxymethyl)amino)-1-oxopropan-2-yl)-D-glutamine;
*N*₂-(((*R*)-2-(((2*S*,3*R*,4*R*,5*S*,6*R*)-3-acetamido-2-(benzyloxy)-5-hydroxy-6-(hydroxymethyl)tetrahydro-2H-pyran-4-yl)oxy)propanoyl)-L-alanyl)-*N*⁵-((*S*)-3-(((*R*)-2-amino-2-carboxyethyl)thio)-1-(((*R*)-1-carboxyethyl)amino)-1-oxopropan-2-yl)-D-glutamine;
*N*⁵-((*S*)-2-((*R*)-2-amino-2-carboxyethoxy)-1-carboxyethyl)-*D*-glutamine;
*N*⁵-((*S*)-2-((*R*)-2-amino-2-carboxyethoxy)-1-carboxyethyl)-*N*²-tetradecanoyl-*D-*glutamine;
*N*²-(*L*-alanyl)-*N*⁵-((*S*)-2-((*R*)-2-amino-2-carboxyethoxy)-1-carboxyethyl)-*D-*glutamine;
*N*⁵-((*S*)-2-((*R*)-2-amino-2-carboxyethoxy)-1-carboxyethyl)-*N*²-(tetradecanoyl-*L-*alanyl)-D-glutamine; or pharmaceutically acceptable salts thereof.

In one embodiment, compounds of the invention are
N⁵-((S)-3-(((R)-2-amino-2-carboxyethyl)thio)-1-(((R)-1-carboxyethyl)amino)-1-oxopropan-2-yl)-N²-heptanoyl-D-glutamine;
N⁵-((S)-3-(((R)-2-amino-2-carboxyethyl)thio)-1-((carboxymethyl)amino)-1-oxopropan-2-yl)-N²-(tetradecanoyl-L-alanyl)-D-glutamine or pharmaceutically acceptable salts thereof.

Included within the scope of the "compounds of the invention" are all salts, solvates, hydrates, prodrugs, radiolabelled derivatives, stereoisomers and optical isomers of the compounds of formula (I).

The invention also includes isotopically-labeled compounds, which are identical to those described herein, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, fluorine, iodine, and chlorine, such as ³H, ¹¹C, ¹⁴C, ¹⁸F, ¹²³I and ¹²⁵I. Compounds of the invention that contain the aforementioned isotopes and/or other isotopes of other atoms are within the scope of the present invention. Isotopically-labeled compounds of the present invention, for example those into which radioactive isotopes such as ³H, ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. ¹¹C and ⁸F isotopes are particularly useful in PET (positron emission tomography), and ¹²⁵I isotopes are particularly useful in SPECT (single photon emission computerized tomography), all useful in brain imaging. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances.

Isotopically labeled compounds of the invention can generally be prepared by carrying out the procedures disclosed in the Examples below, then substituting a readily available isotopically labeled reagent for a non-isotopically labeled reagent.

### DEFINITIONS

Throughout the present specification and the accompanying claims the words "comprise" and "include" and variations such as "comprises", "comprising", "includes" and "including" are to be interpreted inclusively. That is, these words are intended to convey the possible inclusion of other elements or integers not specifically recited, where the context allows.

A peptide is an amino acid polymer wherein each amino acid is linked to its neighbor by an amide bond -CO-NH-, also called peptide bond. The number of amino acids contained in one peptide can vary from 2 to about 100, though the latter is not set precisely. Typically, both extremities of the peptide backbone finish with an amine group on one side and a carboxylic acid group on the other side. The amine extremity is referred to as the "N-terminal extremity" and the carboxylic acid extremity is referred to as the "C-terminal extremity".

As used herein, the term "salt" refers to any salt of a compound according to the present invention prepared from an inorganic or organic acid or base, quaternary ammonium salts and internally formed salts.

As used herein, the term "pharmaceutically acceptable salts" refers to salts that retain the desired biological activity of the subject compound and exhibit minimal undesired toxicological effects.

As used herein, the term "prodrug" means a compound which is converted within the body, e.g. by hydrolysis in the blood, into its active form that has medical effects. Pharmaceutically acceptable prodrugs are described in T. Higuchi and V. Stella, Prodrugs as Novel Delivery Systems, Vol. 14 of the A.C.S. Symposium Series, Edward B. Roche, ed., Bioreversible Carriers in Drug Design, American Pharmaceutical Association and Pergamon Press, 1987, and in D. Fleisher, S. Ramon and H. Barbra "Improved oral drug delivery: solubility limitations overcome by the use of prodrugs", Advanced Drug Delivery Reviews (1996) 19(2) 115-130, each of which are incorporated herein by reference.

As used herein, the term "solvate" refers to a complex of variable stoichiometry formed by a solute (in this invention, a compound of formula (I) or a salt thereof) and a solvent. Such solvents for the purpose of the invention may not interfere with the biological activity of the solute. Examples of suitable solvents include, but are not limited to, water, methanol, ethanol and acetic acid. In one embodiment, the solvent used is a pharmaceutically acceptable solvent. Examples of suitable pharmaceutically acceptable solvents include water, ethanol and acetic acid. In one embodiment, the solvent used is water.

The term "residue" with reference to an amino acid or amino acid derivative means a radical derived from the corresponding α-amino acid by eliminating the hydroxyl of the carboxy group and/or one hydrogen of the α-amino group. For instance, the terms Ala, Gly, Ile, Glu, Phe, Ser, Leu, Cys, represent the "residues" of L-alanine, glycine, L-isoleucine, L-glutamic acid, L-phenylalanine, L-serine, L-leucine, and L-cysteine, respectively.

The term "side chain" with reference to an amino acid or amino acid residue means a group attached to the α-carbon atom of the α-amino acid. For example, the R-group side chain for glycine is hydrogen, for alanine it is methyl, for valine it is isopropyl. For the specific R-groups or side chains of the α-amino acids reference is made to A.L. Lehninger's text on Biochemistry (see chapter 4).

The term "chiral" refers to molecules which have the property of non-superimposability on their mirror image partner.

The term "chiral carbon atom" is a carbon atom which that have four different substituents

The lanthionine(VI) has two chiral centers showed in the structure as *and it can exists in 3 diastereoisomers 2 *threo* (LL and DD) and 1 *meso* (DL or LD).

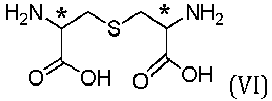

As used herein, the term "alkyl" (when used as a group or as part of a group) refers to a straight or branched hydrocarbon chain unless specified otherwise, containing the specified number of carbon atoms.

For example "C₁-C₂₀ alkyl" refers to an alkyl group, as defined above, containing at least 1, and at most 20 carbon.Example of such alkyl groups include but is not limited methyl, ethyl, propyl, *iso*-propyl, *n*-butyl, *iso*-butyl, *sec-*butyl, or *tert*-butyl, pentyl, hexyl, heptyl, octyl, nonyl, decyl, undecyl, docecyl, tridecyl, tetradecyl, pentadecyl, hexadecyl, heptadecyl, octadecyl, nonadecyl and eicosyl.

For example, "C₁-C₄ alkyl" refers to an alkyl group, as defined above, containing at least 1 and at most 4 carbon atoms Examples of such alkyl groups include methyl, ethyl, propyl, *iso*-propyl, *n*-butyl, *iso*-butyl, *sec-*butyl, or tert-butyl, , pentyl or hexyl.

The term "C₁-C₆ alkyl" refers to an alkyl group, as defined above, containing at least 1 and at most 6 carbon atoms Examples of such alkyl groups include methyl, ethyl, propyl, *iso*-propyl, *n*-butyl, *iso*-butyl, *sec-*butyl, or *tert*-butyl.

The terms "alkenyl" and"alkynyl" refer to unsaturated aliphatic groups analogous in length and possible substitution to the alkyls described above, but which contain at least one double or triple carbon-carbon bond, respectively.

As used herein, the term "halogen" designates -F, -Cl, -Br or -I; the term "thiol" means -SH; the term "hydroxyl"or "hydroxyl" means -OH; and the term "sulfonyl" means -SO₂-;

As used herein, the term "cycloalkyl" refers to a saturated hydrocarbon ring having the specified number of carbon atoms. Cycloalkyl groups are monocyclic ring systems. For example, C₃-C₇ cycloalkyl refers to a cycloalkyl group having from 3 to 7 carbon atoms. Suitable C₃-C₇ cycloalkyl groups include cyclopropyl, cyclobutyl, cyclopentyl and cyclohexyl, cycloheptyl.

The term "alkylamino" refers to an alkyl group, having an amino (-NH-) attached thereto, thus C₁-C₄ alkyamino is represented by -NH-C₁-C₄-alkyl. Examples of C₁-C₄ alkyamino include methylamino, ethylamino propylamino, *iso*-propylamino, n-butylamino, *iso-*butylamino, *sec*-butylamino, or *tert-*butylamino.

As used herein, the term "alkoxy" refers to an -O-alkyl group wherein "alkyl" is defined above. Example of C₁-C₄ alkoxyl groups include methoxy, ethoxy, propyloxy, tert-butoxy and the like.

The term acyl group refers to R₁₀C(O)-, wherein R₁₀ has the meaning defined in Formula(I)

The terms "heterocycle" or "heterocyclic group" refer to 4 to 10-membered ring structures, which can be mono or fused rings, which ring structures include one to four heteroatoms. Heterocyclic groups include pyrrolidine, oxolane, thiolane, imidazole, oxazole, piperidine, piperazine, morpholine or indolyl.

The term "heteroatom" as used herein means an atom of any element other than carbon or hydrogen. Preferred heteroatoms are nitrogen, oxygen, sulfur, phosphorus and selenium.

The term "protecting group" as used herein means temporary substituents which protect a potentially reactive functional group from undesired chemical transformations. Examples of such protecting groups include esters of carboxylic acids, carbamyl of amines respectively. The field of protecting group chemistry has been reviewed (Greene, T.W.; Wuts, P.G.M. Protective Groups in Organic Synthesis, 2nd ed.; Wiley: New York, 1991).

As used herein, the term "halo" refers to the halogen radicals fluoro, chloro, bromo and iodo.

As used herein, the term "treatment" refers to prophylaxis of the condition, ameliorating or stabilising the specified condition, reducing or eliminating the symptoms of the condition, slowing or eliminating the progression of the condition, and preventing or delaying reoccurrence of the condition in a previously afflicted patient or subject.

As used herein, the term "therapeutically effective amount" refers to the quantity of a compound of formula (I), or a pharmaceutically acceptable salt thereof, which will elicit the desired biological response in an animal or human body.

As used herein, the term "subject" refers to an animal or human body.

The terms "D amino acid" or "D" and "L amino acid"or "L" as used herein refer to absolute configuration of the amino acid, rather than a particular direction of rotation of plane-polarized light. The usage herein is consistent with standard usage by those of skill in the art.

Thus, "D" and "L" refer to absolute configuration based on the assignment of the absolute configuration of the dextrotatory and levorotatory forms of glyceraldehyde.

"L" and "D" absolute configuration notation of the chiral centers in compound of formula(I) 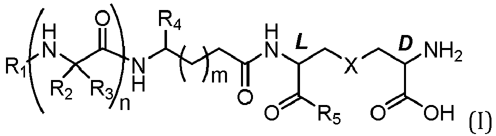 corresponds to (S) and (R) absolute configuration notation according to Cahn, Ingold & Prelog. Rules as provided in (Ie)

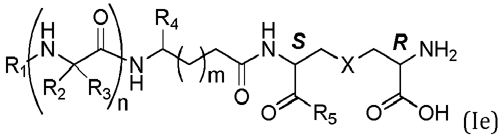

The terms "solid support", "solid phase" and "resin", refer indifferently in the present invention to a support conventionally used in organic chemistry, and particularly in peptide synthesis comprising a matrix polymer and a linker.

The solid support comprises a base matrix such as gelatinous or macroporous resins, for example polystyrene, and an anchor referred to as a "linker" designed to produce after cleavage a given functionality. The name of the solid support, in all strictness, refers to the name of the linker and the matrix.

Advantageously the base support is chosen among the polystyrene supports, polyamide supports, polyethylene glycol supports, polyacrylic supports, composite supports and copolymers thereof, it being possible for said support to be in the form of beads, of a film-coated support such as rings or lanterns, of a plug, or of a non-cross-linked soluble support.

More advantageously, the base support is selected from gelatinous or macroporous resins having a matrix with polystyrene (PS), or having a matrix with polyamide (PL) or polyethylene glycol (PEG), or else composite supports of polyethylene glycol-polystyrene (PEG-PS) or polyethylene glycol-dimethylacrylamide (PEGA).

Advantageously, the solid support is chosen in the group comprising acid-labile resins. Among linkers, TFA-labile ones are especially useful for the production of combinatorial libraries. Indeed, by using methodology cleavage and post-cleavage, workups are straightforward and often require only simple evaporation of TFA that can be done in parallel using vacuum centrifugator or inert gas bubbling.

The present invention also provides processes for the preparation of a compound of formula (I) or a pharmaceutically acceptable salt thereof.

Thus, compounds of formula (I) may be prepared by methods known in the art of organic synthesis, particularly in the peptide synthesis as set forth in part in the following synthesis schemes.

The methodology involves the formation of peptide bond by any well known procedure in the art, such as solid phase synthesis or liquid phase synthesis.

The most commonly employed methods for peptide bond formation in solution include: N,N'-dicyclohexylcarbodiimide (DCC) or 1-ethyl-3-(3'-dimethylaminopropyl)carbodiimide hydrochloride (EDC), i.e. water-soluble carbodiimide, O-(1 H-benzotriazol- 1 -yl)-N,N,N',N'-tetramethyluronium tetrafluoroborate (TBTU), benzotriazol-1-yl-oxytris(dimethylamino)phosphonium hexafluorophosphate (BOP), benzotriazole-1-yl-oxy-tris-pyrrolidino-phosphonium hexafluorophosphate (PyBOP), O-(7-azabenzotriazol- 1 -yl)- 1 ,2,3-tetramethyluronium hexafluorophosphate (HATU), O-benzotriazol- 1 -yl-N,N,N',N'-tetramethyluronium hexafluorophosphate (HBTU), O-benzotriazol- 1 -yl-tetramethyltetrafluoroborate (TBTU), N-hydroxy- 5-norbornene-2,3-dicarbodiimide, or any other coupling agent in a solvent such as ether, acetone, chloroform, dichloromethane, ethyl acetate, DMF, tetrahydrofuran (THF), acetonitrile, dimethylsulfoxide (DMSO), N-methyl pyrrolidinone (NMP), under ice-cooling or at room temperature, preferably in the presence of an acylation catalyst such as dimethylaminopyridine (DMAP), pyridine, N-hydroxybenzotriazole (HOBt), 1-hydroxy-7-azabenzotriazole (HOAt), N-hydroxy succinimide and the like.

Preferably the coupling reaction is carried out by employing PyBOP or HATU in an organic solvent such as DCM and/or DMF in presence of a base like NMM (N-methyl imidazole) or DIEA (diisopropyl ethylamine).

PyBOP or HATU react exclusively with carboxylate salts (R-COO-); mixtures of PyBOP or HATU and a carboxylic acid (R-COOH) remain stable. This procedure eliminates the requirement for a separate neutralization step saving time and minimizing diketopiperazine formation. Three equivalents of base (DIEA or NMM) are necessary to neutralize the carboxylic acid, the amine salt, and the acidic hydroxybenzotriazole or 7-azabenzotriazole.

When using PyBOP or HATU, the reaction mixture has to be kept near basic pH in order to ensure a fast coupling. Under such conditions, the coupling rate is so high that racemization is negligible using urethane-protected amino acid couplings and fairly low in segment coupling. The excess of acid and "onium" salt (PyBOP or HATU) is typically 1.1 molar equivalent in solution synthesis. In solid phase, excess from 1.5-3 equivalents are commonly used. The byproducts are water and DCM soluble that allows purification of product by precipitation by water or diethyl ether (in solution synthesis) or purification by flash chromatography.

This reaction is preferably carried out in a solvent such as methylene chloride, chloroform, tetrahydrofuran, dioxane, dimethylformamide ethyl acetate, or the like, under ice-cooling or at ambient temperature, and the reaction in the presence of an inert gas is usually carried out in anhydrous conditions.

In the synthesis of the compounds of formula (I), it will be necessary, or at least desirable, in many reactions to protect the amino groups and/or the carboxy groups. The synthetic route chosen for the di, tri, tetra or pentapeptide synthesis may require removal of one or the other or both of said protecting groups in order to permit further reaction at the regenerated amino or carboxy group; i.e., the protecting groups used are reversible and, in most instances, are removable independently of each other. Additionally, the choice of protecting group for a given amino group depends upon the role of said amino group in the overall reaction scheme. Amino protecting groups having varying levels of lability, i.e., ease of removal, will be used. The same is true as regards carboxy protecting groups. Such groups are known in the art and attention is directed to the reviews by Bodansky et al., "Peptide Synthesis", 2nd Ed., John Wiley & Sons, N.Y. (1976).

Examples of conventional amino protecting groups include vinyl, allyl, t-butoxycarbonyl, benzyl, benzyloxycarbonyl, 2-nitrobenzyl, 4-nitrobenzyl, 2-nitrobenzyloxylcarbonyl, formyl, benzoyl, acetyl, ethylcarbonyl, chloroacetyl, trichloroacetyl, trifluoroacetyl, methyloxycarbonyl, allyloxycarbonyl, trimethylsilyl, triethylsilyl, triphenylsilyl, t-butyl-dimethylsilyl, methyldiphenylsilyl, 2,4-dimethoxybenzyl, 2,4-dimethoxybenzyloxycarbonyl, 4-methoxybenzyl, 4-(methoxymethyloxy)phenyl, bis-(4-methoxyphenyl)methyl, t-butoxycarbonylmethyl, allyoxycarbonylmethyl, methoxymethyl, methylthiomethyl, methoxyethoxymethyl, [2-(trimethylsilyl) ethoxy] methyl or 2-(methylthiomethoxy)ethoxycarbonyl. In general, amino protecting groups which are readily removed under acid conditions or catalytic hydrogenolysis are preferred, e.g. t-butoxycarbonyl, benzyloxycarbonyl and triphenylmethyl.

Conventional carboxy-protecting groups which can be employed in the present invention to block or protect the carboxylic acid function are well-known to those skilled in the art and, preferably, said groups can be removed, if desired, by methods which do not result in any appreciable destruction of the remaining portion of the molecule, for example, by chemical or enzymatic hydrolysis, treatment with chemical reducing agents under mild conditions, irradiation with ultraviolet light or catalytic hydrogenation. Examples of such readily removable carboxy-protecting groups include moieties such as C₁-C₆ alkyl, diphenylmethyl (benzyhydryl), 2-naphthylmethyl, 4-pyridylmethyl, phenacyl, acetonyl, 2,2,2-trichloroethyl, silyl such as trimethylsilyl and t-butyldimethylsilyl, phenyl, ring substituted phenyl, e.g., 4-chlorophenyl, tolyl, and t-butylphenyl, phenyl C1-6 alkyl, ring substituted phenyl C1-6 alkyl, e.g., benzyl, 4-methoxybenzyl, 4-nitrobenzyl (p-nitrobenzyl), 2-nitrobenzyl (o-nitrobenzyl), and triphenylmethyl (trityl), methoxymethyl, 2,2,2-trichloroethoxycarbonyl, benzyloxymethyl, C₁-C₆ alkanoyloxy C₁-C₆ alkyl such as acetoxymethyl, propionyloxymethyl, C₂-C₆ alkenyl such as vinyl and allyl. Particularly advantageous carboxy protecting groups are benzyl, p-nitrobenzyl, o-nitrobenzyl, 2,4-dimethoxybenzyl, 4-methoxybenzyl, allyl, substituted allyl, t-butyl or diphenylmethyl (DMP).

Other suitable amino and carboxy-protecting groups well known to those skilled in the art can be found in "Protective Groups in Organic Synthesis", 3rd Ed. Theodora W. Greene (John Wiley & Sons, 1999), incorporated herein by reference.

After synthesis of the desired peptide, it is subjected to the deprotection reaction and, in the case of solid peptide synthesis, cut out from the solid support. Such peptide cutting reaction can be carried with hydrogen fluoride or trifluoromethane sulfonic acid for the Boc method, and with TFA for the Fmoc method.

The deprotection method used during stepwise assembly of peptide chain and for the final deblocking of all functional groups to yield the free peptide is determined by the synthetic strategy. In the present invention, suitable orthogonal amine protecting groups may be selected and deprotection may be affected for the group using conditions amenable to hydrolysis of the group as specified in Table.

For example, protective groups can be removed by acid, base, and hydrogenolysis. Groups such as trityl, dimethoxytrityl, acetal and t-butyldimethylsilyl are acid labile and may be used to protect carboxy and hydroxy reactive moieties in the presence of amino groups protected with Cbz groups, which are removable by hydrogenolysis, and Fmoc groups, which are base labile. Carboxylic acid and hydroxy reactive moieties may be blocked with base labile groups such as, without limitation, methyl, ethyl, and acetyl in the presence of amines blocked with acid labile groups such as t-butyl carbamate or with carbamates that are both acid and base stable but hydrolytically removable.

Carboxylic acid and hydroxy reactive moieties may also be blocked with hydrolytically removable protective groups such as the benzyl group, while amine groups capable of hydrogen bonding with acids may be blocked with base labile groups such as Fmoc. Carboxylic acid reactive moieties may be blocked with oxidative ly-removable protective groups such as 2,4-dimethoxybenzyl, while coexisting amino groups may be blocked with fluoride labile silyl carbamates.

Allyl blocking groups are useful in the presence of acid- and base- protecting groups since the former are stable and can be subsequently removed by metal or pi-acid catalysts. For example, an allyl-blocked carboxylic acid can be deprotected with a palladium (0)-catalyzed reaction in the presence of acid labile t-butyl carbamate or base- labile acetate amine protecting groups. Yet another form of protecting group is a resin to which a compound or intermediate may be attached. As long as the residue is attached to the resin, that functional group is blocked and cannot react. Once released from the resin, the functional group is available to react.

For the final deprotection, several options for deprotection reaction processes are shown below and include (a) catalytic hydrogenolysis using molecular hydrogen and palladium (H₂/Pd) or catalytic transfer hydrogenolysis; (b) reduction with metallic sodium in liquid ammonia and (c) or reaction with strong acids using halogenated acids or hydrohalogenic acids, e.g. hydrobromic acid in acetic acid (HBr/AcOH) or liquid HF, BBr₃/DCM, TFA/thioanisole and sulfonic acids.

Of these, cleavage of protecting groups under neutral conditions is highly desirable because such procedures would:
(a) be free from racemization;
(b) leave acid sensitive amino acids intact;
(c) be useful particularly in the synthesis of acid/base sensitive biologically active peptides; and
(d) eliminate side reactions that are commonly observed in acidolysis and base treatments.

Two such methods, catalytic hydrogenation (Schlatter, et al. 1977; Rylander, 1979).

In Hydrogenation and Hydrogenolysis in Synthetic Organic Chemistry, Delft University Press, 1977) and catalytic transfer hydrogenation (Khan & Sivanandaiah, 1978; Anwer & Spatola, 1981; Anantharamaiah & Sivanandaiah, 1977) have become popular for the cleavage of benzyl esters, benzyl ethers and benzyloxycarbonyl protecting groups.

The only limitation being the intolerance of palladium catalysts to the presence of divalent sulfur. While hydrogenation with gaseous hydrogen is typically performed under high pressure (Parr apparatus), transfer hydrogenation is typically performed under ambient pressure. The transfer hydrogenation procedure utilizes simple organic molecules (cyclohexene, cycloxexadiene, formic acid) or inorganic compounds (phosphinic acid and its salts, hydrazine) as an in situ source of hydrogen.

In the examples presented herein, certain protecting and activating groups are specifically illustrated. However, one skilled in the art will recognize that other protecting or activating groups could have been used. The choice of a particular protecting group is dependent to a great extent upon the availability of the necessary reagent, its effect upon solubility of the "protected" compound, its ease of removal and the presence of other groups which might be effected by its use; i.e. its selectivity, or its removal.

"S-protecting group" means a substituent such as the S-protecting groups disclosed in Greene, "Protective Groups in Organic Synthesis", Wiley, New York, 2007 4th edition Paul Lloyd- Williams, Fernando Albericio, Ernest Giralt, "Chemical Approaches to the Synthesis of Peptides and Proteins", CRC Press, 1997 or Houben-Weyl, "Methods of Organic Chemistry, Synthesis of Peptides and Peptidomimetics", VoI E 22a, VoI E 22b, VoI E 22c, VoI E 22d., M. Goodmann Ed., Georg Thieme Verlag, 2002, which protects reactive sulphur against undesirable reactions.

"S-protecting group" can comprise the benzyl group, a Trt group, acetamidomethyl, the S-tert-butyl, S-acetyl, S-methoxymethyl, as the protecting group for a free mercapto group in the amino acid residue and the like.

During the preparation of compounds of the present invention, or intermediates thereto, it may also be desirable or necessary to prevent cross-reaction between chemically active substituents other than those present on naturally occurring or other amino acids. The substituents may be protected by standard blocking groups which may subsequently be removed or retained, as required, by known methods to afford the desired products or intermediates. Selective protection or deprotection may also be necessary or desirable to allow conversion or removal of existing substituents, or to allow subsequent reaction to afford the final desired product.

The selection of a suitable protecting group depends upon the functional group being protected, the conditions to which the protecting group is being exposed and to other functional groups which may be present in the molecule.

The compounds (I) of this invention can be prepared by chemical synthetic methods details of which will be apparent from the following description.

The crude compounds or intermediates obtained according to the processes described herein can be subjected to purification. Purification is carried out by any one of the methods known for this purpose, i.e. any conventional procedure involving extraction, precipitation, chromatography, electrophoresis, or the like. For example, HPLC (high performance liquid chromatography) can be used. The elution can be carried using a water-acetonitrile-based solvent commonly employed for protein purification.

Compounds of formula (I) may be prepared by the general methods outlined hereinafter. In the following description, the groups X, W, R₁, R₂, R₃, R₄, R₅, R₆, R₇, R₈, R₉, m and n, have the meaning as previously defined for compounds of formula (I) unless otherwise stated.

Compounds of the general formula (I), can be prepared by reacting a compound of formula (Ia) or a protected derivative therefore with a suitable acyl chloride or bromide , sulfonyl chloride to obtain a compound of formula (I) in which R₁ is a -Z-R₆ (II) in which Z is -C(O)- or -S(O)₂-, followed by the removal any protecting group.

The acylation reaction is conducted using standard procedure with the appropriate acid chloride or bromide in presence of an organic base, preferably a tertiary amine such as triethylamine, N-methylmorpholine or pyridine in an inert solvent.

The acylation can be carried out in the presence of a suitable acid anhydride (simple or mixed) according to standard procedures. When an anhydride is to be used for this acylation step, mixed anhydrides especially those derived from a low molecular weight carboxylic acid, and particularly the mixed carboxylic-carbonic anhydrides, are preferred.

The sulfonyl reaction is conducted in accordance with standard methods of organic chemistry, see for example, Lieb. Ann. Chem. P. 641, 1990, with the appropriate sulfonyl chloride or bromide.

This reaction is usually carried out in an inert organic solvent. Suitable solvents are aliphatic hydrocarbons, such as pentane, hexane, cyclohexane and petroleum ether, aromatic hydrocarbons, such as toluene, o-, m- and p-xylene, halogenated hydrocarbons, such as dichloromethane (DCM), chloroform and chlorobenzene, ethers, such as diethyl ether, diisopropyl ether, methyl tert-butyl ether (MTBE), dioxane, anisole and tetrahydrofuran (THF), nitriles, such as acetonitrile and propionitrile, ketones, such as acetone, methyl ethyl ketone, diethyl ketone and tert. -butyl methyl ketone, and also dimethyl sulfoxide (DMSO), dimethylformamide (DMF) and dimethylacetamide, preferably THF, MTBE, DCM, chloroform, acetonitrile, toluene or DMF or a mixture thereof.

The reaction is carried out in the presence of a base. Suitable bases are, in general, inorganic compounds, such as alkali metal and alkaline earth metal hydroxides, such as lithium hydroxide, sodium hydroxide, potassium hydroxide and calcium hydroxide, alkali metal and alkaline earth metal oxides, such as lithium oxide, sodium oxide, calcium oxide and magnesium oxide, alkali metal and alkaline earth metal hydrides, such as lithium hydride, sodium hydride, potassium hydride and calcium hydride, alkali metal and alkaline earth metal carbonates, such as lithium carbonate, potassium carbonate and calcium carbonate, and also alkali metal bicarbonates, such as sodium bicarbonate, moreover organic bases, for example tertiary amines, such as trimethylamine, triethylamine, diisopropylethylamine and N-methylpiperidine, pyridine, substituted pyridines, such as collidine, lutidine and 4-dimethylaminopyridine, and also bicyclic amines. Particular preference is given to triethylamine, pyridine, triethylamine and potassium carbonate. The bases are generally employed in equimolar amounts, in excess or, if appropriate, as solvent. The amount of base is typically 0.5 to 5 molar equivalents relative to 1 mole of peptide (I).

Generally, the reaction is carried out at temperatures of from -30°C to 120°C, preferably from -10°C to 100°C.

Compounds of the general formula (I), wherein R₁ is C₁-C₂₀ alkyl, C₂-C₂₀ alkenyl or C₂-C₂₀ alkynyl can be prepared by reacting a compound of formula (I) or protected derivatives thereof, with a suitable alkylating agent.

Suitable alkylating agents include, but are not limited to, alkyl chlorides, bromides, iodides and sulfonate esters. A wide variety of bases and solvents can be employed and these bases and solvents are well known in the art. In addition, a compound having an alkylamino group may be produced by the reductive alkylation of a compound having amino group with a carbonyl compound. As the alkylation of amino group, for example, the method described in "Jikken Kagaku Koza (volume 20) Yuki Gosei 2 (Organic Synthesis 2)" edited by The Chemical Society of Japan (4th edition, Maruzen, 1992, p. 300) or the like maybe employed.

Compounds of formula (I), wherein R₁ is a muramic acid derivative of formula (III), can be prepared by reacting an activated carboxylic acid of a compound of formula (IIIa), with a compound of formula (I) or a protected derivative thereof wherein R₁ is hydrogen, to form a peptide bond according to the procedure described above. 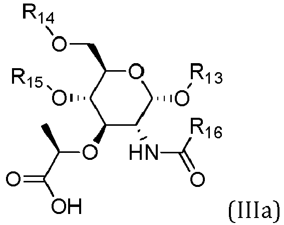 in which:
- **R₁₆** is C₁-C₄ alkyl optionally substituted by Op , wherein p is a hydroxyl protecting group
- **R₁₃** is an oxygen protecting group or benzyl group;
- **R₁₄** is an oxygen protecting group, C₁-C₂₀ alkyl, C₁-C₂₀ alkenyl, C₁-C₂₀ alkynyl each of which may be optionally substituted by C₁-C₄ alkyamino or C₁-C₄ alkoxy;
- **R₁₅** is oxygen protecting group,
followed by the removal of any protecting group as appropriate.

Compounds of formula (I), wherein R₉ is an acyl group C(0)-R₁₀ or sulphonyl group S(0)₂-R₁₀, can be obtained by reaction of a compound of formula (I) wherein R₉ is hydroxyl with a suitable acyl chloride or bromide and sulfonyl chloride, followed by the removal of any protecting group.

Compounds of the general formula (I) can be obtained by reacting a compound of formula (VIII), 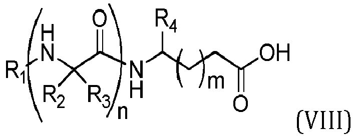 in which R₁, R₂, R₃, R₄, R₅, n and m have the meanings defined in formula(I) or they are protecting groups thereof, with a compound of formula (IX), 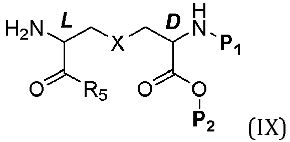 in which R₅ and X have the meanings of formula(I) or they are protecting groups thereof, P₁ represents a nitrogen protecting group, P₂ represents a carboxy protecting group using the general methods described above for obtaining a peptide bond, followed by removal of any protecting group.

The dipeptide of the general formula (VIII) can be obtained by reaction of an activated derivative of the general formula (X), 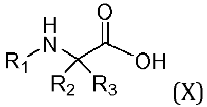 in which R₁, R₂ and R₃ have the meanings of formula(I) or they are protecting groups thereof, with a compound of general formula (XI), 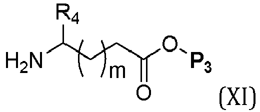 in which R₄ and m have the meanings defined in formula(I) or they are group or function with protecting groups thereof and P₃ is a carboxy proctecting group, using the general methods described above for obtaining a peptide bond, following by removal of any protecting group.

Compounds of formula (IX) also known as *meso*-lanthionine derivatives in which X represents a sulfur atom can be prepared from a compound of formula (XII), 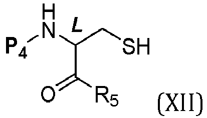 in which R₅ has the meanings of formula(I) or it is a protecting group thereof and P₄ represents a nitrogen protecting group where P₁ and P₄ are orthogonal protections and D-amino acid derivatives of general formula (XIII), 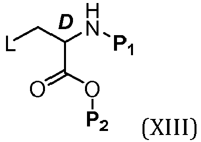 in which P₁ represents a nitrogen protecting group, P₂ represents a carboxy protecting group and L represents a suitable leaving group.

*Meso*lanthionine derivatives can also be prepared from D-amino acid of general formula (XIV), 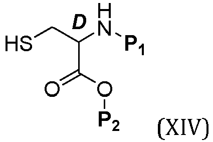 in which P₁ represents a nitrogen protecting group and P₂ represents a carboxy protecting group and L-amino acid of general formula (XV), 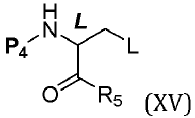 in which P₄ represents a nitrogen protecting group and where P₁ and P₄ are orthogonal protections.

Typically, the S-protected cysteine derivatives are dissolved in an acid that is strong enough to remove the acidolytically removable sulfur-protecting group to afford compound of formula (XII) or (XIV) but not strong enough to remove any amine-protecting group, optionally in the presence of a scavenger. Suitable such acids include trifluoroacetic acid and other strong acids such as trifluoromethanesulfonic acid, optionally in the presence of a cosolvent such as dichloromethane; suitable scavengers include aromatic ethers and sulfides such as anisole and thioanisole, phenols such as cresol, and, most efficiently, silanes including trialkylsilanes such as triethylsilane and triisopropylsilane and silane polymers such as poly(methylhydrosiloxane); and a particularly suitable deprotection reagent is trifluoroacetic acid in the presence of poly(methylhydrosiloxane).

Compounds of formula (IX), in which X represents an oxygen atom known as *meso-*2,6-diamino-4-Oxa-pimelic acid (*meso*OxaDAP) can be prepared reaction of L-aziridine derivatives of general formula (XVI), 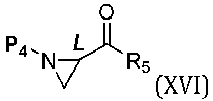 in which R₅ has the meanings of formula(I) or it is a protecting group thereof and P₄ represents a nitrogen protecting group with D-serine derivatives of general formula (XVII), 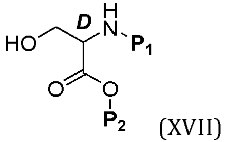 in which P₁ represents a nitrogen protecting group, P₂ represents a carboxy protecting group and where P₁ and P₄ are orthogonal protections.

Altenatively, *meso*OxaDAP derivatives can be prepared from D-aziridine of general formula (XVIII), 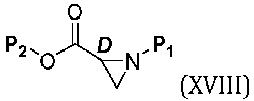 in which p is a nitrogen protecting group with oxygen nucleophiles derived from L-serine or substituted L-serine derivatives of general formula (XIX), 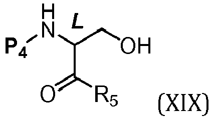 in which P₄ represents a nitrogen protecting group and where P₁ and P₄ are orthogonal protections.

The reaction is conveniently carried out in an aprotic solvent such as dichloroethane, toluene and in the presence of a suitable lewis acid such as Boron trifluoride diethyl etherate.

Compounds of formula (IX) in which X represents nitrogen known as *meso*-2,6-diamino-4-Aza-pimelic acid (*meso*AzaDAP) can be prepared by reaction of an aziridine of formula (XVI) with an amine nucleophile derivatives of general formula (XX), 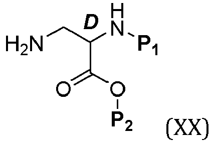 in which P₁ represents a nitrogen protecting group, P₂ represents a carboxy protecting group and where P₁ and P₄ are orthogonal protections.

Alternatively, the protected *meso*AzaDAP derivatives can be prepared by reaction D-aziridine derivatives of general formula (XVIII) and with amine nucleophile derivatives of general formula (XXI), 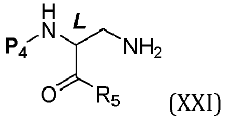 in which P₄ represents a nitrogen protecting group and where P₁ and P₄ are orthogonal protections.

The reaction is conveniently carried out in an aprotic solvent such as dichloroethane, toluene and in the presence of a suitable lewis acid such as Boron trifluoride diethyl etherate.

Another object of the present invention relates to a process for producing a solid phase peptide of formula (I).

Solid phase peptide synthesis techniques may follow any methods conventionally employed for peptide synthesis, for example, employing a condensing agent method, an azide method, a chloride method, an acid anhydride method, a mixed anhydride method, an active ester method, an enzyme method as disclosed in Nobuo Izumiya et al., "Fundamentals and Experiments for Peptide Synthesis (in Japanese)", issued from Maruzen Co., Ltd., 1985, or a standard Fmoc protocol, see e.g. Carpino et al., 1970, J. Am. Chem. Soc. 92(19):5748-5749; Carpino et al., 1972, J. Org. Chem. 37(22):3404-3409.

The process consists in coupling, an aminoacid or a peptide of the general formula (XXII), 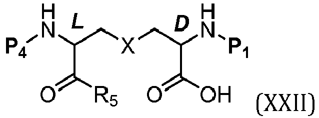 in which R₅ and X have the meanings of formula(I) or they are protecting groups thereof, P₁ and P₄ represent a nitrogen protecting group, using the general methods described above for obtaining a peptide bond, followed by removal of any protecting group and where P₁ and P₄ are orthogonal protections. with an activated solid support of the following formula (XXIII): 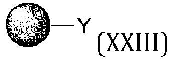 wherein: 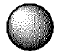 is a solid support as defined above, and Y represents an activating group comprising chlorides, bromides, iodides, p-nitrophenolate.

The coupling reaction may be carried out by employing a condensing reagent such as N,N'-dicyclohexylcarbodiimide (DCC) or 1-ethyl-3-(3'-dimethylaminopropyl)carbodiimide hydrochloride (EDC), i.e. water-soluble carbodiimide, O-(1 H-benzotriazol- 1 -yl)-N,N,N',N'-tetramethyluronium tetrafluoroborate (TBTU), benzotriazol-1-yl-oxytris(dimethylamino)phosphonium hexafluorophosphate (BOP), O-(7-azabenzotriazol- 1 -yl)- 1 ,2,3-tetramethyluronium hexafluorophosphate (HATU), O-benzotriazol- 1 -yl-N,N,N',N'-tetramethyluronium hexafluorophosphate (HBTU), O-benzotriazol- 1 -yl-tetramethyltetrafluoroborate (TBTU), N-hydroxy- 5-norbornene-2,3-dicarbodiimide, or any other coupling agent in a solvent such as ether, acetone, chloroform, dichloromethane, ethyl acetate, DMF, tetrahydrofuran (THF), acetonitrile, dimethylsulfoxide (DMSO), N-methyl pyrrolidinone (NMP), under ice-cooling or at room temperature, preferably in the presence of an acylation catalyst such as dimethylaminopyridine (DMAP), pyridine, N-hydroxybenzotriazole (HOBt), 1-hydroxy-7-azabenzotriazole (HOAt), N-hydroxy succinimide and the like.

Preferably the coupling reaction is carried out by employing DCC, DMAP in an organic solvent such as DCM and/or DMF.

It being understood that the protective groups of the amine group, and the condition of cleavage of the solid support, are different. Following to removal of the amine group protecting group P₄ the compound so obtained can be condensed with compound of formula (XXIV) 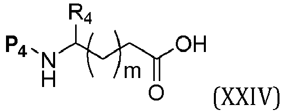 in which m and R₄ have the meanings as defined in formula(I) or are protected derivatives thereof and P₄ represent a nitrogen protecting group.

The product so obtained by removal of nitrogen protecting group P₄ can be reacted with an activated derivative of the general formula (XXV) to obtain a compound of formula (I) wherein n is 1. 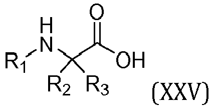 in which R₁, R₂, and R₃ have the meanings of formula (I) or they are protecting groups thereof.

Following the removal of the amine group protected by R₁, the product so obtained can be reacted with an activated derivative of the general formula (XXV) to obtain a compound of formula (I) wherein n is 2.

Then, the product obtained can be separated from its support and, if necessary, the protective groups of the amine and carboxyl groups can be removed.

Peptides synthesized via solid phase synthesis techniques can be cleaved and isolated according to, for example, the following non-limiting techniques: the peptide may be cleaved from the resin using techniques well known to those skilled in the art. For example, solutions of 1% or 2% trifluoroacetic acid (TFA) in dichloromethane (DCM) or a combination of a 1% and a 2% solution of TFA in DCM may be used to cleave the peptide, or a 10% solution of trifluoroethanol (TFE) in DCM may also be used. Acetic acid (AcOH), hydrochloric acid (HCl) or formic acid may also be used to cleave the peptide. In these cases the recovered peptide are fully protected except the NH in the C-terminal position. The specific cleavage reagent, solvents and time required for cleavage will depend on the particular peptide being cleaved.

Higher concentrations of acids or strongly acidic conditions such as HF in the case of for example carbonate Merrifield resin may be used to also remove the protecting. It is advantageous to use scavengers, such as silanes or other scavenger cocktails (especially for cation- and/or acid-sensitive side chain (or group) incorporating peptides), to prevent any side reactions.

After cleavage the cleavage fractions are subjected to standard work-up procedures to isolate the peptide. Typically, the combined cleavage fractions are concentrated under vacuum, followed by reconstitution with polar aprotic or polar aprotic solvents such as ethanol, methanol, isopropyl alcohol, acetone, acetonitrile, dimethyl formamide, dichloromethane, etc., followed by precipitation or crystallization with antisolvent such as water, diethyl-ether or hexanes, and collection by vacuum filtration. Alternatively, the product may be triturated with organic solvents or water after isolation of the peptide.

### PHARMACEUTICAL COMPOSITIONS

Compounds of formula (I), or a pharmaceutically acceptable salt thereof, will normally, but not necessarily, be formulated into pharmaceutical compositions prior to administration to a patient.

Accordingly, in another aspect the invention is directed to pharmaceutical compositions comprising a compound of formula (I), or a pharmaceutically acceptable salt thereof, and one or more pharmaceutically acceptable excipients.

These compositions can be used either as vaccine adjuvants or as non-specific stimulants of anti-infectious and anti-tumoral immunity.

Pharmaceutical compositions comprising a compound of formula (I), or a pharmaceutically acceptable salt thereof, may be prepared using techniques and methods known to those skilled in the art. Some of the methods commonly used in the art are described in Remington's Pharmaceutical Sciences (Mack Publishing Company).

Used as vaccine adjuvants, the products according to the invention can be administered at the same time and by the same method as the antigen (viral, bacterial, parasitic or other antigen) against which it is desired to increase the cell immunity reactions (delayed-type hypersensitivity) or the production of circulating or local antibodies in the immunized subject (man or domestic animal).

The products are administered in relatively low doses (of the order of a mg) as a mixture with the antigen and by the same method (intramuscular, subcutaneous, intravenous, intranasal or oral method). If necessary, the product and the antigen can be emulsified in an appropriate oily excipient or incorporated into liposomes.

The dose of the compounds of the invention used in the treatment of the aforementioned disorders will vary in the usual way with the seriousness of the disorders, the weight of the sufferer, and other similar factors. However, as a general guide suitable unit doses may e lspero be 0.05 to 1000 mg, more suitably 1.0 to 500mg or 1.0 to 200 mg, and such unit doses may be administered more than once a day, for example two or three times a day.

It will be appreciated that it may be necessary to make routine variations to the dosage, depending on the age and condition of the patient and the precise dosage will be ultimately at the discretion of the attendant physician or veterinarian. The dosage will also depend on the route of administration and the particular compound selected.

As non-specific immunostimulants, the compounds of the invention are administered at doses of between 0.1 and 50 mg/kg by the parenteral method (intravenous, subcutaneous or intramuscular method) or by the intranasal, oral, rectal or, if appropriate, intra-tumoral method.

A pharmaceutical composition of a compound of formula (I), or a pharmaceutically acceptable salt thereof, may be formulated for administration by any appropriate route, for example by the inhaled, nasal, oral (including buccal or sublingual), topical (including buccal, sublingual, transdermal, epicutaneous) or parenteral (subcutaneous, intramuscular, intravenous, intradermal) route.

Thus, a pharmaceutical composition of a compound of formula (I), or a pharmaceutically acceptable salt thereof, may be formulated as, for example, a solution or suspension (aqueous or non-aqueous), tablet, capsule, powder, granule, lozenge, lotion, cream, ointment, gel, foam or reconstitutable powder depending on the particular route of administration.

Such pharmaceutical compositions may be prepared by any method known in the art of pharmacy, for example by bringing into association the active ingredient with the excipient(s).

Tablets and capsules for oral administration may be in unit dose presentation form, and may contain conventional excipients such as binding agents, for example syrup, acacia, gelatine, sorbitol, tragacanth, or polyvinylpyrrolidone; fillers, for example lactose, sugar, maize-starch, calcium phosphate, sorbitol or glycine; tabletting lubricants, for example magnesium stearate, talc, polyethylene glycol or silica; disintegrants, for example potato starch; or acceptable wetting agents such as sodium lauryl sulfate. The tablets may be coated according to methods well known in normal pharmaceutical practice.

Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatine, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl hydroxybenzoate or sorbic acid, and, if desired, conventional flavouring or colouring agents.

Pharmaceutical compositions of a compound of formula (I) or a pharmaceutically acceptable salt thereof, for topical administration, may be presented as, for instance, ointments, creams or lotions, eye ointments and eye or ear drops, impregnated dressings and aerosols, and may contain appropriate conventional additives such as preservatives, solvents to assist drug penetration and emollients in ointments and creams. The compositions may also contain compatible conventional carriers, such as cream or ointment bases and ethanol or oleyl alcohol for lotions. Such carriers may be present as from about 1% up to about 98% of the composition. More usually they will form up to about 80% of the composition.

The compositions for parenteral administration can be suspensions, emulsions or aqueous sterile solutions. Polyethylene glycol, propylene glycol, vegetable oils, in particular olive oil, and injectable organic esters, e.g. ethyl oleate, can be employed as the vehicle in the former cases. These compositions can also contain adjuvants, in particular wetting, emulsifying or dispersing agents.

Sterilization can be carried out in several ways, e.g. using a bacteriological filter, by incorporating sterilizing agents into the composition or by heating. The compositions can also be prepared in the form of solid compositions sterilized e.g. by irradiation, which can be dissolved in sterile water or dispersed in any other injectable sterile medium, if appropriate at the time of use.

The compositions for intranasal administration can be suspensions, emulsions or aqueous sterile solutions, which can be associated, if appropriate, with a compatible propellant.

The compositions for rectal administration are suppositories which can contain excipients, such as cacao butter or a semi-synthetic glyceride, in addition to the active product.

Pharmaceutical compositions adapted for parenteral administration include aqueous and nonaqueous sterile injection solutions which may contain antioxidants, buffers, bacteriostats and solutes which render the composition isotonic with the blood of the intended recipient; and aqueous and non-aqueous sterile suspensions which may include suspending agents and thickening agents. The compositions may be presented in unit-dose or multi-dose containers, for example sealed ampoules and vials, and may be stored in a freeze-dried (lyophilized) condition requiring only the addition of the sterile liquid carrier, for example water for injections, immediately prior to use.

Extemporaneous injection solutions and suspensions may be prepared from sterile powders, granules and tablets.

Pharmaceutical compositions for topical administration to the lung may include aerosol compositions and dry powder compositions.

### COMBINATION THERAPIES

Compounds of formula (I) and pharmaceutically acceptable salts thereof may be used in combination preparations. For example, the compounds of formula (I) and pharmaceutically acceptable salts thereof may be used in combination with one or more compounds with activity in inflammation ; one or more compounds with activity in reducing cancer.

Examples of antiinflammatory compounds that may be used in conjunction with the compounds of the invention include antibodies and inhibitors of IL-17, IL-17R, IL-23, IL-1, TNF-alpha, VLA4 or JAK/STAT receptors.

In one embodiment, the combination as herein above defined comprises an antibody selected from Secukinumab (AIN457), Ixekizumab (LY2439821), Brodalumab (AMG-827), Ustekinumab; Canakinumab or Infliximab.

In one embodiment, the combination as herein above defined comprises an inhibitor of JAK3 and JAK1 such for example Tofacitinib.

In a further embodiment, the combination as herein above defined comprises an TNF-α antagonist such as Etanercept.

Dosages of conventional anti-tumor agents are often kept as low as possible because side effects may be observed at higher dosages.

According to the invention, a combination of NOD1 and/or agents that increase NOD1 expression or activity, with available anti-tumor agents may improve the spectrum of cancers against which those anti-tumor agents are effective and reduce the required dosage of those anti-tumor agents. Thus, the invention contemplates combinations of the present NOD1-related agents with one or more anti-tumor or carcinostatic agents.

Any anti-tumor and carcinostatic agent available to one of skill in the art can be used with the present NOD1-related agents. However, in some embodiments, the selected anti-tumor or carcinostatic agents have different mechanisms of actions, or operate against somewhat different types of cancers or tumors.

For example, the NOD1-related agents of the invention can be combined with a carcinostatic agent or an immune activator to combine the pro-apoptotic effects of NOD1 with the anti-neoplastic effect of the carcinostatis agent and/or the pro-immune responses induced by the immune activator. Further, in some cases, radiotherapy or surgical treatment is performed in addition to these methods to improve the effect of the treatment.

Examples of other chemotherapeutic agents that may be used in conjunction with the compounds of the invention include Altretamine, Bleomycin, Busulphan, Calcium Folinate, Capecitabine, Carboplatin, Carmustine, Chlorambucil, Cisplatin, Cladribine, Crisantaspase, Cyclophosphamide, Cytarabine, Dacarbazine, Dactinomycin, Daunorubicin, Docetaxel, Doxorubicin, Epirubicin, Etoposide, Fludarabine, Fluorouracil, Gemcitabine, Hydroxyurea, Idarubicin, Ifosfamide, Irinotecan, Liposomal doxorubicin, Lomustine, Melphalan, Mercaptopurine, Methotrexate, Mitomycin, Mitoxantrone, Oxaliplatin, Paclitaxel, Pentostatin, Procarbazine, Raltitrexed, Streptozocin, Tegafur-uracil, Temozolomide, Thiotepa, Tioguanine/Thioguanine, Topotecan, Treosulfan, Vinblastine, Vincristine, Vindesine, Vinorelbine and a combination thereof.

In some embodiments, the compounds of the invention are administered with one or more hormones. For example, the NOD1-related agents can be administered with one or more androgens, progesterones, estrogens or anti-estrogens. Anti-estrogens act by exerting antagonistic effects on cells or tissues that are responsive to estrogen, or by competing with estrogens for access to receptor sites located on the cell surface. For example, the drugs tamoxifen (brand name: Nolvadex) or Arimidex (Anastrozole), are anti-estrogens that can be used. Tamoxifen has been used in the treatment of breast cancer and to reduce the breast cancer incidence in high-risk women.

The invention thus provides, in a further aspect, a combination comprising a compound of the invention together with a further therapeutic agent or agents

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations.

When a compound of the invention is used in combination with a second therapeutic agent active against the same disease state the dose of each compound may differ from that when the compound is used alone. Appropriate doses will be readily appreciated by those skilled in the art.

### UTILITY

Compounds of formula (I) and their pharmaceutically acceptable salts are NOD modulators, particularly they are NOD1.

Furthermore certain compounds of formula (I), wherein R₁ is a Muramic acid derivative of formula (III) provide agonism of NOD1 and NOD2.

Thus, compounds of formula (I) and its pharmaceutically acceptable salts are useful in the treatment of conditions for which agonism of NOD1 or NOD1 and NOD2 receptor is beneficial.

In a furthert aspect, the present invention provides a compound of formula (I) and its pharmaceutically acceptable salts, for use in therapy

Compounds of formula (I) or pharmaceutically acceptable salts thereof may be of use in the treatment and preventing tumor growth in a mammal, inflammation and/or apoptosis and in the treatment of infectious diseases.

In a yet further aspect, the present invention is directed to a method of treatment of an cancer disease mediated by NOD1 or NODland NOD2 which comprises administering to a subject in need thereof, a safe and therapeutically effective amount of a compound of formula (I), or a pharmaceutically acceptable salt thereof. Compounds of formula (I) or pharmaceutically acceptable salts thereof may be of use in the treatment of of infectious diseases.

In a furthert aspect, the present invention provides a compound of formula (I) and its pharmaceutically acceptable salts, for use in the treatment of infectious diseases.

In a yet further aspect, the present invention is directed to a method of treatment of infectious diseases mediated by NOD1 or NOD1 and NOD2 which comprises administering to a subject in need thereof, a safe and therapeutically effective amount of a compound of formula (I), or a pharmaceutically acceptable salt thereof.

Compounds of formula (I) or pharmaceutically acceptable salts thereof may be of use in the treatment of inflammatory and/or autoimmune diseases.

Example of inflammatory and or autoimmune diseases include asthma, chronic obstructive pulmonary disease (COPD) and bronchitis, allergic diseases, such as allergic rhinitis and atopic dermatitis, cystic fibrosis, lung allograph rejection, multiple sclerosis, rheumatoid arthritis, juvenile rheumatoid arthritis, osteoarthritis, ankylosing spondylitis, systemic lupus erythematosus, psoriasis, Hashimoto's disease, pancreatisis, autoimmune diabetes, autoimmune ocular disease, ulcerative colitis, Crohn's disease, inflammatory bowel disease (IBS), inflammatory bowel syndrome (IBD), Sjorgen's syndrome, optic neuritis, type I diabetes, neuromyelitis optica, Myasthenia Gravis, uveitis, Guillain-Barre syndrome, psoriatic arthritis, Graves' disease and scleritis.

In one embodiment, compounds of formula (I) or pharmaceutically acceptable salts thereof may be of use in the treatment of inflammatory bowel diseases or multiple sclerosis

In a furthert aspect, the present invention provides a compound of formula(I) or its pharmaceutically acceptable salts, for use in the treatment of cancer of infectious diseases inflammatory and or autoimmune diseases.

In a further aspect, the present invention is directed to a method of treatment of inflammatory and/or autoimmune diseases mediated by NOD1 or NOD1 and NOD2 which comprises administering to a subject in need thereof, a safe and therapeutically effective amount of a compound of formula (I), or a pharmaceutically acceptable salt thereof.

### EXPERIMENTAL

The following Intermediates and Examples illustrate the preparation of compounds of the invention.

In the procedures that follow, after each starting material, reference to a description is typically provided. This is provided merely for assistance to the skilled chemist. The starting material may not necessarily have been prepared from the batch referred to.

The yields were calculated assuming that products were 100% pure if not stated otherwise.

### Abbreviations

The following abbreviations are used in the text

| | | | |
|---|---|---|---|
| °C | degree Celsius | mg | milligrams |
| µg/mL | micrograms per milliters | mg/kg | Milligrams/kilograms |
| µL | microliters | MgSO₄ | magnesium sulfate |
| Azi | aziridine | MHz | megahertz |
| BF₃.OEt₂ | Boron trifluoride diethyl etherate | min | Minutes |
| Bn | benzyl | mL | milliliters |
| Boc | tert-butyloxy carbonyl | mmol | millimoles |
| Br | brome | MS | mass spectrometry |
| C₁₂ | Lauroyl or dodecanoyl | MurNAc | N-Acetyl muramic acid |
| C14 | Myristoyl or tetradecanoyl | Na₂SO₄ | sodium sulfate |
| C₇ | Enantic or heptanoyl | NaHCO₃ | sodium hydrogenocarbonate |
| CBr₄ | carbon tetrabromide | NH₄OH | ammonium hydroxide |
| CD₃OD | deuterated methanol | nm | nanometers |
| CDCl₃ | for deuterated chloroform | NMM | N-4-methylmorpholine |
| d | doublet | NMR | nuclear magnetic resonance |
| D₂O | Deuterium oxide | OD | optical density |
| DCM | dichloromethane | OMe | methoxy |
| DMF | N,N-dimethyl formamide | OxaDAP | (2S, 6R)-2,6-diamino-4-oxa-pimelic acid or *meso*-2,6-diamino-4-oxa-pimelic acid |
| DMSO-*d₆* | deuterated dimethylsulfoxide | Pd/C | Palladium on activated charcoal |
| EDT | 1,2-ethanedithiol | Phe | phenylalanine |
| EDCI or EDC | 1-(3-diethylaminopropyl)-3-ethylcarbodiimide hydrochloride | Pht | Phtalimide |
| ES | electrospray ionization | pNZ | para nitro benzyloxy carbonyl |
| Et₂O | diethyl ether | PPh₃ | triphenyl phosphine |
| EtOAc | ethyl acetate | ppm | parts per million |
| EtOH | ethanol | PyBOP | Benzotriazole-1-yl-oxy-tris-pyrrolidino-phosphonium hexafluorophosphate |
| Fmoc | Fluorenylmethyloxycarbonyl | q | quartet |
| g | grams | rt | room temperature |
| ¹H | proton | s | singlet |
| h | hours | sc | subcutaneous |
| H₂O | water | SEAP | Secreted embryonic alkaline phosphatase |
| H₂SO₄ | sulphuric acid | Ser | serine |
| HATU | O-benzotriazol- 1 -yl-N,N,N',N'-tetramethyluronium hexafluorophosphate | sl | large singlet |
| HCl | hydrochloric acid | t | triplet |
| HEK | Human Embryonic Kidney Cells | THF | tetrahydrofuran |
| Hz | Hertz | TEA | triethylamine |
| | | | |
| iE-LAN | D-Glu-γ-*meso*LANOH | | |
| | | | |
| Lac | Lactoyl or 2-hydroxypropanoyl | TFA | trifluoroacetic acid |
| meso Lan | *meso*-lanthionine | TIPS | triisopropoyl silane |
| m | multiplet | TLC | thin layer chromatography |
| M | molar | Tr | trityl |
| m/z | mass-to-charge ratio | UV | ultra violet |
| MeOH | methanol | Z | benzyloxy carbonyl |
| | | δ | Chemical shift |

Amino acids are designated herein using standard 1-letter or three-letter codes, e.g. as designated in Standard ST.25 in the Handbook On Industrial Property Information and Documentation. The abbreviations for the α-amino acids used in this application are set forth in Table 1.

**Table 1**

| **Amino Acid** | **3-letter Amino acid code** | **1 letter Amino acid code** |
|---|---|---|
| Alanine | Ala | A |
| Cysteine | Cys | C |
| Glutamic acid | Glu | E |
| Glycine | Gly | G |
| Leucine | Leu | L |
| Isoleucine | Ile | I |
| Valine | Val | V |
| Phenylalanine | Phe | F |
| Serine | Ser | S |
| Lanthionine | LAN | - |
| 2,6-diamino pimelic acid | DAP | - |
| 4-oxa-(2, 6)-diamino pimelic acid | OxaDAP | - |

NMR spectra were recorded on a Brucker 300 spectrometer. For 1H (300 MHz) spectra δ values were referenced to CDCl₃ (7.26 ppm), CD₃OD (3.30 ppm), or DMSO-*d₆* (2.50 ppm).

Mass spectral analyses were carried out using an Agilent 6130 MSD simple quadrupole mass spectrometer.

Protected amino acids were purchased from Novabiochem® or Iris Biotech GMBH. All commercially available reagents were used without further purification. All the solvents used for reactions were distilled over appropriate drying reagents prior to use. Commercially available ACS grade solvents (> 99.0% purity) were used for column chromatography without any further purification.

The Example disclosed herein have been named using the naming convention provided with Chem-Draw Ultra 11.0 software, available in Chem. Office.

All reactions and fractions from column chromatography were monitored by thin layer chromatography (TLC) using glass plates with a UV fluorescent indicator (normal SiO2, Merck 60 F254). One or more of the following methods were used for visualization: UV absorption by fluorescence quenching; (Ninhydrin/Ethanol or H₂SO₄/Ethanol) solution. Flash chromatography was performed using Merck type 60, 230-400mesh silica gel. IonS3 exchange chromatography was carried out using Biorad AG 50W-X8 hydrogen form resin.

### Intermediate 1

### Fmoc-Cys(Tr)OBn

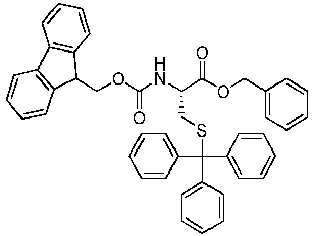

Fmoc-Cys(Trt)OH (5.17 g, 8.83 mmol) was dissolved in dry DMF (50 mL), then cesium carbonate (2.16 g, 6.63 mmol) was added, the mixture was stirred at rt for 15min, and benzyl bromide (1.66g, 9.72 mmol) was added dropwise. The stirring was maintained for 18h. Then, the mixture was diluted with water and extracted with EtOAc 3 times. The organic layer was washed with water, brine, dried over MgSO₄, filtered and concentrated. The crude product was purified by flash column chromatography (silica gel, DCM 100%) to give 5.91g (8.74 mmol, 99% yield).the title compound. ¹H NMR (CDCl₃) δ 7.71 (2H, d), 7.55 (2H,d), 7.37-7.11 (25H, m), 5.10 (2H, s), 4.31 (3H, m), 4.16 (1H, t), 2.56 (2H,m).

### Intermediate 2

### Fmoc-CysOBn

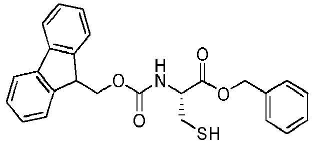

Intermediate 1 (3 g, 4.44 mmol) was dissolved in TFA/DCM mixture (1/1) (17 mL) and TIPS (2.32 g, 14.65 mmol) was added. After stirring for 1h at rt, the solvents were removed under vacuo, coevaporated with toluene. The compound was triturated with Petroleum ether twice to provide 1.65 g (3.81 mmol, 86% yield) of the title compound. ¹H NMR (CDCl₃) δ 7.72 (2H, d), 7.55 (2H, d), 7.36-7.07 (10H, m), 5.15 (2H, s) 4.65 (1H, m), 4.37 (2H, d), 4.17 (1H, t), 2.96 (2H, m), 1.20 (1H, t).

### Intermediate 3

### Z-D-SerOBn

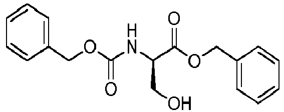

Z-D-SerOH (10.22 g, 42.72 mmol) was dissolved in dry DMF (100 mL), then cesium carbonate (8.35 g, 25.64 mmol) was added, the mixture was stirred at rt for 15min, and benzyl bromide (7.67 g, 44.86 mmol) was added dropwise. The stirring was maintained for 18h. Then, the mixture was diluted with water and extracted with ethyl acetate 3 times. The organic layer was washed with water, brine, dried over MgSO₄, filtered and concentrated. The crude product was purified by flash column chromatography [silica gel, DCM/MeOH (1%)] to give 13.01 g (39.50 mmol, 92% yield) of the title compound. ¹H NMR (DMSO-*d₆*) δ 7.51 (1H, d), 7.28 (10H, m), 5.07 (2H, s), 4.98 (2H, s), 4.91 (1H, t), 4.13 (1H, m), 3.62 (2H, t).

### Intermediate 4

### Z-D-Ala(Br)OBn

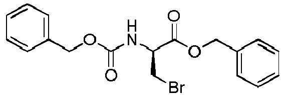

The intermediate 3 (6.33 g, 19.22 mmol) and CBr₄ (10.20 g, 30.75 mmol) were dissolved under argon in dry DCM (180 mL) and cooled to 0°C. To the stirred solution was added PPh₃ (10.08 g, 38.44 mmol) in several portions within 1h. The solution was allowed to warm to rt and stirred for an additional 2h. The solvent was diluted with DCM, washed twice with saturated NaHCO₃, water and brine. The organic layer was dried over MgSO₄, filtered and evaporated. The crude compound was purified by flash column chromatography column (silica gel, Petroleum ether/ethyl acetate 10/1 to 8/2) to give 4.85 g (12.36 mmol, 64% yield) of the title compound. ¹H NMR (DMSO-*d₆*) δ 7.95 (1H, d), 7.29 (10H, m), 5.13 (2H, s), 5.00 (2H, s), 4.47 (1H, m), 3.76-3.62 (2H, m).

### Intermediate 5

### Fmoc-mesoLAN(Z, OBn)OBn

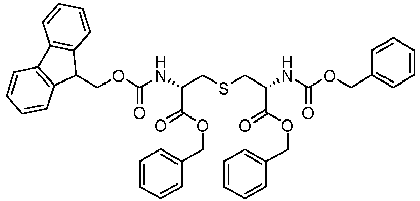

To a solution of intermediate 2 (2.6 g, 5.99 mmol) and bromoserine derivative 4 (2.47 g, 6.29 mmol) in EtOAc (90 mL) a pH 8.5 solution of NaHCO₃ (90 mL) was added, followed by addition of TBAHS (8.14 g, 23.99 mmol). The mixture was vigorously stirred at rt overnight. Then, the mixture was diluted with EtOAc (150 mL) and was washed successively with saturated NaHCO₃, water and brine. The organic layer was dried over MgSO₄, filtered and concentrated in vacuo. The residue was purified by flash column chromatography (silica gel, DCM/MeOH 0 to 2%) to give 2.55 g (3.42 mmol, 57% yield) of the title compound. ¹H NMR (DMSO-*d₆*) δ 7.86 (2H, d), 7.63 (2H, d), 7.36-7.26 (20H, m), 5.06 (2H, s), 5.05 (2H, s), 4.96 (2H, s), 4.23-4.14 (5H, m), 2.90-2.77 (4H, m).

### Intermediate 6

### Fmoc-Cys(Tr)OMe

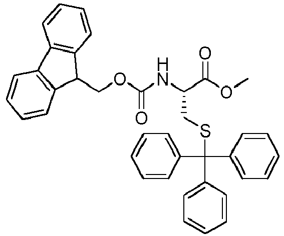

Fmoc-Cys(Tr)OH (3.0 g, 5.12 mmol) was dissolved in dry DMF (20 mL), then cesium carbonate (830 mg, 2.55 mmol) was added, the mixture was stirred at rt for 15min, then the mixture was cooled to 0°C and methyl iodide (727 mg, 5.12 mmol) was added dropwise. The stirring was maintained for 18h. Then, the solvent was removed in vacuo and the residue was diluted with EtOAc (50 mL) and the organic layer was washed with water and brine dried over MgSO₄, filtered and concentrated. The crude product was purified by flash column chromatography (silica gel, DCM 100%) to give 2.84 g (4.74 mmol, 92% yield) of the title compound. ¹H NMR (CDCl₃-*d₁*) δ 7.72 (2H, d), 7.62 (2H, d), 7.43 (4H, m), 7.33-7.20 (15H, m), 5.24 (1H, d) 4.38 (3H, m), 4.24 (1H, m), 3.74 (3H, s), 2.68 (2H, d).

### Intermediate 7

### Fmoc-CysOMe

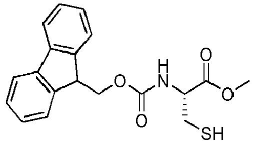

The intermediate 6 (1.8 g, 3.01 mmol) was dissolved in TFA/DCM mixture (1/1) (30 mL) and TIPS (1.57 g, 9.90 mmol) was added. After stirring for 4h at rt, the solvents were removed under vacuo, coevaporated with toluene. The compound was triturated with a mixture of petroleum ether/Et₂O (2/1) the precipitate was filtrated to provide 1.06 g (2.96 mmol, 98% yield) of the title compound. NMR (CDCl₃-*d₁*) δ 7.78 (2H, d), 7.65 (2H, d), 7.41-7.30 (4H, m), 5.70 (1H, d) 4.70 (1H, m), 4.45 (2H, m), 4.26 (1H, t), 3.78 (3H, s), 3.02 (2H, d).

### Intermediate 8

### BocD-Ala(Br)OMe

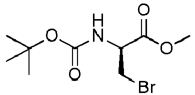

The Boc-D-SerOMe (4.43 g, 20.2 mmol) and CBr₄ (11.0 g, 33.23 mmol) were dissolved under argon in dry DCM (100 mL) and cooled to 0°C. To the stirred solution was added PPh₃ (11.0 g, 41.98 mmol) in several portions within 1h. The solution was allowed to warm to rt and stirred overnight. The solvent was diluted with DCM, washed twice with saturated NaHCO₃, water and brine. The organic layer was dried over MgSO₄, filtered and evaporated. The crude compound was purified by flash column chromatography column (silica gel, Petroleum ether/DCM 7/3 to 100% of DCM) to give 4.12 g (14.60 mmol, 72% yield) of the title compound. ¹H NMR (DMSO-*d₆*) δ 7.63 (1H, d), 4.42 (1H, m), 3.92-3.56 (2H, m), 3.68 (3H, s), 1.43 (9H, s).

### Intermediate 9

### Fmoc-mesoLAN(Boc, OMe)OMe

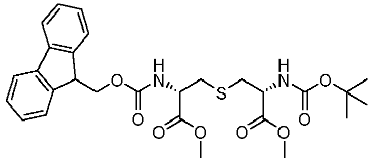

To a solution of intermediate 7 (970 mg, 2.71 mmol) and bromoserine derivative 8 (924 mg, 2.85 mmol) in EtOAc (30 mL) a pH 8.5 solution of NaHCO₃ (30 mL) was added, followed by addition of TBAHS (3.70 g, 10.91 mmol). The mixture was vigorously stirred at rt overnight. Then, the mixture was diluted with EtOAc (100 mL) and was washed successively with saturated NaHCO₃, water and brine. The organic layer was dried over MgSO₄, filtered and concentrated in vacuo. The residue was purified by flash column chromatography (silica gel, DCM/MeOH 0 to 2%) to give 1.20 g (2.00 mmol, 74% yield) of the title compound. ¹H NMR (DMSO-*d₆*) δ 7.90 (2H, d), 7.72 (2H, d), 7.45-7.31 (4H, m), 4.33-4.24 (4H, m), 3.96 (1H, m), 3.68 (3H, s), 3.66 (3H, s), 3.00-2.68 (4H, m), 1.45 (9H, s).

### Intermediate 10

### H-mesoLAN(Boc, OMe)OMe

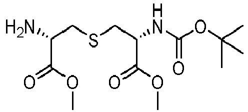

Intermediate 9 (503 mg, 0.84 mmol) was dissolved with DMF (10 mL), piperidine (15 mg, 0.16 mmol) was added dropwise , and the mixture was heated at 50°C for 1h. Then, water (25 mL) was added, and the mixture was extracted with EtOAc three times. The organic layer was washed with water (3 times) and brine, dried over MgSO₄, filtered and concentrated in vacuo. The crude compound was purified by flash column chromatography (silica gel, DCM/MeOH 0 to 5%) to provide 222 mg (0.58 mmol, 69% yield) of the title compound. ¹H NMR (DMSO-*d₆*) δ 4.18 (1H, m), 3.63 (3H, s), 3.61 (3H, s), 3.53 (1H, t), 2.82-2.73 (4H, m), 1.38 (9H, s).

### Intermediate 11

### H-mesoLAN(Z, OBn)OBn

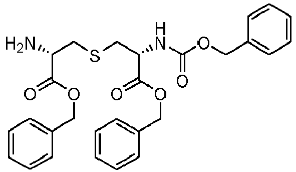

The intermediate 5 (2.41 g, 3.23 mmol) was dissolved with DMF (25 mL), piperidine (55 mg, 0.65 mmol) was added dropwise , and the mixture was heated at 50°C for 45min. Then, water (25 mL) was added, and the mixture was extracted with EtOAc three times. The organic layer was washed with water (3 times) and brine, dried over MgSO₄, filtered and concentrated in vacuo. The crude compound was purified by flash column chromatography (silica gel, DCM/MeOH 0 to 2%) to provide 1.68 g (3.21 mmol, 99% yield) of the title compound. ¹H NMR (DMSO-*d₆*) δ 7.28-7.23 (15H, m), 5.08 (2H, s), 5.03 (2H, s), 4.97 (2H, s), 4.21 (1H, m), 2.94-2.68 (4H, m).

### Intermediate 12

### Boc-D-GluOBn-γ-mesoLAN(Z, OBn)OBn

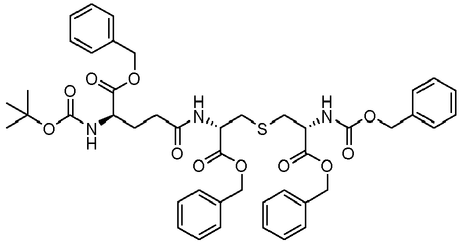

To a solution of intermediate 11 (1.67 g, 3.2 mmol) in dry DCM (30 mL) were added Boc-D-GluOBn (1.08 g, 3.2 mmol) and PyBOP (1.83 g, 3.53 mmol), the pH was adjusted to 10 with NMM. The reaction mixture was stirred at rt under argon for 16h. Then the reaction was diluted with DCM (100 mL), and washed twice with a saturated solution of ammonium chloride. The organic layer was dried over MgSO₄, filtrated and concentrated in vacuo. The crude product was purified by flash column chromatography (silica gel, DCM/MeOH 0 to 1%) to give 2.70 g (3.2 mmol, 100% yield) of the title compound. ¹H NMR (CDCl₃) δ 7.28-7.26 (20H, m), 6.67 (1H, m), 5.61 (1H, m), 5.36 (1H, m), 5.24 (2H, s), 5.13 (2H, s), 5.08 (2H, s), 5.05 (2H, s), 4.75 (1H, m), 4.52 (1H, m), 4.34 (1H, m), 2.88 (4H, m), 2.21 (2H, m), 2.19-1.85 (2H, m), 1.36 (9H, s).

### Intermediate 13

### TFA.D-GluOBn-γ-mesoLAN(Z, OBn)OBn

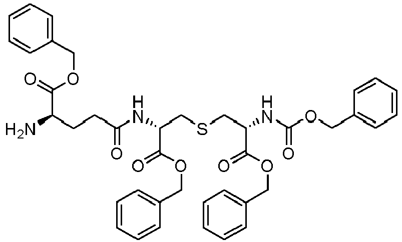

The intermediate **12** (2.70 g, 3.2 mmol) was dissolved in TFA/DCM mixture (1/1) (40 mL). After stirring for 1h at rt, the solvents were removed under vacuo, the residue was coevaporated 3 times with toluene. The crude compound was purified by flash column chromatography (silica gel, DCM/MeOH 0 to 3%) to give 2.15 g (2.51 mmol, 78% yield) of the title compound. ¹H NMR (DMSO-*d₆*) δ 8.46 (1H, d), 7.82 (1H, t), 7.36-7.23 (20H, m), 5.22-4.96 (8H, m), 4.45 (1H, m), 4.21 (1H, m), 4.07 (1H, t), 2.95-2.86 (2H, m), 2.82-2.66 (2H, m), 2.26 (2H, m), 1.94 (2H, m).

### Intermediate 14

### C₁₂-D-GluOBn-γ-mesoLAN(Z, OBn)OBn

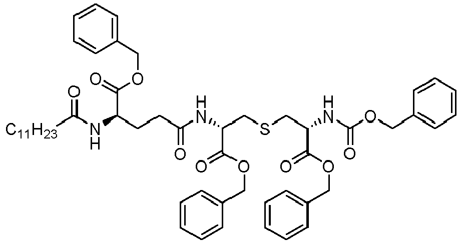

To a solution of intermediate **13** (125 mg, 0.15 mmol) in dry DCM under argon atmosphere was added TEA (26 µL, 0.19 mmol). The mixture was cooled to 0°C and a solution of lauroyl chloride (35 mg, 0.16 mmol) in dry DCM (2 mL) was added dropwise. The solution was allowed to warm to rt and stirred overnight. The reaction was diluted in DCM (20 mL), washed with NaHCO₃, 1N HCl, water, and brine. The organic layer was dried over Mg2SO4, filtered and concentrated in vacuo. The crude product was purified by flash column chromatography (silica gel, DCM/MeOH 0 to 1%) to provide 87 mg (0.094 mmol, 65% yield) of the title compound. ¹H NMR (CDCl₃) δ 7.36-7.35 (20H, m), 7.13 (1H, m), 6.55 (1H, m), 5.88 (1H, m), 5.18 (6H, s), 5.14 (2H, s), 4.84 (1H, m), 4.73 (1H, m), 4.62 (1H, m), 2.99 (4H, m), 2.33 (5H, m), 2.00 (1H, m), 1.30 (18H, m), 0.92 (3H, t).

### Intermediate 15

### C₁₄-D-GluOBn-γ-mesoLAN(Z, OBn)OBn

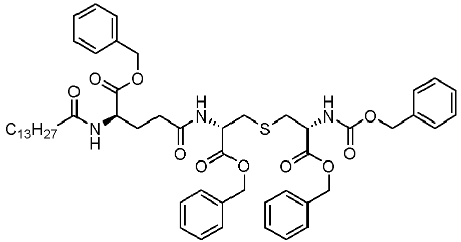

To a solution of compound **13** (123 mg, 0.14 mmol) in dry DCM under argon atmosphere was added TEA (29 µL, 0.21 mmol). The mixture was cooled to 0°C and a solution of myristoyl chloride (39 mg, 0.16 mmol) in dry DCM (2 mL) was added dropwise. The solution was allowed to warm to rt and stirred overnight. The reaction was diluted in DCM (20 mL), washed with NaHCO₃, 1N HCl, water, and brine. The organic layer was dried over Mg2SO4, filtered and concentrated in vacuo. The crude product was purified by flash column chromatography (silica gel, DCM/MeOH 0 to 1%) to provide 128 mg (0.13 mmol, 93% yield) of the title compound. ¹H NMR (DMSO-*d₆*) δ 8.43 (1H, d), 8.23 (1H, d), 7.90 (1H, d), 7.36-7.35 (20H, m), 5.14 (4H, s), 5.12 (2H, s), 5.06 (2H, s), 4.54 (1H, m), 4.29 (2H, m), 4.62 (1H, m), 2.96 (2H, m), 2.84 (2H,m), 2.24 (2H, m), 2.11 (2H, m), 2.00 (1H, m), 1.85 (1H, m), 1.47 (2H,m), 1.24 (20H, m), 0.86 (3H, t).

### Intermediate 16

### Boc-D-Glu(OBn)OMe

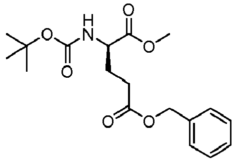

Boc-D-γGlu(OBn)OH (5.0 g, 14.84 mmol) was dissolved in dry DMF (50 mL), then potassium carbonate (2.32 g, 16.81 mmol) was added, the mixture was stirred at rt for 15 min, then the mixture was cooled to 0°C and methyl iodide (4.21 g, 29.64 mmol) was added dropwise. The stirring was maintained for 18h. Then, the solvent was removed in vacuo and the residue was diluted with EtOAc (50 mL) and the organic layer was washed with water and brine dried over MgSO₄, filtered and concentrated. The crude product was purified by flash column chromatography (silica gel, DCM/MeOH 0 to 1%) to give 5.12 g (14.58 mmol, 98% yield) of the title compound. ¹H NMR (DMSO-*d₆*) δ 7.38-7.31 (5H, m), 7.30 (1H, d), 5.09 (2H, s), 4.00 (1H, m), 3.62 (3H, s), 2.49 (2H, m), 1.99 (1H, m), 1.81 (1H, m), 1.38 (9H,s).

### Intermediate 17

### TFA. D-Glu(OBn)OMe

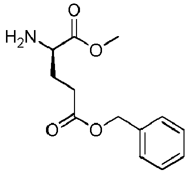

Intermediate 16 (5.1 g, 14.52 mmol) was dissolved in TFA/DCM mixture (3/1) (20 mL). After stirring for 2h at rt, the solvents were removed under vacuo; the residue was coevaporated 3 times with toluene. The crude compound was triturated with Et₂O to provide 5.19 g (14.22 mmol, 98% yield) of the title compound. ¹H NMR (DMSO-*d₆*) δ 8.45 (2H, s), 7.39-7.36 (5H, m), 5.11 (2H, s), 4.10 (1H, m), 3.62 (3H, s), 2.65 (2H, m), 2.07 (2H, m).

### Intermediate 18

### C₇-D-Glu(OBn)OMe

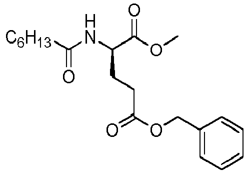

To a solution of intermediate 18 (1.37 g, 3.75 mmol) in dry DCM (20 mL) under argon atmosphere was added TEA (843 µL, 6.00 mmol). The mixture was cooled to 0°C and a solution of heptanoyl chloride (613 mg, 4.13 mmol) in dry DCM (2 mL) was added dropwise. The solution was allowed to warm to rt and stirred overnight. The reaction was diluted in DCM (50 mL), washed with NaHCO₃, 1N HCl, water, and brine. The organic layer was dried over Mg2SO4, filtered and concentrated in vacuo. The crude product was purified by flash column chromatography (silica gel, DCM/MeOH 0 to 1%) to provide 1.13 g (3.11 mmol, 83% yield) of the title compound. ¹H NMR (DMSO-*d₆*) δ 8.19 (1H, d), 7.36 (5H, s), 5.09 (2H, s), 4.28 (1H, m), 3.61 (3H, s), 2.45 (2H, m), 2.08 (2H, m), 2.00 (1H, m), 1.85 (1H, m), 1.52 (2H, m), 1.25 (6H, m), 0.89 (3H, t).

### Intermediate 19

### C₇-D-GluOMe

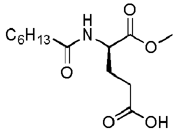

Intermediate 18 (1.13 g, 3.11 mmol) was dissolved in a mixture of THF/MeOH mixture (1/1) (20 mL) palladium 10% on carbon (165 mg) was added and the mixture was stirred underhydrogen atmosphere for 2h. Then, the mixture was filtered over Celite, the celite was washed with MeOH. The filtrate was concentrated in vacuo to give 840 mg (1.13 mmol, 100% yield) of the title compound. This compound was used for the next step without any further purification. ¹H NMR (DMSO-*d₆*) δ 12.02 (1H, s), 8.16 (1H, d), 4.27 (1H, m), 3.17 (3H, s), 2.28 (2H, m), 2.11 (2H, m), 1.95 (1H, m), 1.75 (1H, m), 1.48 (2H, m), 1.25 (6H, m), 0.86 (3H, t).

### Intermediate 20

### C₇-D-GluOMe-γ-Lan(Boc, OMe)OMe

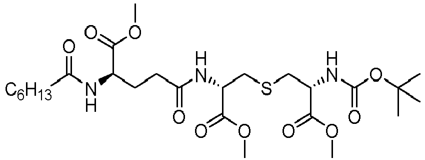

To a solution of intermediate 19 (80 mg, 0.29 mmol) in dry DMF (10 mL) were added compound 10 (113 mg, 0.34 mmol) HATU (120 mg, 0.32 mmol), and NMM (88 mg, 0.87 mmol). The reaction mixture was stirred at rt under argon for 16h. Then the reaction was diluted with DCM (100 mL), and washed twice with a saturated solution of ammonium chloride. The organic layer was dried over MgSO₄, filtrated and concentrated in vacuo. The crude product was purified by flash column chromatography (silica gel, DCM/MeOH 0 to 2%) to give 86 mg (0.14 mmol, 50% yield) of the title compound. ¹H NMR (DMSO-*d₆*) δ 8.34 (1H, d), 8.14(1H, d), 7.29 (1H, d), 4.44 (1H, m), 4.20 (2H, m), 3.67 (3H, s), 3.65 (3H, s), 2.92-2.69 (4H, m), 2.27 (2H, m), 2.13 (2H, m), 1.94 (1H, m), 1.81 (1H, m), 1.50 (2H, m), 1.37 (9H, m), 1.28 (6H, s), 0.86 (3H, t).

### Intermediate 21

### Boc-Ala-D-GluOBn-γ-mesoLAN(Z, OBn)Obn

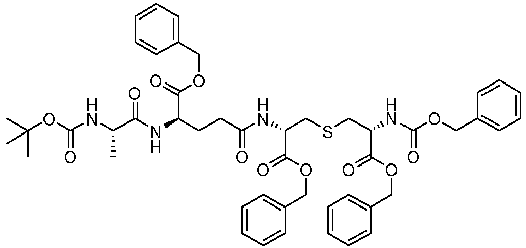

To a solution of intermediate 13 (148 mg, 0.17 mmol) in dry DCM (5 mL) were added BocAlaOH (36 mg, 0.19 mmol) and PyBOP (98 mg, 0.19 mmol), the pH was adjusted to 10 with NMM. The reaction mixture was stirred at rt under argon for 16h. Then the reaction was diluted with DCM (15 mL), and washed twice with a saturated solution of ammonium chloride. The organic layer was dried over MgSO₄, filtrated and concentrated in vacuo. The crude product was purified by flash column chromatography (silica gel, DCM/MeOH 0 to 1%) to give 134 mg (0.15 mmol, 85% yield) of the title compound. ¹H NMR (CDCl₃) δ 7.36-7.32 (20H, m), 7.18 (2H, m), 5.97 (1H, m), 5.61 (1H, m), 5.17 (4H, s), 5.13 (2H, s), 5.12 (2H, s), 5.05 (2H, s), 4.82 (1H, m), 4.62 (1H, m), 4.24 (1H, m), 2.99 (4H, m), 2.28 (2H, m), 2.15-2.00 (2H, m), 1.46 (9H, s), 1.33 (3H, d).

### Intermediate 22

### TFA.Ala-D-GluOBn-γ-mesoLAN(Z, OBn)OBn

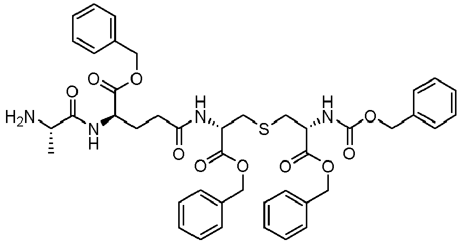

The intermediate 21 (130 mg, 0.14 mmol) was dissolved in TFA/DCM mixture (1/1) (10 mL). After stirring for 1h at rt, the solvents were removed under vacuo; the residue was coevaporated 3 times with toluene. The crude compound was purified by flash column chromatography (silica gel, DCM/MeOH 0 to 5%) to give 115 mg (0.12 mmol, 83% yield) of the title compound. ¹H NMR (DMSO-*d₆*) δ 8.87 (1H, d), 8.47 (1H, d), 8.13 (1H, d), 7.90 (1H, d), 7.38-7.35 (20H, m), 5.21-5.06 (8H, m), 4.52 (1H, m), 4.39 (1H, m), 4.30 (1H, m), 3.89 (1H,m), 3.00-2.75 (4H, m), 2.25 (2H, m), 2.05 (1H,m), 1.87 (1H, m), 1.38 (3H, d).

### Intermediate 23

### Boc-Leu-D-Glu(OBn)OMe

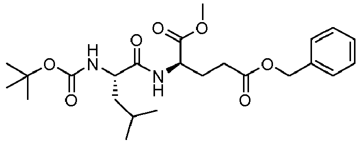

To a solution of intermediate 17 (800 mg, 2.19 mmol) in dry DMF (20 mL) were added BocLeuOH (337 mg, 1.46 mmol) and HATU (610 mg, 1.60 mmol), the pH was adjusted to 10 with NMM (443 mg, 4.38 mmol). The reaction mixture was stirred at rt under argon for 3h. Then the reaction was diluted with EtOAc (70 mL), and washed twice with a saturated solution of ammonium chloride. The organic layer was dried over MgSO₄, filtrated and concentrated in vacuo. The crude product was purified by flash column chromatography (silica gel, DCM/MeOH 0 to 1%) to give 499 mg (1.07 mmol, 73% yield) of the title compound. ¹H NMR (DMSO-*d₆*) δ 8.20 (1H, d), 7.35 (5H, m), 6.80 (1H, m), 5.13 (2H, s), 4.27 (1H, m), 4.00 (1H, m), 3.67 (3H, s), 2.44 (2H, m), 2.00 (1H, m), 1.87 (1H, m), 1.59 (1H, m), 1.46 (11H, m), 0.87 (3H, d).

### Intermediate 24

### Boc-Ile-D-Glu(Obn)OMe

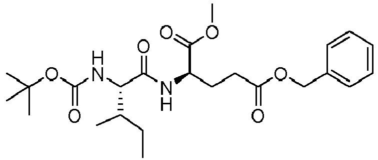

To a solution of intermediate 17 (800 mg, 2.19 mmol) in dry DMF (20 mL) were added BocIleOH (337 mg, 1.46 mmol) and HATU (610 mg, 1.60 mmol), the pH was adjusted to 10 with NMM (443 mg, 4.38 mmol). The reaction mixture was stirred at rt under argon for 3h. Then the reaction was diluted with EtOAc (70 mL), and washed twice with a saturated solution of ammonium chloride. The organic layer was dried over MgSO₄, filtrated and concentrated in vacuo. The crude product was purified by flash column chromatography (silica gel, DCM/MeOH 0 to 1%) to give 662 mg (1.42 mmol, 97% yield) of the title compound. ¹H NMR (DMSO-*d₆*) δ 8.25 (1H, d), 7.35 (5H, m), 6.63 (1H, d), 5.11 (2H, s), 4.26 (1H, m), 3.83 (1H, m), 3.62 (3H, s), 2.43 (2H, m), 2.04 (1H, m), 1.87 (1H, m), 1.69 (1H, m), 1.24 (11H, m), 0.79 (6H, m).

### Intermediate 25

### Boc-Val-D-Glu(OBn)OMe

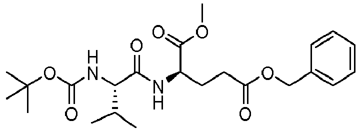

To a solution of intermediate 17 (800 mg, 2.19 mmol) in dry DMF (20 mL) were added BocValOH (317 mg, 1.46 mmol) and HATU (610 mg, 1.60 mmol), the pH was adjusted to 10 with NMM (443 mg, 4.38 mmol). The reaction mixture was stirred at rt under argon for 3h. Then the reaction was diluted with EtOAc (70 mL), and washed twice with a saturated solution of ammonium chloride. The organic layer was dried over MgSO₄, filtrated and concentrated in vacuo. The crude product was purified by flash column chromatography (silica gel, DCM/MeOH 0 to 1%) to give 608 mg (1.35 mmol, 92% yield) of the title compound. ¹H NMR (DMSO-*d₆*) δ 8.24 (1H, d), 7.38 (5H, m), 6.60 (1H, d), 5.08 (2H, s), 4.28 (1H, m), 3.79 (1H, m), 3.62 (3H, s), 2.44 (2H, m), 1.99-1.83 (3H, m), 1.38 (9H, s), 0.83 (6H, m).

### Intermediate 26

### Boc-Phe-D-Glu(OBn)OMe

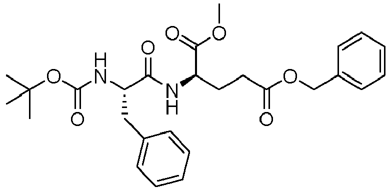

To a solution of intermediate 17 (800 mg, 2.19 mmol) in dry DMF (20 mL) were added BocPheOH (387 mg, 1.46 mmol) and HATU (610 mg, 1.60 mmol), the pH was adjusted to 10 with NMM (443 mg, 4.38 mmol). The reaction mixture was stirred at rt under argon for 3h. Then the reaction was diluted with EtOAc (70 mL), and washed twice with a saturated solution of ammonium chloride. The organic layer was dried over MgSO₄, filtrated and concentrated in vacuo. The crude product was purified by flash column chromatography (silica gel, DCM/MeOH 0 to 1%) to give 704 mg (1.41 mmol, 96% yield) of the title compound. ¹H NMR (DMSO-*d₆*) δ 8.33 (1H, d), 7.34 (5H, m), 7.24 (4H, m), 7.13 (1H, m), 6.87 (1H, d), 5.09 (2H, s), 4.28 (1H, m), 4.22 (1H, m), 3.67 (3H, s), 2.92 (1H, m), 2.74 (1H, m), 2.29 (2H, m), 1.95 (1H, m), 1.84 (1H, m), 1.32 (9H, s).

### Intermediate 27

### Boc-Leu-D-GluOMe

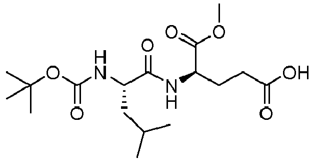

Intermediate 23 (150 g, 0.32 mmol) was dissolved in a mixture of THF/MeOH mixture (1/1) (10 mL) palladium 10% on carbon (17 mg) was added and the mixture was stirred under hydrogen atmosphere for 2h. Then, the mixture was filtered over Celite, the celite was washed with MeOH. The filtrate was concentrated in vacuo to give 120 mg (0.32 mmol, 99% yield) of the title compound. This compound was used for the next step without any further purification.

### Intermediate 28

### Boc-Ile-D-GluOMe

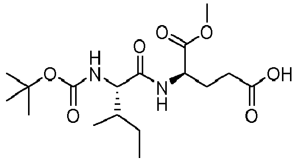

Intermediate 24 (150 g, 0.32 mmol) was dissolved in a mixture of THF/MeOH mixture (1/1) (10 mL) palladium 10% on carbon (17 mg) was added and the mixture was stirred under hydrogen atmosphere for 2h. Then, the mixture was filtered over Celite, the celite was washed with MeOH. The filtrate was concentrated in vacuo to give 118 mg (0.31 mmol, 97% yield) of the title compound. This compound was used for the next step without any further purification.

### Intermediate 29

### Boc-Val-D-GluOMe

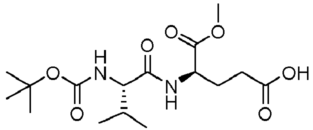

Intermediate 25 (150 g, 0.33 mmol) was dissolved in a mixture of THF/MeOH mixture (1/1) (10 mL) palladium 10% on carbon (17 mg) was added and the mixture was stirred under hydrogen atmosphere for 2h. Then, the mixture was filtered over Celite, the celite was washed with MeOH. The filtrate was concentrated in vacuo to give 120 mg (0.33 mmol, 100% yield) of the title compound. This compound was used for the next step without any further purification.

### Intermediate 30

### Boc-Phe-D-GluOMe

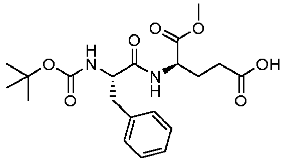

Intermediate 26 (150 g, 0.30 mmol) was dissolved in a mixture of THF/MeOH mixture (1/1) (10 mL) palladium 10% on carbon (17 mg) was added and the mixture was stirred under hydrogen atmosphere for 2h. Then, the mixture was filtered over Celite, the celite was washed with MeOH. The filtrate was concentrated in vacuo to give 120 mg (0.30 mmol, 100% yield) of the title compound. This compound was used for the next step without any further purification.

### Intermediate 31

### Boc-Leu-D-GluOMe-γ-mesoLAN(Boc, OMe)OMe

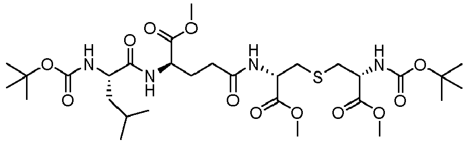

To a solution of intermediate 10 (70 mg, 0.21 mmol) in dry DMF (10 mL) were added intermediate 27 (117 mg, 0.31 mmol), HATU (87 mg, 0.22 mmol), the pH was adjusted to 9 with NMM (63 mg, 0.62 mmol). The reaction mixture was stirred at rt under argon for 16h. Then the reaction was diluted with EtOAc (60 mL), and washed twice with a saturated solution of ammonium chloride. The organic layer was dried over MgSO₄, filtrated and concentrated in vacuo. The crude product was purified by flash column chromatography (silica gel, DCM/MeOH 0 to 2%) to give 65 mg (0.09 mmol, 45% yield) of the title compound. ¹H NMR (DMSO-*d₆*) δ 8.38 (1H, d), 8.26 (1H, d), 7.32 (1H, d), 6.59 (1H, d), 4.44 (1H, m), 4.16 (2H, m), 3.86 (1H, m), 3.65 (3H, s), 3.64 (3H, s), 3.63 (3H, s), 2.92-2.74 (4H, m), 2.24 (2H, m), 1.94 (3H, m), 1.84 (18H, m), 0.82 (6H, m).

### Intermediate 32

### Boc-Ile-D-GluOMe-γ-mesoLAN(Boc, OMe)OMe

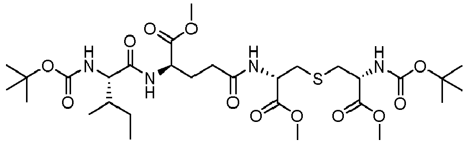

To a solution of intermediate 10 (70 mg, 0.21 mmol) in dry DMF (10 mL) were added intermediate 28 (117 mg, 0.31 mmol), HATU (87 mg, 0.22 mmol), the pH was adjusted to 9 with NMM (63 mg, 0.62 mmol). The reaction mixture was stirred at rt under argon for 16h. Then the reaction was diluted with EtOAc (60 mL), and washed twice with a saturated solution of ammonium chloride. The organic layer was dried over MgSO₄, filtrated and concentrated in vacuo. The crude product was purified by flash column chromatography (silica gel, DCM/MeOH 0 to 1%) to give 100 mg (0.15 mmol, 70% yield) of the title compound. ¹H NMR (DMSO-*d₆*) δ 8.38 (1H, d), 8.24 (1H, d), 7.33 (1H, m), 6.62 (1H, t), 4.45 (1H, m), 4.25-4.13 (2H, m), 3.83 (1H, m), 3.65 (3H, s), 3.64 (3H, s), 3.63 (3H, s), 2.91-2.71 (4H, m), 2.34 (1H, m), 2.20 (1H, m), 1.94 (1H, m), 1.83 (1H, m), 1.65 (1H, m), 1.35 (18H, m), 1.10 (1H, m), 0.82 (6H, m).

### Intermediate 33

### Boc-Val-D-GluOMe-γ-mesoLAN(Boc, OMe)OMe

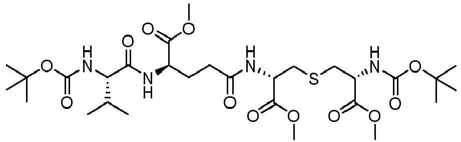

To a solution of intermediate 10 (70 mg, 0.21 mmol) in dry DMF (10 mL) were added intermediate 29 (112 mg, 0.31 mmol), HATU (87 mg, 0.22 mmol), the pH was adjusted to 9 with NMM (63 mg, 0.62 mmol). The reaction mixture was stirred at rt under argon for 16h. Then the reaction was diluted with EtOAc (60 mL), and washed twice with a saturated solution of ammonium chloride. The organic layer was dried over MgSO₄, filtrated and concentrated in vacuo. The crude product was purified by flash column chromatography (silica gel, DCM/MeOH 0 to 1%) to give 65 mg (0.09 mmol, 45% yield) of the title compound. ¹H NMR (DMSO-*d₆*) δ 8.38 (1H, d), 8.24 (1H, d), 7.33 (1H, m), 6.62 (1H, t), 4.45 (1H, m), 4.25-4.13 (2H, m), 3.83 (1H, m), 3.65 (3H, s), 3.64 (3H, s), 3.63 (3H, s), 2.92-2.61 (4H, m), 2.22 (2H, m), 1.94 (2H, m), 1.45 (18H, m), 0.85 (6H, m).

### Intermediate 34

### Boc-Phe-D-GluOMe-γ-mesoLAN(Boc, OMe)OMe

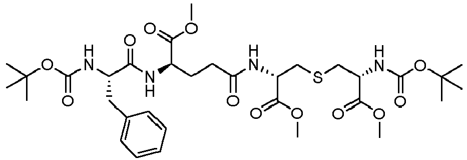

To a solution of intermediate 10 (70 mg, 0.21 mmol) in dry DMF (10 mL) were added intermediate 30 (127 mg, 0.31 mmol), HATU (87 mg, 0.22 mmol), the pH was adjusted to 9 with NMM (63 mg, 0.62 mmol). The reaction mixture was stirred at rt under argon for 16h. Then the reaction was diluted with EtOAc (60 mL), and washed twice with a saturated solution of ammonium chloride. The organic layer was dried over MgSO₄, filtrated and concentrated in vacuo. The crude product was purified by flash column chromatography (silica gel, DCM/MeOH 0 to 1%) to give 147 mg (0.20 mmol, 97% yield) of the title compound. ¹H NMR (DMSO-*d₆*) δ 8.36 (1H, m), 7.33-7.19 (5H, m), 6.87 (1H, t), 4.47 (1H, m), 4.28-4.15 (2H, m), 3.65 (3H, s), 3.64 (3H, s), 3.26 (3H, s), 2.98-2.66 (4H, m), 2.32 (1H, m), 2.18 (1H, m), 1.98 (1H, m), 1.80 (1H, m), 1.45 (18H, m).

### Intermediate 35

### TFA.Leu-D-Glu(OBn)OMe

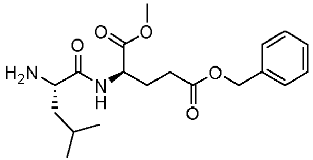

The intermediate 23 (350 mg, 0.75 mmol) was dissolved in DCM (10 mL) and TFA (581 µL, 7.53 mmol) was added. After stirring for 1h at rt, the solvents were removed under vacuo; the residue was coevaporated 3 times with toluene. The title compound was used for the next step without any further purification.

### Intermediate 36

### TFA.Ile-D-Glu(OBn)OMe

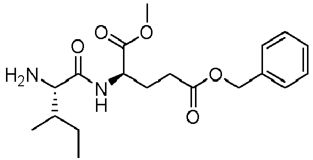

The intermediate 24 (500 mg, 1.07 mmol) was dissolved in DCM (10 mL) and TFA (830 µL, 10.76 mmol) was added. After stirring for 1h at rt, the solvents were removed under vacuo; the residue was coevaporated 3 times with toluene. The title compound was used for the next step without any further purification.

### Intermediate 37

### TFA.Val-D-Glu(OBn)OMe

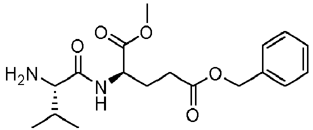

The intermediate 25 (450 mg, 0.99 mmol) was dissolved in DCM (10 mL) and TFA (770 µL, 9.98 mmol) was added. After stirring for 1h at rt, the solvents were removed under vacuo; the residue was coevaporated 3 times with toluene. The title compound was used for the next step without any further purification.

### Intermediate 38

### TFA.Phe-D-Glu(OBn)OMe

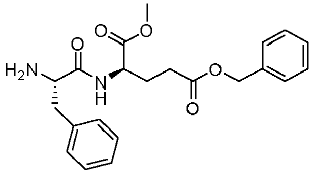

The intermediate 26 (550 mg, 1.10 mmol) was dissolved in DCM (10 mL) and TFA (851 µL, 11.03 mmol) was added. After stirring for 1h at rt, the solvents were removed under vacuo; the residue was coevaporated 3 times with toluene. The title compound was used for the next step without any further purification.

### Intermediate 39

### C₁₄-Leu-D-Glu(OBn)OMe

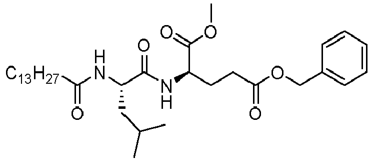

To a solution of intermediate 35 (150 mg, 0.31 mmol) in dry DCM (10 mL) under argon atmosphere was added TEA (132 µL, 0.94 mmol). The mixture was cooled to 0°C and a solution of myristoyl chloride (85 mg, 0.34 mmol) in dry DCM (2 mL) was added dropwise. The solution was allowed to warm to rt and stirred overnight. The reaction was diluted in DCM (50 mL), washed with NaHCO₃, 1N HCl, water, and brine. The organic layer was dried over MgSO₄, filtered and concentrated in vacuo. The crude product was purified by flash column chromatography (silica gel, DCM/MeOH 0 to 1%) to provide 175 mg (0.30 mmol, 97% yield) of the title compound. ¹H NMR (DMSO-*d₆*) δ 8.19 (1H, d), 7.36 (5H, s), 5.09 (2H, s), 4.28 (1H, m), 3.61 (3H, s), 2.45 (2H, m), 2.08 (2H, m), 2.00 (1H, m), 1.85 (1H, m), 1.52 (4H, m), 1.25 (20H, m), 0.89 (9H, m).

### Intermediate 40

### C₁₄-Ile-D-Glu(OBn)OMe

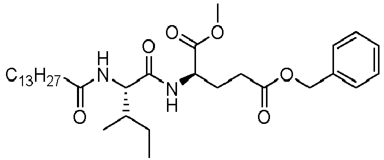

To a solution of intermediate 36 (150 mg, 0.31 mmol) in dry DCM (10 mL) under argon atmosphere was added TEA (132 µL, 0.94 mmol). The mixture was cooled to 0°C and a solution of myristoyl chloride (85 mg, 0.34 mmol) in dry DCM (2 mL) was added dropwise. The solution was allowed to warm to rt and stirred overnight. The reaction was diluted in DCM (50 mL), washed with NaHCO₃, 1N HCl, water, and brine. The organic layer was dried over Mg₂SO₄, filtered and concentrated in vacuo. The crude product was purified by flash column chromatography (silica gel, DCM/MeOH 0 to 1%) to provide 177 mg (0.31 mmol, 98% yield) of the title compound. ¹H NMR (DMSO-*d₆*) δ 8.20 (1H, d), 7.35 (5H, s), 5.10 (2H, s), 4.32 (1H, m), 3.85 (1H, m), 3.61 (3H, s), 2.35 (2H, m), 2.03 (2H, m), 1.98 (1H, m), 1.80 (1H, m), 1.52 (4H, m), 1.24 (20H, m), 0.85 (9H, m).

### Intermediate 41

### C₁₄-Val-D-Glu(OBn)OMe

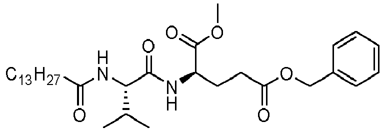

To a solution of intermediate 37 (150 mg, 0.32 mmol) in dry DCM (10 mL) under argon atmosphere was added TEA (136 µL, 0.96 mmol). The mixture was cooled to 0°C and a solution of myristoyl chloride (87 mg, 0.35 mmol) in dry DCM (2 mL) was added dropwise. The solution was allowed to warm to rt and stirred overnight. The reaction was diluted in DCM (50 mL), washed with NaHCO₃, 1N HCl, water, and brine. The organic layer was dried over Mg₂SO₄, filtered and concentrated in vacuo. The crude product was purified by flash column chromatography (silica gel, DCM/MeOH 0 to 1%) to provide 177 mg (0.32 mmol, 97% yield) of the title compound. ¹H NMR (DMSO-*d₆*) δ 8.25 (1H, d), 7.36 (5H, s), 5.08 (2H, s), 4.25-4.13

(3H, m), 3.62 (3H, s), 2.48 (2H, m), 2.05 (2H, m), 2.00 (1H, m), 1.85 (1H, m), 1.45 (2H, m), 1.25 (20H, m), 0.89 (9H, m).

### Intermediate 42

### C₁₄-Phe-D-Glu(OBn)OMe

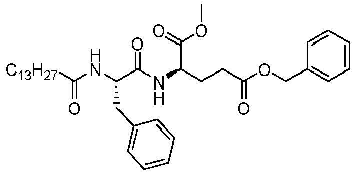

To a solution of intermediate 38 (150 mg, 0.29 mmol) in dry DCM (10 mL) under argon atmosphere was added TEA (123 µL, 0.87 mmol). The mixture was cooled to 0°C and a solution of myristoyl chloride (79 mg, 0.32 mmol) in dry DCM (2 mL) was added dropwise. The solution was allowed to warm to rt and stirred overnight. The reaction was diluted in DCM (50 mL), washed with NaHCO₃, 1N HCl, water, and brine. The organic layer was dried over MgSO₄, filtered and concentrated in vacuo. The crude product was purified by flash column chromatography (silica gel, DCM/MeOH 0 to 1%) to provide 167 mg (0.27 mmol, 94% yield) of the title compound. ¹H NMR (DMSO-*d₆*) δ 8.19 (1H, d), 7.36 (5H, s), 7.33-7.19 (5H, m), 5.10 (2H, s), 4.28 (1H, m), 4.28-4.15 (2H, m), 3.61 (3H, s), 2.55 (2H, m), 2.18 (2H, m), 2.10 (1H, m), 1.95 (1H, m), 1.52 (2H, m), 1.25 (20H, m), 0.89 (3H, t).

### Intermediate 43

### C₁₄-Leu-D-GluOMe

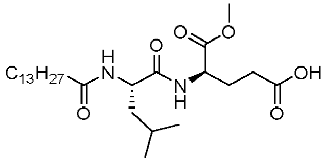

Intermediate 39 (170 g, 0.29 mmol) was dissolved in a mixture of THF/MeOH mixture (1/1) (10 mL) palladium 10% on carbon (16 mg) was added and the mixture was stirred under hydrogen atmosphere for 2h. Then, the mixture was filtered over Celite, the celite was washed with MeOH. The filtrate was concentrated in vacuo to give 143 mg (0.29 mmol, 100% yield) of the title compound. This compound was used for the next step without any further purification.

### Intermediate 44

### C₁₄-Ile-D-GluOMe

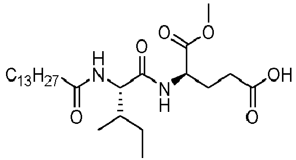

Intermediate 40 (172 g, 0.29 mmol) was dissolved in a mixture of THF/MeOH mixture (1/1) (10 mL) palladium 10% on carbon (16 mg) was added and the mixture was stirred under hydrogen atmosphere for 2h. Then, the mixture was filtered over Celite, the celite was washed with MeOH. The filtrate was concentrated in vacuo to give 145 mg (0.29 mmol, 100% yield) of the title compound. This compound was used for the next step without any further purification.

### Intermediate 45

### C₁₄-Val-D-GluOMe

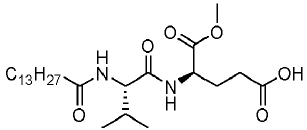

Intermediate 41 (172 g, 0.30 mmol) was dissolved in a mixture of THF/MeOH mixture (1/1) (10 mL) palladium 10% on carbon (16 mg) was added and the mixture was stirred under hydrogen atmosphere for 2h. Then, the mixture was filtered over Celite, the celite was washed with MeOH. The filtrate was concentrated in vacuo to give 144 mg (0.30 mmol, 100% yield) of the title compound. This compound was used for the next step without any further purification.

### Intermediate 46

### C₁₄-Phe-D-GluOMe

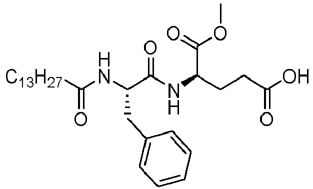

Intermediate 42 (167 g, 0.27 mmol) was dissolved in a mixture of THF/MeOH mixture (1/1) (10 mL) palladium 10% on carbon (16 mg) was added and the mixture was stirred under hydrogen atmosphere for 2h. Then, the mixture was filtered over Celite, the celite was washed with MeOH. The filtrate was concentrated in vacuo to give 142 mg (0.27 mmol, 100% yield) of the title compound. This compound was used for the next step without any further purification.

### Intermediate 47

### C₁₄-Leu-D-GluOMe-γ-mesoLAN(Boc, OMe)OMe

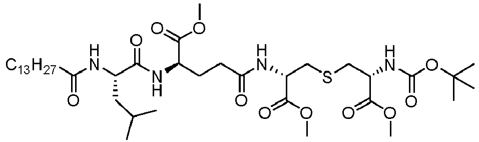

To a solution of intermediate 10 (104 mg, 0.31 mmol) in dry DMF (10 mL) were added intermediate 43 (140 mg, 0.28 mmol), HATU (129 mg, 0.34 mmol), the pH was adjusted to 9 with NMM (102 µL, 0.92 mmol). The reaction mixture was stirred at rt under argon for 2h. Then the reaction was diluted with EtOAc (60 mL), and washed twice with a saturated solution of ammonium chloride. The organic layer was dried over MgSO₄, filtrated and concentrated in vacuo. The crude product was purified by flash column chromatography (silica gel, DCM/MeOH 0 to 2%) to give 116 mg (0.14 mmol, 50% yield) of the title compound. ¹H NMR (DMSO-*d₆*) δ 8.36 (1H, t), 7.87 (1H, d), 7.31 (1H, d), 4.44 (2H, m), 4.20 (2H, d), 3.68 (3H, s), 3.66 (3H, s), 3.67 (3H, s), 2.94-2.71 (2H, m), 2.17 (4H, m), 2.09 (1H, m), 1.93 (1H, m), 1.52 (13H, m), 1.26 (20H, m), 0.85 (9H, m).

### Intermediate 48

### C₁₄-Ile-D-GluOMe-γ-mesoLAN(Boc, OMe)OMe

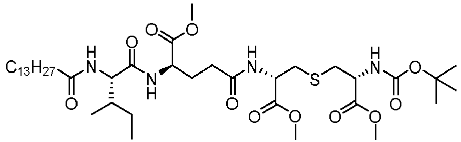

To a solution of intermediate 10 (104 mg, 0.31 mmol) in dry DMF (10 mL) were added intermediate 44 (140 mg, 0.28 mmol), HATU (129 mg, 0.34 mmol), the pH was adjusted to 9 with NMM (102 µL, 0.92 mmol). The reaction mixture was stirred at rt under argon for 2h. Then the reaction was diluted with EtOAc (60 mL), and washed twice with a saturated solution of ammonium chloride. The organic layer was dried over MgSO₄, filtrated and concentrated in vacuo. The crude product was purified by flash column chromatography (silica gel, DCM/MeOH 0 to 2%) to give 115 mg (0.14 mmol, 51% yield) of the title compound. ¹H NMR (DMSO-*d₆*) δ 8.35 (1H, m), 7.87 (1H, d), 7.36 (1H, d), 4.45 (2H, m), 4.15 (3H, m), 3.68 (3H, s), 3.67 (3H, s), 3.66 (3H, s), 2.84-2.81 (2H, m), 2.16 (4H, m), 2.08 (1H, m), 1.98 (1H, m), 1.53 (13H, m), 1.26 (20H, m), 0.87 (9H, m).

### Intermediate 49

### C₁₄-Val-D-GluOMe-γ-mesoLAN(Boc, OMe)OMe

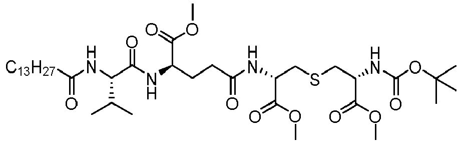

To a solution of intermediate 10 (107 mg, 0.32 mmol) in dry DMF (10 mL) were added intermediate 44 (140 mg, 0.29 mmol), HATU (133 mg, 0.35 mmol), the pH was adjusted to 9 with NMM (105 µL, 0.95 mmol). The reaction mixture was stirred at rt under argon for 2h. Then the reaction was diluted with EtOAc (60 mL), and washed twice with a saturated solution of ammonium chloride. The organic layer was dried over MgSO₄, filtrated and concentrated in vacuo. The crude product was purified by flash column chromatography (silica gel, DCM/MeOH 0 to 2%) to give 97 mg (0.12 mmol, 41% yield) of the title compound. ¹H NMR (DMSO-*d₆*) δ 8.36 (1H, m), 7.86 (1H, d), 7.37 (1H, d), 4.44 (2H, m), 4.14 (3H, m), 3.68 (3H, s), 3.67 (3H, s), 3.66 (3H, s), 2.90-2.76 (2H, m), 2.10 (4H, m), 1.98 (1H, m), 1.85 (1H, m), 1.54 (13H, m), 1.26 (20H, m), 0.87 (9H, m).

### Intermediate 50

### C₁₄-Phe-D-GluOMe-γ-mesoLAN(Boc, OMe)OMe

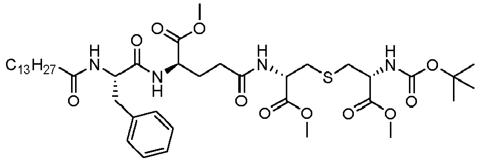

To a solution of intermediate 10 (97 mg, 0.28 mmol) in dry DMF (10 mL) were added intermediate 45 (140 mg, 0.27 mmol), HATU (121 mg, 0.32 mmol), the pH was adjusted to 9 with NMM (95 µL, 0.86 mmol). The reaction mixture was stirred at rt under argon for 2h. Then the reaction was diluted with EtOAc (60 mL), and washed twice with a saturated solution of ammonium chloride. The organic layer was dried over MgSO₄, filtrated and concentrated in vacuo. The crude product was purified by flash column chromatography (silica gel, DCM/MeOH 0 to 2%) to give 95 mg (0.11 mmol, 42% yield) of the title compound. ¹H NMR (DMSO-*d₆*) δ 8.46 (1H, m), 8.36 (1H, m), 7.33-7.16 (5H, m), 4.62 (1H, m), 4.45 (1H, m), 4.25 (1H, m), 4.16 (1H, m), 3.66 (3H, s), 3.67 (3H, s), 3.45 (3, s), 3.02-2.71 (2H, m), 2.13 (4H, m), 1.55 (2H, m), 1.45 (13H, m), 1.30 (20H, m), 0.85 (3H, t).

### Intermediate 51

### Boc-D-AlaOBn

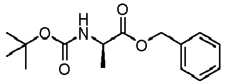

Boc-D-AlaOH (5.0 g, 26.44 mmol) was dissolved in dry DMF (50 mL), then cesium carbonate (5.17 g, 15.86 mmol) was added, the mixture was stirred at rt for 15 min, and benzyl bromide (4.75 g, 27.76 mmol) was added dropwise. The stirring was maintained for 18h. Then, the mixture was diluted with water and extracted with EtOAc 3 times. The organic layer was washed with water, brine, dried over MgSO₄, filtered and concentrated. The crude product was purified by flash column chromatography (silica gel, DCM/MeOH 0 to 1%) to give 6.97 g (25.0 mmol, 94% yield) of the title compound. ¹H NMR (DMSO-*d₆*) δ 7.28-7.24 (6H, m), 5.04 (2H, q), 3.99 (1H, q), 1.30 (9H, s), 1.18 (3H, d).

### Intermediate 52

### TFA.D-AlaOBn

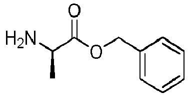

The compound **51** (6.90 g, 25.0 mmol) was dissolved in TFA/DCM mixture (1/1) (10 mL). After stirring for 1h at rt, the solvents were removed under vacuo; the residue was coevaporated 3 times with toluene. The crude compound was triturated with Et₂O to provide 7.3 g (25.0 mmol, 100% yield) of the title compound. ¹H NMR (DMSO-*d₆*) δ 8.38 (2H, sl), 7.36-7.26 (5H, m), 5.17 (2H, s), 4.11 (1H, q), 1.34 (3H, d).

### Intermediate 53

### Fmoc-Cys(Tr)-D-AlaOBn

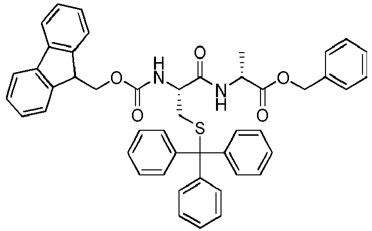

To a solution of intermediate 52 (4.58 g, 15.60 mmol) in dry DCM (50 mL) were added Fmoc-Cys(Tr)OH (9.15 g, 15.62 mmol) and PyBOP (8.94 g, 17.18 mmol), the pH was adjusted to 10 with NMM. The reaction mixture was stirred at rt under argon for 16h. Then the reaction was diluted with DCM (200 mL), and washed twice with a saturated solution of ammonium chloride. The organic layer was dried over MgSO₄, filtrated and concentrated in vacuo. The crude product was purified by flash column chromatography (silica gel, DCM/MeOH 0 to 1%) to give 9.02 g (12.09 mmol, 77% yield) of the title compound. ¹H NMR (DMSO-*d₆*) δ 8.28 (1H, d), 7.81 (2H, d), 7.67 (2H, d), 7.61 (1H, d), 7.30 (2H, m), 7.24-7.14 (23H, m), 5.00 (2H, s), 4.21-4.11 (5H, m), 2.30 (2H, m), 1.18 (3H, d).

### Intermediate 54

### Fmoc-Cys-D-AlaOBn

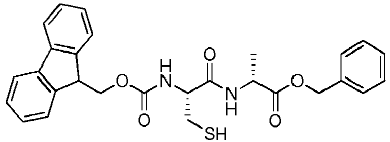

The intermediate 53 (4.03 g, 5.39 mmol) was dissolved in TFA/DCM mixture (1/1) (25 mL) and TIPS (2.82 g, 17.81 mmol) was added. After stirring for 2h at rt, the solvents were removed under vacuo, coevaporated with toluene. The compound was triturated with a mixture of petroleum ether/Et₂O (2/1) the precipitate was filtrated to provide 2.33 g (4.64 mmol, 86% yield) of the title compound. ¹H NMR (DMSO-*d₆*) δ 8.42 (1H, d), 7.82 (2H, d), 7.67 (2H, d), 7.49 (1H, d), 7.34 (2H, m), 7.28-7.21 (7H, m), 5.04 (2H, s), 4.29-4.10 (5H, m), 2.62 (2H, m), 2.20 (1H, t), 1.23 (3H, d).

### Intermediate 55

### Fmoc-mesoLAN(Z, OBn)-D-AlaOBn

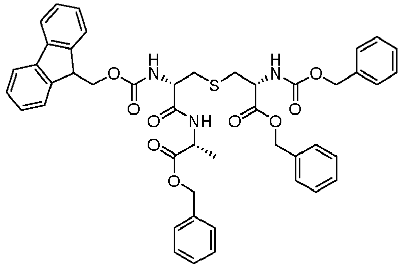

To a solution of intermediate 54 (3.33 g, 6.6 mmol) and intermediate **4** (2.73 g, 6.94 mmol) in EtOAc (100 mL) was added a pH 8.5 solution of NaHCO₃ (100 mL) followed by addition of TBAHS (8.97 g, 26.44 mmol). The mixture was vigorously stirred at rt overnight. Then, the mixture was diluted with EtOAc (200 mL) and was washed successively with saturated NaHCO₃, water and brine. The organic layer was dried over MgSO₄, filtered and concentrated in vacuo. The residue was purified by flash column chromatography (silica gel, DCM/MeOH 0 to 2%) to give 4.53 g (5.55 mmol, 84% yield) of the title compound. ¹H NMR (DMSO-*d₆*) δ 8.50 (1H, m), 7.82 (3H, m), 7.65 (2H, d), 7.36-7.21 (18H, m), 5.06 (2H, s), 5.03 (2H, s), 4.96 (2H,s), 4.25-4.13 (6H, m), 2.77 (4H, m), 1.21 (3H, d).

### Intermediate 56

### H-mesoLAN(Z, OBn)-D-AlaOBn

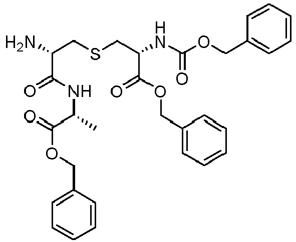

The intermediate 55 (1.00 g, 1.22 mmol) was dissolved with DMF (10 mL), piperidine (20 mg, 0.24 mmol) was added dropwise , and the mixture was heated at 50°C for 45min. Then, water (10 mL) was added, and the mixture was extracted with EtOAc three times. The organic layer was washed with water (3 times) and brine, dried over MgSO₄, filtered and concentrated in vacuo. The crude compound was purified by flash column chromatography (silica gel, DCM/MeOH 0 to 1%) to provide 0.740 g (1.22 mmol, 100% yield) of the title compound. ¹H NMR (DMSO-*d₆*) δ 8.27 (1H, m), 7.88 (1H, d), 7.32-7.20 (15H, m), 5.08 (2H, s), 5.04 (2H, s), 4.98 (2H,s), 4.28-4.17 (2H, m), 2.70 (4H, m), 1.84 (2H, sl), 1.21 (3H, d).

### Intermediate 57

### Boc-D-AlaOMe

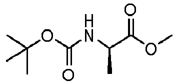

Boc-D-AlaOH (10.0 g, 52.9 mmol) was dissolved in dry DMF (60 mL), then potassium carbonate (8.0 g, 58.0 mmol) was added, the mixture was stirred at rt for 15 min, and methyl iodide (6.58 mL, 106.0 mmol) was added dropwise. The stirring was maintained for 18h. Then, the mixture was concentrated in vacuo and the residue was dissolved in EtOAc (100mL). The organic layer was washed with water, brine, dried over MgSO₄, filtered and concentrated. The crude product was purified by flash column chromatography (silica gel, DCM/MeOH 0 to 1%) to give 10.36 g (51.0 mmol, 96% yield) of the title compound. ¹H NMR (DMSO-*d₆*) δ 7.46 (1H, d), 4.35 (1H, m), 3.66 (3H, s), 1.30 (9H, s), 1.18 (3H, d).

### Intermediate 58

### TFA.D-AlaOMe

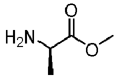

The compound **57** (10.36 g, 51.0 mmol) was dissolved in TFA/DCM mixture (1/1) (100 mL). After stirring for 1h at rt, the solvents were removed under vacuo; the residue was coevaporated 3 times with toluene. The crude compound was triturated with Et₂O to provide 11.0 g (51.0 mmol, 99% yield) of the compound. This compound was used for the next step without any further purification.

### Intermediate 59

### Fmoc-Cys(Tr)-D-AlaOMe

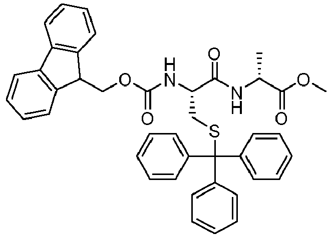

To a solution of intermediate 58 (5.6 g, 26.0 mmol) in dry DMF (120 mL) were added Fmoc-Cys(Tr)OH (10.0 g, 17.1 mmol) and HATU (7.10 g, 19.0 mmol), the pH was adjusted to 10 with NMM (6.98 mL, 68.3mmol). The reaction mixture was stirred at rt under argon for 16h. Then the reaction was concentrated in vacuo, the residue was dissolved in EtOAc (200 mL), and washed twice with a saturated solution of ammonium chloride. The organic layer was dried over MgSO₄, filtrated and concentrated in vacuo. The crude product was purified by flash column chromatography (silica gel, DCM/MeOH 0 to 1%) to give 6.17 g (9.20 mmol, 54% yield) of the title compound. ¹H NMR (DMSO-*d₆*) δ 8.28 (1H, d), 7.74 (2H, d), 7.63 (2H, d), 7.41 (1H, d), 7.33 (19H, m), 4.21-4.11 (5H, m), 3.57 (3H, s), 2.36 (2H, m), 1.23 (3H, d).

### Intermediate 60

### Fmoc-Cys-D-AlaOMe

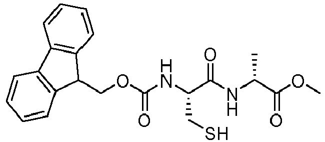

The intermediate 59 (6.17 g, 9.20 mmol) was dissolved in TFA/DCM mixture (1/1) (200 mL) and TIPS (6.22 mL, 30.35 mmol) was added. After stirring for 2h at rt, the solvents were removed under vacuo, coevaporated with toluene. The compound was triturated with a mixture of petroleum ether/Et₂O (2/1) the precipitate was filtrated to provide 3.42 g (7.98 mmol, 87% yield) of the title compound. ¹H NMR (DMSO-*d₆*) δ 8.48 (1H, d), 7.90 (2H, d), 7.74 (2H, d), 7.45 (1H, d), 7.40 (2H, m), 7.30 (2H, m), 4.32-4.16 (5H, m), 3.62 (3H, s), 2.80-2.65 (2H, m), 2.27 (1H, t), 1.27 (3H, d).

### Intermediate 61

### Fmoc-mesoLAN(Boc, OMe)-D-AlaOMe

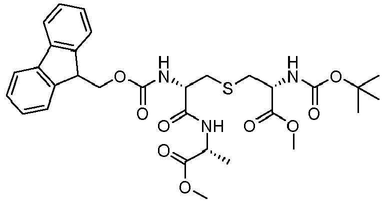

To a solution of intermediate 60 (3.42 g, 7.98 mmol) and intermediate 8 (2.5 g, 8.80 mmol) in EtOAc (100 mL) was added a pH 8.5 solution of NaHCO₃ (100 mL) followed by addition of TBAHS (11.0 g, 32.0 mmol). The mixture was vigorously stirred at rt overnight. Then, the mixture was diluted with EtOAc (200 mL) and was washed successively with saturated NaHCO₃, water and brine. The organic layer was dried over MgSO₄, filtered and concentrated in vacuo. The residue was purified by flash column chromatography (silica gel, DCM/MeOH 0 to 2%) to give 3.3 g (5.20 mmol, 66% yield) of the title compound. ¹H NMR (DMSO-*d₆*) δ 8.12 (1H, d), 7.90 (2H, d), 7.75 (2H, d), 7.60 (1H, d), 7.42 (2H, m), 7.30 (2H, m), 4.31-4.22 (6H, m), 2.81 (4H, m), 1.37 (9H, s), 1.28 (3H, d).

### Intermediate 62

### H-mesoLAN(Boc, OMe)-D-AlaOMe

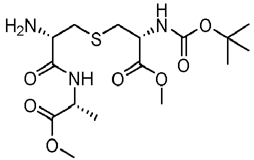

The intermediate 61 (3.3 g, 5.2 mmol) was dissolved with DMF (10 mL), piperidine (103 µL, 1.04 mmol) was added dropwise, and the mixture was heated at 50°C for 1h. Then, water (10 mL) was added, and the mixture was extracted with EtOAc three times. The organic layer was washed with water (3 times) and brine, dried over MgSO₄, filtered and concentrated in vacuo. The crude compound was purified by flash column chromatography (silica gel, DCM/MeOH 2 to 5%) to provide 1.46 g (3.57 mmol, 68% yield) of the title compound. ¹H NMR (DMSO-*d₆*) δ 8.31 (1H, d), 7.34 (1H, d), 4.30 (1H, m), 4.13 (1H, m), 3.62 (6H, s), 2.92-2.53 (4H, m), 1.90 (2H, sl), 1.45 (9H, s), 1.21 (3H, d).

### Intermediate 63

### Boc-D-GluOBn-γ-mesoLAN(Z, OBn)-D-AlaOBn

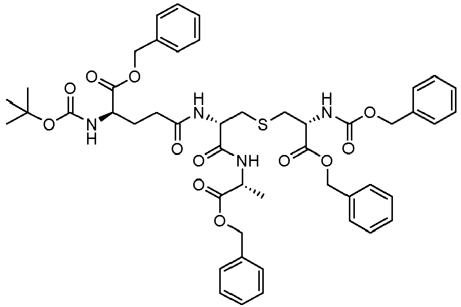

To a solution of intermediate 56 (727 mg, 1.22 mmol) in dry DCM (15 mL) were added Boc-D-GluOBn (411 mg, 1.22 mmol) and PyBOP (700 mg, 1.34 mmol), the pH was adjusted to 10 with NMM. The reaction mixture was stirred at rt under argon for 16h. Then the reaction was diluted with DCM (50 mL), and washed twice with a saturated solution of ammonium chloride. The organic layer was dried over MgSO₄, filtrated and concentrated in vacuo. The crude product was purified by flash column chromatography (silica gel, DCM/MeOH 0 to 1%) to give 769 mg (0.84 mmol, 69% yield) of the title compound. ¹H NMR (DMSO-*d₆*) δ 8.50 (1H, m), 8.01 (1H, d), 7.77 (1H, d), 7.28-7.21 (20H, m), 5.06 (2H, s), 5.04 (4H, s), 4.97 (2H, s), 4.46 (1H, m), 4.27-4.19 (2H, m), 3.96 (1H, m), 2.74 (4H, m), 2.17 (2H, m), 1.86 (1H, m), 1.71 (1H, m), 1.29 (9H, s), 1.19 (3H, d).

### Intermediate 64

### TFA.D-GluOBn-γ-mesoLAN(Z, OBn)-D-AlaOBn

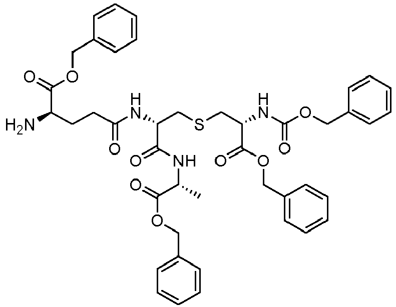

Intermediate 63 (765 mg, 0.83 mmol) was dissolved in TFA/DCM mixture (1/1) (40 mL). After stirring for 2h at rt, the solvents were removed under vacuo; the residue was coevaporated 3 times with toluene. The crude compound was purified by flash column chromatography (silica gel, DCM/MeOH 0 to 2%) to give 777 mg (0.83 mmol, 100% yield) of the title compound. ¹H NMR (DMSO-*d₆*) δ 8.57 (1H, m), 8.33 (2H, sl), 8.15 (1H, m), 7.79, (1H, m), 7.41-7.08 (20H, m), 5.23-4.97 (8H, m), 4.48 (1H, m), 4.15 (2H, m), 3.96 (1H, m), 2.66 (4H, m), 2.27 (2H, m), 1.97 (2H, m), 1.20 (3H, d).

### Intermediate 65

### C₇-D-GluOBn-γ-mesoLAN(Z, OBn)-D-AlaOBn

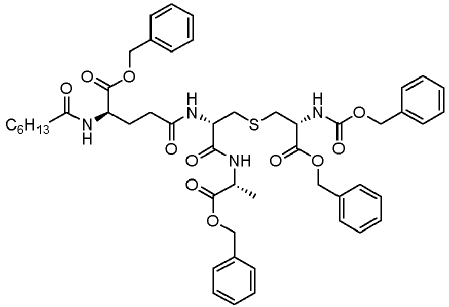

To a solution of intermediate 64 (75 mg, 0.08 mmol) in dry DCM (10 mL) under argon atmosphere was added TEA (17 µL, 0.12 mmol). The mixture was cooled to 0°C and a solution of heptanoyl chloride (13 µL, 0.08 mmol) in dry DCM (1 mL) was added dropwise. The solution was allowed to warm to rt and stirred overnight. The reaction was diluted in DCM (20 mL), washed with NaHCO₃, 1N HCl, water, and brine. The organic layer was dried over MgSO₄, filtered and concentrated in vacuo. The crude product was purified by flash column chromatography (silica gel, DCM/MeOH 0 to 1%) to provide 71 mg (0.07 mmol, 94% yield) of the title compound. ¹H NMR (DMSO-*d₆*) δ 8.51 (1H, d), 8.25 (1H, d), 8.11 (1H, d), 7.85 (1H, d), 7.28-7.22 (20H, m), 5.06 (2H, s), 5.03 (4H, s), 4.97 (2H, s), 4.44 (1H, m), 4.22 (3H, m), 2.92-2.47 (4H, m), 2.15 (2H,m), 2.03 (2H, m), 2.00 (1H, m), 1.82 (1H, m), 1.39 (2H,m), 1.19 (10H, m), 0.78 (3H, t).

### Intermediate 66

### C₁₂-D-GluOBn-γ-mesoLAN(Z, OBn)-D-AlaOBn

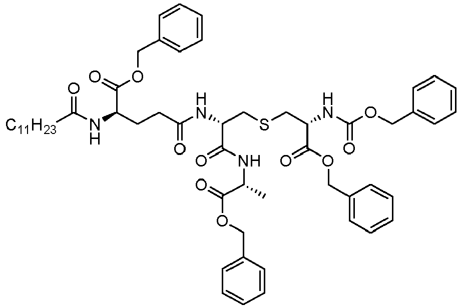

To a solution of intermediate 64 (93 mg, 0.10 mmol) in dry DCM (10 mL) under argon atmosphere was added TEA (20 µL, 0.15 mmol). The mixture was cooled to 0°C and a solution of lauroyl chloride (23 mg, 0.105 mmol) in dry DCM (2 mL) was added dropwise. The solution was allowed to warm to rt and stirred overnight. The reaction was diluted in DCM (20 mL), washed with NaHCO₃, 1N HCl, water, and brine. The organic layer was dried over MgSO₄, filtered and concentrated in vacuo. The crude product was purified by flash column chromatography (silica gel, DCM/MeOH 0 to 1%) to provide 81 mg (0.081 mmol, 81% yield) of the title compound. ¹H NMR (DMSO-*d₆*) δ 8.48 (1H, d), 8.12 (1H, d), 8.05 (1H, d), 7.76 (1H, d), 7.28-7.21 (20H, m), 5.08 (2H, s), 5.06 (4H, s), 5.03 (2H, s), 4.46 (1H, m), 4.25 (3H, m), 2.73 (4H, m), 2.15 (2H,m), 2.03 (2H, m), 1.95 (1H, m), 1.75 (1H, m), 1.41 (2H,m), 1.17 (16H, m), 0.77 (3H, t).

### Intermediate 67

### C₁₄-D-GluOBn-γ-mesoLAN(Z, OBn)-D-AlaOBn

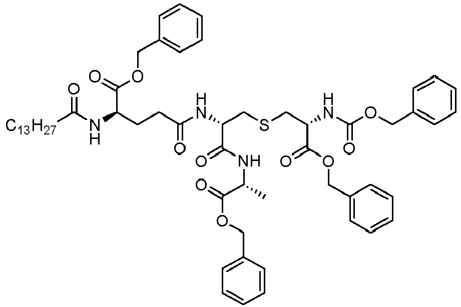

To a solution of intermediate 64 (145 mg, 0.15 mmol) in dry DCM (10 mL) under argon atmosphere was added TEA (33 µL, 0.23 mmol). The mixture was cooled to 0°C and a solution of myristoyl chloride (40 mg, 0.16 mmol) in dry DCM (1 mL) was added dropwise. The solution was allowed to warm to rt and stirred overnight. The reaction was diluted in DCM (20 mL), washed with NaHCO₃, 1N HCl, water, and brine. The organic layer was dried over MgSO₄, filtered and concentrated in vacuo. The crude product was purified by flash column chromatography (silica gel, DCM/MeOH 0 to 1%) to provide 121 mg (0.11 mmol, 75% yield) of the title compound. ¹H NMR (DMSO-*d₆*) δ 8.49 (1H, d), 8.13 (1H, d), 8.04 (1H, d), 7.75 (1H, d), 7.35-7.33 (20H, m), 5.06 (2H, s), 5.03 (4H, s), 4.97 (2H, s), 4.44 (1H, m), 4.20 (3H, m), 2.87 (1H, m), 2.74 (2H, m), 2.55 (1H, m), 2.25 (2H,m), 2.18 (2H, m), 2.13 (1H, m), 1.90 (1H, m), 1.80 (1H, m), 1.38 (2H,m), 1.19 (23H, m), 0.87 (3H, t).

### Intermediate 68

### BocAla-D-GluOBn-γ-mesoLAN(Z, OBn)-D-AlaOBn

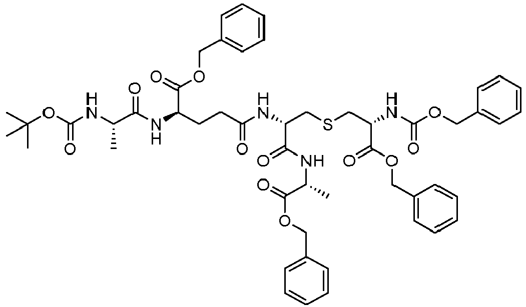

To a solution of intermediate 64 (595 mg, 0.64 mmol) in dry DCM (20 mL) were added Boc-AlaOH (121 mg, 0.64 mmol) and PyBOP (370 mg, 0.71 mmol), the pH was adjusted to 10 with NMM. The reaction mixture was stirred at rt under argon for 16h. Then the reaction was diluted with DCM (100 mL), and washed twice with a saturated solution of ammonium chloride. The organic layer was dried over MgSO₄, filtrated and concentrated in vacuo. The crude product was purified by flash column chromatography (silica gel, DCM/MeOH 0 to 1%) to give 537 mg (0.55 mmol, 85% yield) of the title compound.¹H NMR (DMSO-*d₆*) δ 8.55 (1H, m), 8.32 (1H, d), 8.12 (1H, m), 7.82 (1H, d), 7.37-7.32 (20H, m), 6.82 (1H, d), 5.14 (2H, s), 5.10 (4H, s), 5.04 (2H, s), 4.47 (1H, m), 4.47-4.29 (3H, m), 3.98 (1H, m), 2.84 (4H, m), 2.24 (2H, m), 2.01 (1H, m), 1.83 (1H, m), 1.37 (9H, s), 1.25 (3H, d), 1.17 (3H, d).

### Intermediate 69

### TFA.Ala-D-GluOBn-γ-mesoLAN(Z, OBn)-D-AlaOBn

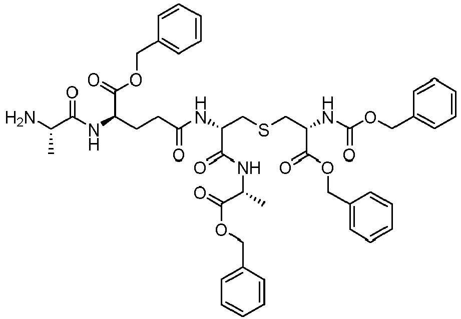

The intermediate 68 (530 mg, 0.54 mmol) was dissolved in TFA/DCM mixture (1/1) (30 mL). After stirring for 1h at rt, the solvents were removed under vacuo; the residue was coevaporated 3 times with toluene. The crude compound was purified by flash column chromatography (silica gel, DCM/MeOH 0 to 5%) to give 540 mg (0.54 mmol, 100% yield) of the title compound. ¹H NMR (DMSO-*d₆*) δ 8.50 (1H, m), 8.33 (1H, d), 8.07 (1H, m), 7.77 (1H, d), 7.29-7.23 (21H, m), 5.06 (4H, s), 5.03 (2H, s), 4.97 (2H, s), 4.47 (1H, m), 4.27-4.22 (3H, m), 3.44 (1H, m), 2.74 (4H, m), 2.14 (2H, m), 1.98 (1H, m), 1.80 (1H, m), 1.21-1.12 (6H, m).

### Intermediate 70

### C₇-Ala-D-Glu(OBn)OMe

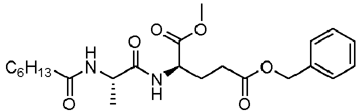

To a solution of intermediate 17 (1.6 g, 4.40 mmol) in dry DMF (10 mL) were added C₇-AlaOH (593 mg, 2.94 mmol) and HATU (1.2 g, 3.20 mmol), the pH was adjusted to 10 with NMM (1.20 mL, 11.80 mmol). The reaction mixture was stirred at rt under argon for 18h. Then the solvent was removed in vacuo and the residue was dissolved in EtOAc (50 mL), and washed twice with a saturated solution of ammonium chloride. The organic layer was dried over MgSO₄, filtrated and concentrated in vacuo. The crude product was purified by flash column chromatography (silica gel, DCM/MeOH 0 to 1%) to give 997 mg (2.29 mmol, 78% yield) of the title compound. ¹H NMR (DMSO-*d₆*) δ 8.25 (1H, d), 8.33 (1H, d), 7.90 (1H, d), 7.36 (5H, m), 5.09 (2H, s), 4.31 (2H, m), 3.62 (3H, s), 2.44 (2H, m), 2.10 (2H, m), 2.00 (1H, m), 1.85 (1H, m), 1.46 (2, m), 1.21-1.16 (9H, m), 0.87 (3H, t).

### Intermediate 71

### C₇-Ala-D-GluOMe

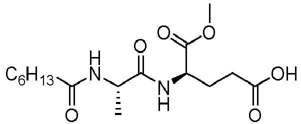

Intermediate 70 (997 mg, 2.29 mmol) was dissolved in a mixture of THF/MeOH mixture (1/1) (20 mL) palladium 10% on carbon (122 mg) was added and the mixture was stirred under hydrogen atmosphere for 2h. Then, the mixture was filtered over Celite, the celite was washed with MeOH. The filtrate was concentrated in vacuo to give 790 mg (2.29 mmol, 100% yield) of the title compound. This compound was used for the next step without any further purification.

### Intermediate 72

### C₇-Ala-D-GluOMe-γ-mesoLAN(Boc, OMe)-D-AlaOMe

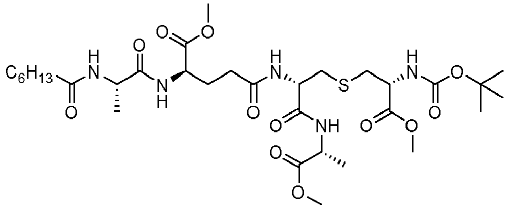

To a solution of intermediate 71 (85 mg, 0.25 mmol) in dry DMF (10 mL) were added intermediate 62 (116 mg, 0.28 mmol) and HATU (100.0 mg, 0.27 mmol), the pH was adjusted to 10 with NMM (75 µL, 0.74 mmol). The reaction mixture was stirred at rt under argon for 18h. Then the solvent was removed in vacuo and the residue was dissolved in EtOAc (50 mL), and washed twice with a saturated solution of ammonium chloride. The organic layer was dried over MgSO₄, filtrated and concentrated in vacuo. The crude product was purified by flash column chromatography (silica gel, DCM/MeOH 0 to 2%) to give 152 mg (0.20 mmol, 84% yield) of the title compound. ¹H NMR (DMSO-*d₆*) δ 8.50 (1H, d), 8.26 (1H, d), 8.09 (1H, d), 7.89 (1H, d), 7.25 (1H, d), 4.51 (1H, m), 4.29 (3H, m), 4.10 (1H, m), 3.65 (3H, s), 3.63 (3H, s), 3.62 (3H, s), 2.91-2.61 (4H, m), 2.16 (2H, m), 2.08 (2H, m), 1.98 (1H, m), 1.82 (1H, m), 1.47 (2H, m), 1.40 (9H, s), 1.30-1.15 (12H, m), 0.83 (3H, t).

### Intermediate 73

### C₁₂-Ala-D-GluOBn-γ-mesoLAN(Z, OBn)-D-AlaOBn

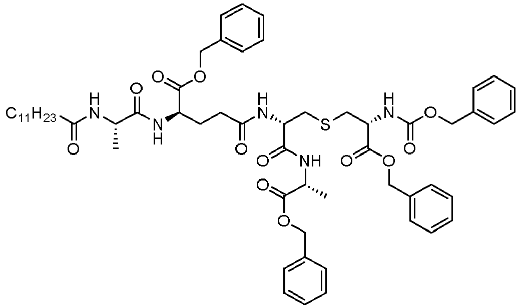

To a solution of intermediate 69 (132 mg, 0.13 mmol) in dry DCM (10 mL) under argon atmosphere was added TEA (27 µL, 0.19 mmol). The mixture was cooled to 0°C and a solution of lauroyl chloride (30 mg, 0.14 mmol) in dry DCM (2 mL) was added dropwise. The solution was allowed to warm to rt and stirred overnight. The reaction was diluted in DCM (20 mL), washed with NaHCO₃, 1N HCl, water, and brine. The organic layer was dried over MgSO₄, filtered and concentrated in vacuo. The crude product was purified by flash column chromatography (silica gel, DCM/MeOH 0 to 2%) to provide 127 mg (0.12 mmol, 90% yield) of the title compound. ¹H NMR (DMSO-*d₆*) δ 8.49 (1H, dd), 8.25 (1H, d), 8.03 (1H, m), 7.79 (2H, m), 7.34-7.20 (20H, m), 5.06 (2H, s), 5.02 (4H, s), 4.94 (2H, s), 4.43 (1H, m), 4.31-4.17 (4H, m), 2.74 (4H, m), 2.15 (2H, m), 2.02 (2H, m), 1.99 (1H, m), 1.75 (1H, m), 1.38 (2H, m), 1.21-1.09 (22H, m), 0.78 (3H, t).

### Intermediate 74

### C₁₄-Ala-D-GluOBn-γ-mesoLAN(Z, OBn)-D-AlaOBn

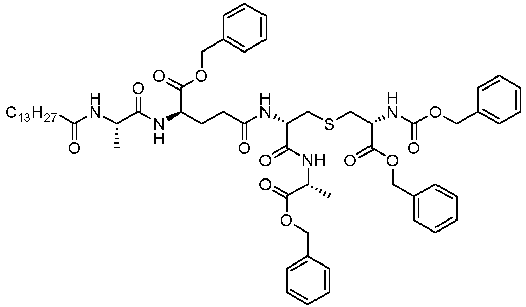

To a solution of intermediate 69 (134 mg, 0.13 mmol) in dry DCM (10 mL) under argon atmosphere was added TEA (28 µL, 0.20 mmol). The mixture was cooled to 0°C and a solution of myristoyl chloride (35 mg, 0.14 mmol) in dry DCM (2 mL) was added dropwise. The solution was allowed to warm to rt and stirred overnight. The reaction was diluted in DCM (50 mL), washed with NaHCO₃, 1N HCl, water, and brine. The organic layer was dried over MgSO₄, filtered and concentrated in vacuo. The crude product was purified by flash column chromatography (silica gel, DCM/MeOH 0 to 1%) to provide 140 mg (0.13 mmol, 95% yield) of the title compound. ¹H NMR (DMSO-*d₆*) δ 8.47 (1H, m), 8.24 (1H, d), 8.01 (1H, m), 7.78 (2H, m), 7.27 (20H, m), 5.02 (8H, m), 4.43 (1H, m), 4.24 (4H, m), 2.86-2.70 (4H, m), 2.15 (2H, m), 2.09 (2H, m), 1.99 (1H, m), 1.81 (1H, m), 1.38 (2H, m), 1.20 (37H, m), 0.76 (3H, t).

### Intermediate 75

### Boc-GlyOBn

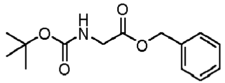

Boc-GlyOH (2.0 g, 11.42 mmol) was dissolved in dry DMF (20 mL), then cesium carbonate (2.23 g, 6.85 mmol) was added, the mixture was stirred at rt for 15 min, then the mixture was cooled to 0°C and benzyl bromide (1.43 mL, 12.0 mmol) was added dropwise. The stirring was maintained for 18h. Then, the solvent was removed in vacuo and the residue was diluted with EtOAc (70 mL) and the organic layer was washed with water and brine dried over MgSO₄, filtered and concentrated. The crude product was purified by flash column chromatography (silica gel, DCM/MeOH 0 to 1%) to give 3.03 g (12.0 mmol, 100% yield) of the title compound. ¹H NMR (DMSO-*d₆*) δ 7.29 (5H, m), 7.19 (1H, t), 5.05 (2H, s), 3.66 (2H, d), 1.30 (9H, s).

### Intermediate 76

### BocGlyOMe

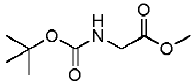

Boc-GlyOH (2.0 g, 11.41 mmol) was dissolved in dry DMF (20 mL), then potassium carbonate (1.71 g, 12.31 mmol) was added, the mixture was stirred at rt for 15 min, then the mixture was cooled to 0°C and methyl iodide (3.24 g, 22.83 mmol) was added dropwise. The stirring was maintained for 18h. Then, the solvent was removed in vacuo and the residue was diluted with EtOAc (50 mL) and the organic layer was washed with water and brine dried over MgSO₄, filtered and concentrated. The crude product was purified by flash column chromatography (silica gel, DCM/MeOH 0 to 1%) to give 2.06 g (10.88 mmol, 95% yield) of the title compound. ¹H NMR (DMSO-*d₆*) δ 7.19 (1H, m), 3.74 (2H, d), 3.65 (3H, s), 1.39 (9H, s).

### Intermediate 77

### TFA.GlyOBn

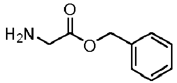

Intermediate 75 (3.0 g, 11.8 mmol) was dissolved in TFA/DCM mixture (1/3) (55 mL). After stirring for 1h at rt, the solvents were removed under vacuo; the residue was coevaporated 3 times with toluene. The crude compound was triturated with Et₂O to provide 3.21 g (11.5 mmol, 96% yield) of the title compound. This compound was used for the next step without any further purification.

### Intermediate 78

### TFA.GlyOMe

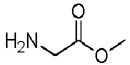

Intermediate 76 (2.059 g, 10.88 mmol) was dissolved in TFA/DCM mixture (1/3) (20 mL). After stirring for 4h at rt, the solvents were removed under vacuo; the residue was coevaporated 3 times with toluene. The crude compound was triturated with Et₂O to provide 2.21 g (10.88 mmol, 100% yield) of the title compound. ¹H NMR (DMSO-*d₆*) δ 7.19 (1H, m), 3.74 (2H, d), 3.65 (3H, s), 1.39 (9H, s).

### Intermediate 79

### Fmoc-Cys(Tr)-GlyOBn

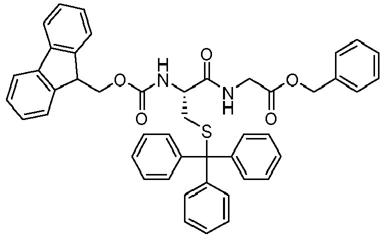

To a solution of intermediate 77 (3.21 g, 11.5 mmol) in dry DCM (30 mL) were added Fmoc-Cys(Tr)OH (6.73 g, 11.5 mmol) Py-BOP (6.58 g, 12.65 mmol), the pH was adjusted to 10 with NMM. The reaction mixture was stirred at rt under argon for 16h. Then the solvent of the reaction was removed in vacuo and the residue was dissolved in EtOAc (150 mL), and washed with a saturated solution of NaHCO₃, water and brine. The organic layer was dried over MgSO₄, filtrated and concentrated in vacuo. The crude product was purified by flash column chromatography (silica gel, DCM/MeOH 0 to 1%) to give 5.61 g (7.65 mmol, 66% yield) of the title compound. ¹H NMR (DMSO-*d₆*) δ 8.23 (1H, t), 7.88 (2H, d), 7.75 (2H, d), 7.45-7.22 (23H, m), 4.99 (2H,s), 4.24-3.76 (5H, m), 3.76 (2H, d), 3.25 (2H, s), 2.48 (2H, d).

### Intermediate 80

### Fmoc-Cys(Tr)-GlyOMe

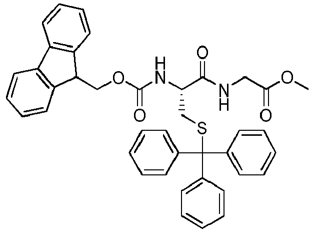

To a solution of intermediate 78 (2.00 g, 9.85 mmol) in dry DMF (120 mL) were added Fmoc-Cys(Tr)OH (4.00 g, 6.83 mmol) HATU (2.82 g, 7.42 mmol) and NMM (2.76, 27.32 mmol). The reaction mixture was stirred at rt under argon for 16h. Then the solvent of the reaction was removed in vacuo and the residue was dissolved in EtOAc (150 mL), and washed with a saturated solution of NaHCO₃, water and brine. The organic layer was dried over MgSO₄, filtrated and concentrated in vacuo. The crude product was purified by flash column chromatography (silica gel, DCM/MeOH 0 to 1%) to give 3.09 g (4.71 mmol, 69% yield) of the title compound. ¹H NMR (DMSO-*d₆*) δ 8.23 (1H, t), 7.88 (2H, d), 7.75 (2H, d), 7.45-7.22 (19H, m), 4.24 (3H, m), 4.12 (1H, m), 3.76 (2H, d), 3.55 (3H, s), 2.48 (2H, d).

### Intermediate 81

### Fmoc-Cys-GlyOBn

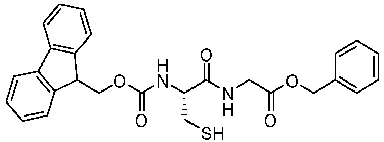

Intermediate 79 (3.0 g, 4.09 mmol) was dissolved in TFA/DCM mixture (1/1) (30 mL) and TIPS (2.14 g, 13.51 mmol) was added. After stirring for 2h at rt, the solvents were removed under vacuo, coevaporated with toluene. The compound was triturated with a mixture of petroleum the precipitate was filtrated to provide 2.0 g (4.09 mmol, 100% yield) of the the title compound.¹H NMR (DMSO-*d₆*) δ 8.42 (1H, t), 7.82 (2H, d), 7.68 (2H, m), 7.53 (2H, m), 7.37-7.23 (9H, m), 5.05 (2H, s), 4.26-4.08 (4H, m), 3.86 (2H, m), 2.71 (2H, m), 2.23 (1H, t).

### Intermediate 82

### Fmoc-Cys-GlyOBn

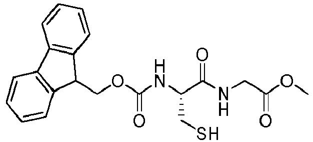

Intermediate 80 (3.09 g, 4.71 mmol) was dissolved in TFA/DCM mixture (1/1) (60 mL) and TIPS (2.46 g, 15.53 mmol) was added. After stirring for 2h at rt, the solvents were removed under vacuo, coevaporated with toluene. The compound was triturated with a mixture of petroleum the precipitate was filtrated to provide 1.79 g (4.33 mmol, 92% yield) of the the title compound.¹H NMR (DMSO-*d₆*) δ 8.53 (1H, m), 7.91 (2H, d), 7.76 (2H, d), 7.44-7.21 (4H, m), 4.27 (4H, m), 3.88 (2H, d), 3.63 (3H, s), 2.71 (2H, m), 2.35 (1H, t).

### Intermediate 83

### Fmoc-mesoLan(Z, OBn)-GlyOBn

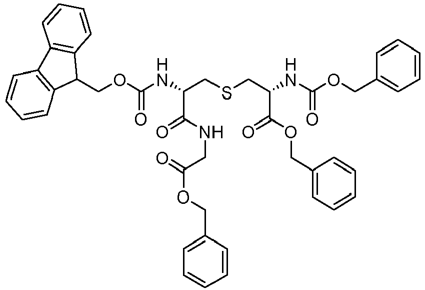

To a solution of intermediate 81 (1.92 g, 3.92 mmol) and intermediate 4 (1.61 g, 4.12 mmol) in EtOAc (60 mL) was added a pH 8.5 solution of NaHCO₃ (60 mL) followed by addition of TBAHS (5.32 g, 15.70 mmol). The mixture was vigorously stirred at rt overnight. Then, the mixture was diluted with EtOAc (200 mL) and was decanted then the organic layer was washed successively with saturated NaHCO₃, water and brine. The organic layer was dried over MgSO₄, filtered and concentrated in vacuo. The residue was purified by flash column chromatography (silica gel, DCM/MeOH 0 to 0.5%) to give 2.70 g (3.26 mmol, 83% yield) of the title compound. ¹H NMR (DMSO-*d₆*) δ 8.52 (1H, m), 7.82 (3H, m), 7.64 (3H, m), 7.36-7.21 (19H, m), 5.04 (6H, m), 4.17 (5H, m), 3.84 (2H, d), 2.88-2.61 (4H, m).

### Intermediate 84

### Fmoc-mesoLan(Boc, OMe)-GlyOMe

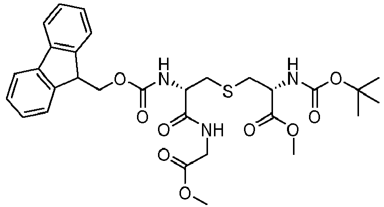

To a solution of intermediate 82 (1.79 g, 4.32 mmol) and intermediate 8 (1.51 g, 5.2 mmol) in EtOAc (60 mL) was added a pH 8.5 solution of NaHCO₃ (60 mL) followed by addition of TBAHS (5.93 g, 17.49 mmol). The mixture was vigorously stirred at rt overnight. Then, the mixture was diluted with EtOAc (200 mL) and was decanted then the organic layer was washed successively with saturated NaHCO₃, water and brine. The organic layer was dried over MgSO₄, filtered and concentrated in vacuo. The residue was purified by flash column chromatography (silica gel, DCM/MeOH 0 to 2%) to give 2.18 g (3.54 mmol, 82% yield) of the title compound. ¹H NMR (DMSO-*d₆)* δ 8.52 (1H, m), 7.90 (2H, d), 7.74 (2H, d), 7.45-7.30 (4H, m), 4.24 (4H, m), 3.87 (2H, d), 3.63 (3H, s), 2.88-2.61 (4H, m), 1.37 (9H,s).

### Intermediate 85

### H-mesoLan(Z, OBn)-GlyOBn

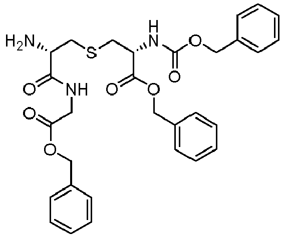

Intermediate 83 (1.00 g, 1.25 mmol) was dissolved with DMF (10 mL), piperidine (25 µL, 0.25 mmol) was added dropwise, and the mixture was heated at 50°C for 30 min. Then, water (20 mL) was added, and the mixture was extracted with EtOAc 3 times. The organic layer was washed with water (3 times) and brine, dried over MgSO₄, filtered and concentrated in vacuo. The crude compound was purified by flash column chromatography (silica gel, DCM/MeOH 2% to 5%) to provide 560 mg (0.96 mmol, 77% yield) of the title compound. ¹HNMR (DMSO-*d₆*) δ 8.35 (1H, t), 7.80 (2H, m), 7.25 (15H, m), 5.04 (6H, m), 4.23 (1H, m), 3.84 (2H, d), 3.28 (1H, m), 2.89-2.66 (4H, m), 1.87 (2H,sl).

### Intermediate 86

### H-mesoLan(Boc, OMe)-GlyOMe

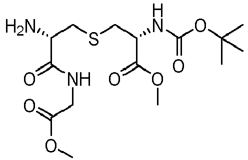

Intermediate 84 (2.18 g, 3.54 mmol) was dissolved with DMF (20 mL), piperidine (60 mg, 0.71 mmol) was added dropwise , and the mixture was heated at 50°C for 45 min. Then, water (20 mL) was added, and the mixture was extracted with EtOAc 3 times. The organic layer was washed with water (3 times) and brine, dried over MgSO₄, filtered and concentrated in vacuo. The crude compound was purified by flash column chromatography (silica gel, DCM/MeOH 2% to 5%) to provide 0.857 g (2.18 mmol, 61% yield) of the title compound. ¹HNMR (DMSO-*d₆*) δ 8.45 (1H, m), 7.30 (1H, d), 4.13 (1H, m), 3.86 (2H, d), 3.64 (3H, s), 3.63 (3H,s), 3.32 (1H, m), 2.89-2.60 (4H, m), 1.39 (9H,s).

### Intermediate 87

### Boc-D-GluOMe

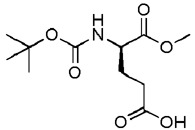

Intermediate 16 (1.0 g, 2.85 mmol) was dissolved in a mixture of THF/MeOH mixture (1/1) (20 mL) palladium 10% on carbon (30 mg) was added and the mixture was stirred under hydrogen atmosphere for 3H00. Then, the mixture was filtered over Celite; the celite was washed with MeOH. The filtrate was concentrated in vacuo to give 720 mg (2.77 mmol, 97% yield) of the title compound. This compound was used for the next step without any further purification. ¹H NMR (DMSO-*d₆*) δ 7.39 (1H, d), 4.31 (1H, m), 3.68 (3H, s), 2.33 (2H, m), 2.17 (1H, m), 2.07 (1H, m), 1.38 (9H, s).

### Intermediate 88

### C₁₂-D-Glu(OBn)OMe

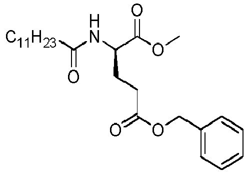

To a solution of intermediate 17 (1.07 g, 2.39 mmol) in dry DCM (20 mL) under argon atmosphere was added TEA (454 µL, 3.23 mmol). The mixture was cooled to 0°C and a solution of lauroyl chloride (706 mg, 3.23 mmol) in dry DCM (2 mL) was added dropwise. The solution was allowed to warm to rt and stirred overnight. The reaction was diluted in DCM (50 mL), washed with NaHCO₃, 1N HCl, water, and brine. The organic layer was dried over MgSO₄, filtered and concentrated in vacuo. The crude product was purified by flash column chromatography (silica gel, DCM/MeOH 0 to 1%) to provide 744 mg (1.71 mmol, 58% yield) of the title compound. ¹H NMR (DMSO-*d₆*) δ 8.19 (1H, d), 7.34 (5H, s), 5.10 (2H, s), 4.27 (1H, m), 3.62 (3H, s), 2.26 (2H, t), 2.11 (2H, t), 1.90 (1H, m), 1.82 (1H, m), 1.52 (2H, m), 1.24 (16H, m), 0.86 (3H, t).

### Intermediate 89

### C₁₄-D-Glu(OBn)OMe

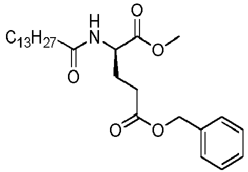

To a solution of Intermediate 17 (1.03 g, 2.83 mmol) in dry DCM (20 mL) under argon atmosphere was added TEA (438 µL, 3.12 mmol). The mixture was cooled to 0°C and a solution of myristoyl chloride (763 mg, 3.12 mmol) in dry DCM (2 mL) was added dropwise. The solution was allowed to warm to rt and stirred overnight. The reaction was diluted in DCM (50 mL), washed with NaHCO₃, 1N HCl, water, and brine. The organic layer was dried over MgSO₄, filtered and concentrated in vacuo. The crude product was purified by flash column chromatography (silica gel, DCM/MeOH 0 to 1%) to provide 892 mg (1.94 mmol, 68% yield) of the title compound. ¹H NMR (DMSO-*d₆*) δ 8.22 (1H, d), 7.36 (5H, s), 5.09 (2H, s), 4.27 (1H, m), 3.61 (3H, s), 2.43 (2H, t), 2.11 (3H, m), 1.90 (1H, m), 1.49 (2H, m), 1.24 (20H, m), 0.86 (3H, t).

### Intermediate 90

### C₁₂-D-GluOMe

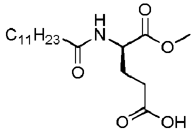

Intermediate 88 (721 mg, 1.66 mmol) was dissolved in a mixture of THF/MeOH mixture (1/1) (15 mL) palladium 10% on carbon (88 mg) was added and the mixture was stirred under hydrogen atmosphere for 3h. Then, the mixture was filtered over Celite, the celite was washed with MeOH. The filtrate was concentrated in vacuo to give 568 mg (1.65 mmol, 99% yield) of the title compound. This compound was used for the next step without any further purification. ¹H NMR (DMSO-*d₆*) δ 8.19 (1H, d), 4.23 (1H, m), 3.61 (3H, s), 2.26 (2H, t), 2.11 (2H, t), 1.95 (1H, m), 1.75 (1H, m), 1.52 (2H, m), 1.24 (16H, m), 0.86 (3H, t).

### Intermediate 91

### C₁₄-D-GluOMe

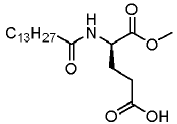

Intermediate 89 (860 mg, 1.86 mmol) was dissolved in a mixture of THF/MeOH mixture (1/1) (15 mL) palladium 10% on carbon (99 mg) was added and the mixture was stirred under hydrogen atmosphere for 3h. Then, the mixture was filtered over Celite, the celite was washed with MeOH. The filtrate was concentrated in vacuo to give 640 mg (1.72 mmol, 92% yield) of the title compound. This compound was used for the next step without any further purification. ¹H NMR (DMSO-*d₆*) δ 8.24 (1H, d), 4.27 (1H, m), 3.61 (3H, s), 2.43 (2H, t), 2.11 (3H, m), 1.90 (1H, m), 1.49 (2H, m), 1.24 (20H, m), 0.86 (3H, t).

### Intermediate 92

### Boc-D-GluOMe-γ-mesoLan(Boc, OMe)-GlyOMe

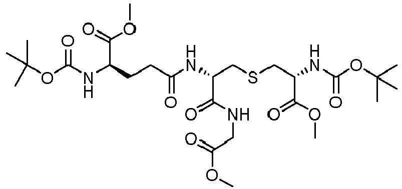

To a solution of intermediate 87 (500 mg, 1.91 mmol) in dry DMF (5 mL) were added to intermediate 86 (753 mg, 1.91 mmol) HATU (873 mg, 2.29 mmol) and NMM (387 mg, 3.83 mmol). The reaction mixture was stirred at rt under argon for 16h. Then the solvent of the reaction was removed in vacuo and the residue was dissolved in EtOAc (50 mL), and washed with a saturated solution of NaHCO₃, water and brine. The organic layer was dried over MgSO₄, filtrated and concentrated in vacuo. The crude product was purified by flash column chromatography (silica gel, DCM/MeOH 0 to 1%) to give 985 mg (1.54 mmol, 81% yield) of the title compound. ¹H NMR (DMSO-*d₆*) δ 8.46 (1H, m), 8.14 (1H, m), 7.26 (2H, m), 4.47 (1H, m), 4.14 (1H, m), 3.96 (1H, m), 3.86 (2H, m), 3.64 (3H, s), 3.62 (3H, s), 3.60 (3H, s), 2.91-2.59 (4H, m), 2.27 (2H, t), 1.95 (1H, m), 1.79 (1H, m), 1.35 (18H, m).

### Intermediate 93

### C₇-GluOMe-γ-mesoLan(Boc, OMe)-GlyOMe

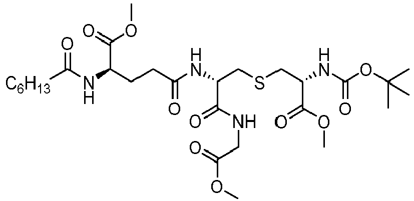

To a solution of intermediate 86 (149 mg, 0.38 mmol) in dry DMF (10 mL) were added compound 19 (90 mg, 0.33 mmol) HATU (140 mg, 0.36 mmol), the pH was adjusted to 10 with NMM (101µL, 0.98 mmol). The reaction mixture was stirred at rt under argon for 16h. Then the solvent was removed in vacuo and the residue was dissolved in EtOAc (20 mL), and washed twice with a saturated solution of ammonium chloride. The organic layer was dried over MgSO₄, filtrated and concentrated in vacuo. The crude product was purified by flash column chromatography (silica gel, DCM/MeOH 1 to 2%) to give 133 mg (0.20 mmol, 62% yield) of the title compound. ¹H NMR (DMSO-*d₆*) δ 8.41 (1H, m), 8.14 (1H, m), 7.26 (1H, m), 4.47 (1H, m), 4.25 (1H, m), 4.12 (1H, m), 3.84 (2H, d), 3.62 (6H, s), 3.61 (3H, s), 2.91-2.64 (4H, m), 2.22 (2H, t), 2.12 (2H, t), 1.95 (1H, m), 1.75 (1H, m), 1.46 (2H, m), 1.37 (9H, m), 1.28 (6H, m), 0.86 (3H, t).

### Intermediate 94

### C₁₂-GluOMe-γ-mesoLan(Boc, OMe)-GlyOMe

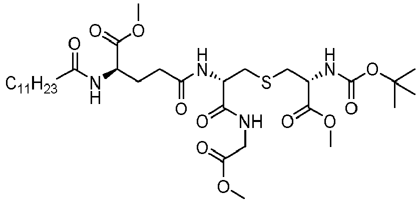

To a solution of intermediate 86 (145 mg, 0.37 mmol) in dry DMF (10 mL) were added compound 90 (110 mg, 0.32 mmol) HATU (130 mg, 0.35 mmol), the pH was adjusted to 10 with NMM (98µL, 0.96 mmol). The reaction mixture was stirred at rt under argon for 16h. Then the solvent was removed in vacuo and the residue was dissolved in EtOAc (20 mL), and washed twice with a saturated solution of ammonium chloride. The organic layer was dried over MgSO₄, filtrated and concentrated in vacuo. The crude product was purified by flash column chromatography (silica gel, DCM/MeOH 1 to 2%) to give 185 mg (0.25 mmol, 80% yield) of the title compound. ¹H NMR (DMSO-*d₆*) δ 8.43 (1H, t), 8.17(1H, m), 7.28 (1H, m), 4.49 (1H, m), 4.22 (2H, m), 3.85 (2H, d), 3.62 (6H, s), 3.61 (3H, s), 2.91-2.63 (4H, m), 2.22 (2H, t), 2.13 (2H, t), 1.96 (1H, m), 1.77 (1H, m), 1.49 (2H, m), 1.43 (9H, s), 1.27 (16H, m), 0.88 (3H, t).

### Intermediate 95

### C₁₄-GluOMe-γ-mesoLan(Boc, OMe)-GlyOMe

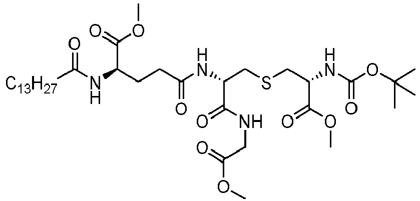

To a solution of intermediate 86 (104 mg, 0.26 mmol) in dry DMF (10 mL) were added intermediate 91 (85.0 mg, 0.23 mmol) HATU (96.0 mg, 0.25 mmol) and NMM (70 µL, 0.68 mmol). The reaction mixture was stirred at rt under argon for 16h. Then the solvent of the reaction was removed in vacuo and the residue was dissolved in EtOAc (50 mL), and washed with a saturated solution of NaHCO₃, water and brine. The organic layer was dried over MgSO₄, filtrated and concentrated in vacuo. The crude product was purified by flash column chromatography (silica gel, DCM/MeOH 0 to 2%) to give 144 mg (0.19 mmol, 84% yield) of the title compound. ¹H NMR (DMSO-*d₆*) δ 8.44 (1H, m), 8.15 (1H, m), 7.26 (1H, m), 4.47 (1H, m), 4.22 (2H, m), 3.84 (2H, d), 3.63 (6H, s), 3.61 (3H, s), 2.91-2.62 (4H, m), 2.21 (2H, m), 2.10 (2H, t), 1.96 (1H, m), 1.80 (1H, m), 1.48 (2H, m), 1.37 (9H, s), 1.21 (20H, m), 0.86 (3H, t).

### Intermediate 96

### Boc-D-GluOBn-γ-mesoLAN(Z, OBn)-GlyOBn

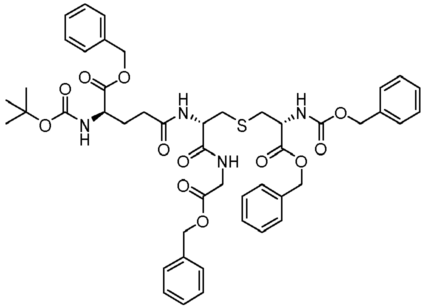

To a solution of intermediate 85 (547 mg, 0.94 mmol) in dry DCM (20 mL) were added Boc-D-GluOBn (318 mg, 0.94 mmol) and PyBOP (540 mg, 1.04 mmol), the pH was adjusted to 10 with NMM. The reaction mixture was stirred at rt under argon for 16h. Then the reaction was diluted with DCM (100 mL), and washed twice with a saturated solution of ammonium chloride. The organic layer was dried over MgSO₄, filtrated and concentrated in vacuo. The crude product was purified by flash column chromatography (silica gel, DCM/MeOH 0 to 1%) to give 758 mg (0.84 mmol, 89% yield) of the title compound. ¹H NMR (DMSO-*d₆*) δ 8.44 (1H, m), 8.15 (1H, m), 7.76 (1H, m), 7.26 (20H, m), 5.03 (8H, m), 4.47 (1H, m), 4.22 (1H, m), 3.94 (1H, m), 3.82 (2H, d), 2.82-2.66 (4H, m), 2.16 (2H, m), 1.96 (1H, m), 1.80 (1H, m), 1.29 (9H, s).

### Intermediate 97

### TFA.D-GluOBn-γ-mesoLAN(Z, OBn)-GlyOBn

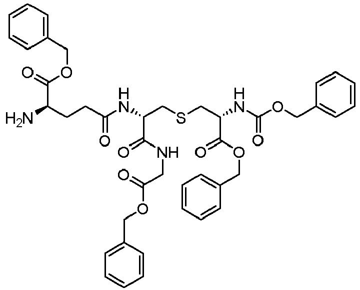

The intermediate 96 (743 mg, 0.82 mmol) was dissolved in TFA/DCM mixture (1/1) (10 mL). After stirring for 1h at rt, the solvents were removed under vacuo, the residue was coevaporated 3 times with toluene. The crude compound was triturated with Et₂O and filtered to give 688 mg (0.75 mmol, 91% yield) of the title compound. ¹H NMR (DMSO-*d₆*) δ 8.52 (1H, t), 8.35 (2H, sl), 8.20 (1H, d), 7.82 (1H, t), 7.36-7.23 (20H, m), 5.22-4.96 (8H, m), 4.45 (1H, m), 4.21 (1H, m), 4.07 (1H, t), 3.83 (2H, d), 2.95-2.86 (2H, m), 2.27 (2H, m), 1.98 (2H, m).

### Intermediate 98

### Boc-Ala-D-GluOBn-γ-mesoLAN(Z, OBn)-GlyOBn

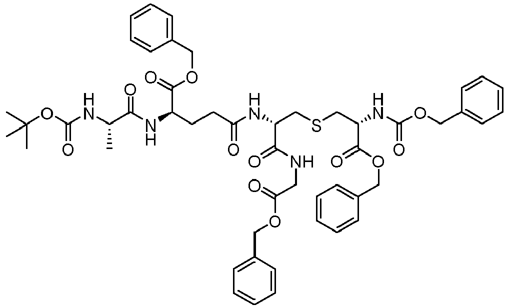

To a solution of intermediate 96 (679 mg, 0.74 mmol) in dry DCM (20 mL) were added Boc-AlaOH (140 mg, 0.74 mmol) and PyBOP (425 mg, 0.81 mmol), the pH was adjusted to 10 with NMM. The reaction mixture was stirred at rt under argon for 16h. Then the reaction was diluted with DCM (100 mL), and washed twice with a saturated solution of ammonium chloride. The organic layer was dried over MgSO₄, filtrated and concentrated in vacuo. The crude product was purified by flash column chromatography (silica gel, DCM/MeOH 0 to 1%) to give 662 mg (0.68 mmol, 91% yield) of the title compound. ¹H NMR (DMSO-*d₆*) δ 8.60 (1H,d), 8.49 (1H, d), 8.10 (1H, d), 7.79 (1H, t), 7.28-7.26 (20H, m), 5.03 (8H, m), 4.45 (1H, m), 4.25 (2H, m), 3.83 (2H, d), 3.61 (1H, m), 2.88-2.66 (4H, m), 2.16 (2H, m), 2.01 (1H, m), 1.80 (1H, m),, 1.36 (9H, s).

### Intermediate 99

### TFA.Ala-D-GluOBn-γ-mesoLAN(Z, OBn)-GlyOBn

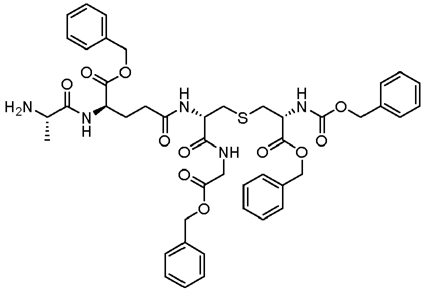

The intermediate 98 (660 mg, 0.68 mmol) was dissolved in TFA/DCM mixture (1/1) (15 mL). After stirring for 1h at rt, the solvents were removed under vacuo, the residue was coevaporated 3 times with toluene. The crude compound was purified by flash column chromatography (silica gel, DCM/MeOH 0 to 6.5%) to give 627 mg (0.63 mmol, 93% yield) of the title compound. ¹H NMR (DMSO-*d₆*) δ 8.60 (1H,d), 8.49 (1H, d), 8.10 (1H, d), 7.79 (1H, t), 7.28-7.26 (20H, m), 5.03 (8H, m), 4.45 (1H, m), 4.25 (2H, m), 3.83 (2H, d), 3.61 (1H, m), 2.88-2.66 (4H, m), 2.16 (2H, m), 2.01 (1H, m), 1.80 (1H, m).

### Intermediate 100

### C₇-Ala-D-GluOMe-γ-mesoLan(Boc, OMe)-GlyOMe

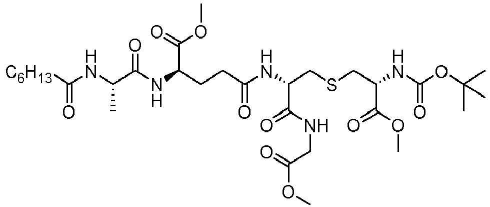

To a solution of intermediate 86 (118 mg, 0.30 mmol) in dry DMF (10 mL) were added intermediate 71 (90.0 mg, 0.26 mmol) HATU (11.0 mg, 0.29 mmol) and NMM (80 µL, 0.78 mmol). The reaction mixture was stirred at rt under argon for 16h. Then the solvent of the reaction was removed in vacuo and the residue was dissolved in EtOAc (30 mL), and washed with a saturated solution of NaHCO₃, water and brine. The organic layer was dried over MgSO₄, filtrated and concentrated in vacuo. The crude product was purified by flash column chromatography (silica gel, DCM/MeOH 0 to 2%) to give 150 mg (0.20 mmol, 80% yield) of the title compound. ¹H NMR (DMSO-*d₆*) δ 8.44 (1H, t), 8.27 (1H, d), 8.14 (1H, d), 7.90 (1H, d), 7.26 (1H, d), 4.49 (1H, m), 4.34 (1H, m), 4.24 (1H, m), 4.12 (1H, m), 3.85 (2H, d), 3.62 (6H, s), 3.61 (3H, s), 2.91-2.53 (4H, m), 2.22 (2H, t), 2.13 (2H, m), 1.97 (1H, m), 1.82 (1H, m), 1.47 (2H, m), 1.37 (9H, s), 1.30 (10H, m), 0.87 (3H, t).

### Intermediate 101

### C₁₂-Ala-D-GluOBn-γ-mesoLAN(Z, OBn)-GlyOBn

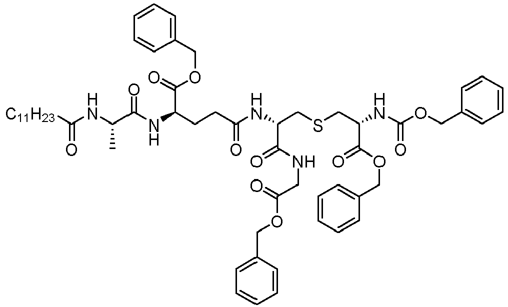

To a solution of intermediate 99 (99 mg, 0.11 mmol) in dry DCM under argon atmosphere was added TEA (21 µL, 0.15 mmol). The mixture was cooled to 0°C and a solution of lauroyl chloride (22 mg, 0.11 mmol) in dry DCM (1 mL) was added dropwise. The solution was allowed to warm to rt and stirred overnight. The reaction was diluted in DCM (20 mL), washed with NaHCO₃, 1N HCl, water, and brine. The organic layer was dried over MgSO₄, filtered and concentrated in vacuo. The crude product was purified by flash column chromatography (silica gel, DCM/MeOH 0 to 2%) to provide 82 mg (0.07 mmol, 77% yield) of the title compound. ¹H NMR (DMSO-*d₆*) δ 8.40 (1H,d), 8.23 (1H, d), 8.07 (1H, d), 7.79 (2H, m), 7.26 (20H, m), 5.03 (8H, m), 4.45 (1H, m), 4.25 (3H, m), 3.83 (2H, d), 2.87-2.66 (4H, m), 2.16 (2H, m), 2.04 (2H, m), 1.99 (1H, m), 1.75 (1H, m), 1.41 (2H, m), 1.15 (18H, m), 0.77 (3H, t).

### Intermediate 102

### C₁₄-Ala-D-GluOBn-γ-mesoLAN(Z, OBn)-GlyOBn

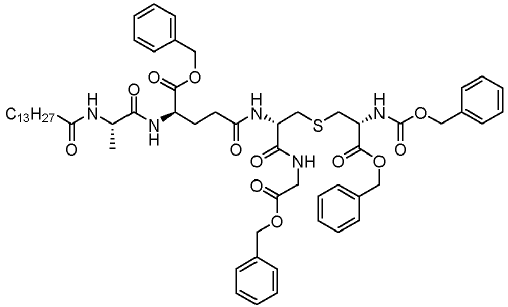

To a solution of compound 99 (9 mg, 0.10 mmol) in dry DCM under argon atmosphere was added TEA (21 µL, 0.15 mmol). The mixture was cooled to 0°C and a solution of myristoyl chloride (26 mg, 0.10 mmol) in dry DCM (1 mL) was added dropwise. The solution was allowed to warm to rt and stirred overnight. The reaction was diluted in DCM (20 mL), washed with NaHCO₃, 1N HCl, water, and brine. The organic layer was dried over MgSO₄, filtered and concentrated in vacuo. The crude product was purified by flash column chromatography (silica gel, DCM/MeOH 0 to 2%) to provide 84 mg (0.78 mmol, 78% yield) of the title compound. ¹H NMR (DMSO-*d₆*) δ 8.40 (1H,d), 8.23 (1H, d), 8.07 (1H, d), 7.79 (2H, m), 7.26 (20H, m), 5.03 (8H, m), 4.45 (1H, m), 4.25 (3H, m), 3.83 (2H, d), 2.87-2.66 (4H, m), 2.16 (2H, m), 2.01 (2H, m), 1.99 (1H, m), 1.75 (1H, m), 1.41 (2H, m), 1.15 (22H, m), 0.77 (3H, t).

### Intermediate 103

### Fmoc-Ala-D-Glu(OBn)OMe

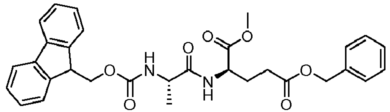

To a solution of intermediate 17 (4.40 g, 12.0 mmol) in dry DMF (50 mL) were added intermediate Fmoc-AlaOH (3.0 g, 9.60 mmol) HATU (4.0 g, 11.0 mmol) and NMM (9.78 mL, 19.3 mmol). The reaction mixture was stirred at rt under argon for 16h. Then the solvent of the reaction was removed in vacuo and the residue was dissolved in EtOAc (150 mL), and washed with a saturated solution of NaHCO₃, water and brine. The organic layer was dried over MgSO₄, filtrated and concentrated in vacuo. The crude product was purified by flash column chromatography (silica gel, DCM/MeOH 0 to 2%) to give 5.11 g (9.38 mmol, 97% yield) of the title compound. ¹H NMR (DMSO-*d₆*) δ 8.29 (1H, d), 7.88 (2H, d), 7.73 (2H, m), 7.50 (1H, d), 7.33 (9H, m), 5.06 (2H, s), 4.26 (4H, m), 4.22 (1H, m), 3.61 (3H, s), 2.41 (2H, m), 2.03 (1H, m), 1.89 (1H, m), 1.21 (3H, d).

### Intermediate 104

### Fmoc-Ala-D-GluOMe

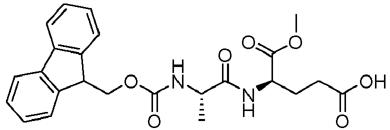

Intermediate 103 (2.0 g, 3.7 mmol) was dissolved in a mixture of THF/MeOH mixture (1/1) (40 mL) Nickel de Ranay (215 mg) was added and the mixture was stirred under hydrogen atmosphere for 2h. Then, the mixture was filtered over Celite, the celite was washed with MeOH. The filtrate was concentrated in vacuo to give 406 mg (0.89 mmol, 40% yield) of the title compound. This compound was used for the next step without any further purification. ¹H NMR (DMSO-*d₆*) δ 8.28 (1H, d), 7.88 (2H, d), 7.73 (2H, m), 7.39 (5H, m), 4.27 (4H, m), 4.12 (1H, m), 3.62 (3H, s), 2.25 (2H, m), 1.95 (1H, m), 1.79 (1H, m), 1.22 (3H, d).

### Intermediate 105

### Fmoc-Ala-D-GluOMe-γ-mesoLan(Boc, OMe)OMe

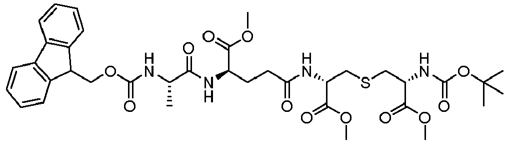

To a solution of intermediate 104 (150 mg, 0.33 mmol) in dry DMF (10 mL) were added intermediate 10 (128 mg, 0.38 mmol) HATU (140 mg, 0.36 mmol) and NMM (37 µL, 0.36 mmol). The reaction mixture was stirred at rt under argon for 16h. Then the solvent of the reaction was removed in vacuo and the residue was dissolved in EtOAc (30 mL), and washed with a saturated solution of NaHCO₃, water and brine. The organic layer was dried over MgSO₄, filtrated and concentrated in vacuo. The crude product was purified by flash column chromatography (silica gel, DCM/MeOH 0 to 2%) to give 202 mg (0.26 mmol, 79% yield) of the title compound. ¹H NMR (DMSO-*d₆*) δ 8.76 (1H, d), 8.60 (1H, d), 8.38 (1H, d), 8.31 (1H, d), 7.89 (2H, d), 7.73 (2H, t), 7.51 (2H, m), 7.44 (2H, t), 7.30 (3H, m), 4.43 (1H, m), 4.24 (3H, m), 4.09 (2H, m), 3.62 (6H, s), 3.62 (9H, s), 2.88-2.70 (4H, m), 2.21 (2H, t), 2.10 (2H, t), 1.96 (1H, m), 1.80 (1H, m), 1.37 (9H, m), 1.23 (3H, d).

### Intermediate 106

### Fmoc-Ala-D-GluOMe-γ-mesoLan(Boc, OMe)-GlyOMe

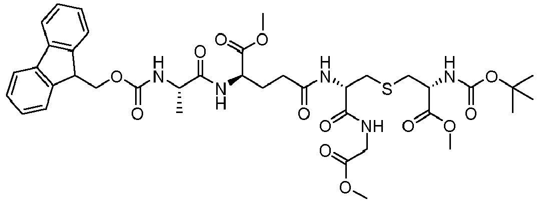

To a solution of intermediate 104 (120 mg, 0.26 mmol) in dry DMF (10 mL) were added intermediate 86 (120 mg, 0.30 mmol) HATU (110 mg, 0.29 mmol) and NMM (27 µL, 0.26 mmol). The reaction mixture was stirred at rt under argon for 16h. Then the solvent of the reaction was removed in vacuo and the residue was dissolved in EtOAc (30 mL), and washed with a saturated solution of NaHCO₃, water and brine. The organic layer was dried over MgSO₄, filtrated and concentrated in vacuo. The crude product was purified by flash column chromatography (silica gel, DCM/MeOH 0 to 2%) to give 150 mg (0.18 mmol, 68% yield) of the title compound. ¹H NMR (CDCl₃-*d₁*) δ 7.78 (2H, d), 7.62 (2H, d), 7.40 (2H, t), 7.31 (2H, t), 4.75 (1H, m), 4.60 (1H, m), 4.47 (2H, m), 4.28 (2H, m), 4.05 (2H, m), 3.62 (6H, s), 3.61 (3H, s), 2.96-2.80 (4H, m), 2.34 (2H, m), 1.84 (2H, m), 1.45 (12H, m).

### Intermediate 107

### Fmoc-Ala-D-GluOMe-γ-mesoLan(Boc, OMe)-D-AlaOMe

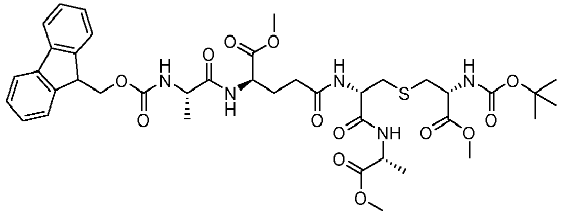

To a solution of intermediate 104 (155 mg, 0.38 mmol) in dry DMF (10 mL) were added intermediate 62 (150 mg, 0.33 mmol) HATU (140 mg, 0.36 mmol) and NMM (37 µL, 0.36 mmol). The reaction mixture was stirred at rt under argon for 16h. Then the solvent of the reaction was removed in vacuo and the residue was dissolved in EtOAc (30 mL), and washed with a saturated solution of NaHCO₃, water and brine. The organic layer was dried over MgSO₄, filtrated and concentrated in vacuo. The crude product was purified by flash column chromatography (silica gel, DCM/MeOH 0 to 2%) to give 200 mg (0.24 mmol, 72% yield) of the title compound. ¹H NMR (DMSO-*d₆*) δ 8.53 (1H, d), 8.30 (1H, d), 8.12 (1H, d), 7.88 (2H, m), 7.73 (2H, m), 7.49-7.26 (m, 6H), 4.50 (1H, m), 4.25 (5H, m), 4.12 (2H, m), 3.61 (9H, s), 2.88-2.73 (4H, m), 2.20 (2H, m), 1.95 (1H, m), 1.79 (1H, m), 1.28 (9H, m), 1.22 (6H, m).

### Intermediate 108

### H-Ala-D-GluOMe-γ-mesoLan(Boc, OMe)OMe

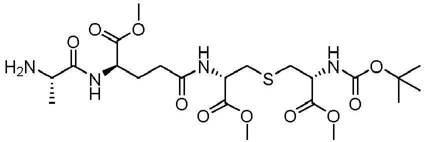

Intermediate 105 (202 mg, 0.26 mmol) was dissolved with DMF (10 mL), piperidine (5 µL, 0.05 mmol) was added. The mixture was heated at 50°C for 45 min. Then, water (20 mL) was added, and the mixture was extracted with EtOAc 3 times. The organic layer was washed with water (3 times) and brine, dried over MgSO₄, filtered and concentrated in vacuo. The crude compound was purified by flash column chromatography (silica gel, DCM/MeOH 5% to 10%) to provide 54 mg (0.09 mmol, 37% yield) of the title compound. ¹HNMR (MeOD-*d₁*) δ 8.65 (1H, d), 8.33 (1H, d), 7.45 (1H, m), 4.66 (1H, m), 4.49 (1H, m), 4.46 (1H, m), 4.0.1 (1H, m), 3.65 (9H, s), 3.18-2.87 (4H, m), 2.40 (2H, m), 2.25 (1H, m), 2.00 (1H, m), 1.55 (3H, d), 1.46 (9H, s).

### Intermediate 109

### H-Ala-D-GluOMe-γ-mesoLan(Boc, OMe)-GlyOMe

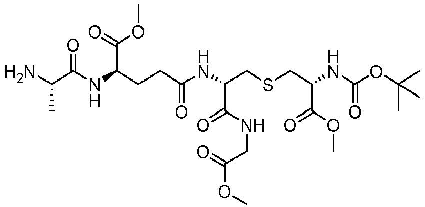

Intermediate 106 (150 mg, 0.18 mmol) was dissolved with DMF (10 mL), piperidine (3µL, 0.03 mmol) was added. The mixture was heated at 50°C for 45 min. Then, water (20 mL) was added, and the mixture was extracted with EtOAc 3 times. The organic layer was washed with water (3 times) and brine, dried over MgSO₄, filtered and concentrated in vacuo. The crude compound was purified by flash column chromatography (silica gel, DCM/MeOH 2% to 5%) to provide 74 mg (0.12 mmol, 67% yield) of the title compound. ¹HNMR (DMSO-*d₆*) δ 8.65 (1H, d), 8.33 (1H, d), 7.45 (1H, m), 4.66 (1H, m), 4.49 (1H, m), 4.46 (1H, m), 3.86 (2H, d), 3.65 (9H, s), 3.18-2.87 (4H, m), 2.40 (2H, m), 2.25 (1H, m), 2.01 (1H, m), 1.54 (3H, d), 1.47 (9H, s).

### Intermediate 110

### H-Ala-D-GluOMe-γ-mesoLan(Boc, OMe)-D-AlaOMe

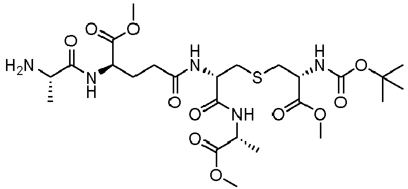

Intermediate 107 (200 mg, 0.24 mmol) was dissolved with DMF (10 mL), piperidine (5µL, 0.04 mmol) was added. The mixture was heated at 50°C for 45 min. Then, water (20 mL) was added, and the mixture was extracted with EtOAc 3 times. The organic layer was washed with water (3 times) and brine, dried over MgSO₄, filtered and concentrated in vacuo. The crude compound was purified by flash column chromatography (silica gel, DCM/MeOH 2% to 5%) to provide 101 mg (0.16 mmol, 69% yield) of the title compound. ¹HNMR (MeOD) δ 4.61 (1H, m), 4.49 (2H, m), 4.36 (1H, m), 3.74 (9H, s), 3.50 (1H, m), 3.04-2.76 (4H, m), 2.40 (2H, m), 2.28 (1H, m), 1.95 (1H, m), 1.46 (9H, d), 1.35 (3H, d), 1.19 (3H, d).

### Intermediate 111

### Ac-Lac-D-GluOMe-γ-mesoLan(Boc, OMe)-GlyOMe

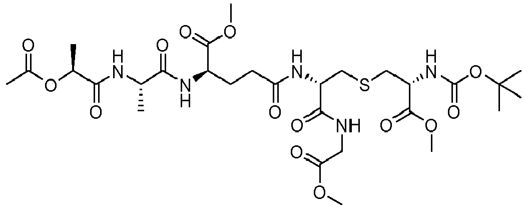

To a solution of intermediate 109 (115 mg, 0.18 mmol) in dry DMF (5 mL) were added intermediate (-)-O-Acetyl-L-lactic acid (25.0 mg, 0.18 mmol) HATU (79 mg, 0.21 mmol) and NMM (20 µL, 0.19 mmol). The reaction mixture was stirred at rt under argon for 16h. Then the solvent of the reaction was removed in vacuo and the residue was dissolved in EtOAc (20 mL), and washed with a saturated solution of NaHCO₃, water and brine. The organic layer was dried over MgSO₄, filtrated and concentrated in vacuo. The crude product was purified by flash column chromatography (silica gel, DCM/MeOH 0 to 2%) to give 81 mg (0.11 mmol, 59% yield) of the title compound. ¹H NMR (DMSO-*d₆*) δ 8.45 (1H, t), 8.28 (1H, d), 8.11 (2H, m), 7.27 (1H, d), 4.95 (1H, q), 4.10 (1H, m), 3.86 (3H, d), 3.62 (9H, s), 2.89-2.68 (4H, m), 2.18 (2H, t), 2.03 (3H, s), 1.98 (1H, m), 1.80 (1H, m), 1.37 (9H, s), 1.26 (3H, d), 1.22 (3H, d).

### Intermediate 112

### 1-OBn-4,6-0-Benzylidene-MurNAc-Ala-D-Glu(OBn)Ome-γ-mesoLAN(Boc, OMe)OMe

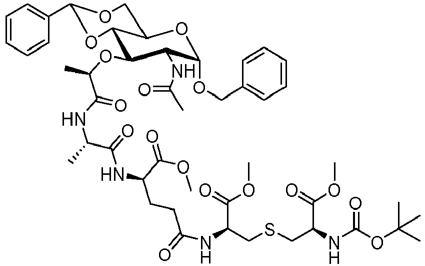

Benzyl-2-acetamido-4,6-0-dibenzylidene-2-deoxy-3-0-[(R)-1-carboxyethyl]-α-D-glucopyranoside (106 mg, 0.22 mmol; prepared by the process set forth in Gross et al., Liebigs Ann. Chem., 37-45 (1986)) was suspended in dry DMF (4 mL), then were added compound 108 (124 mg, 0.22 mmol) HATU (94 mg, 0.25 mmol) and NMM (24 µL, 0.23 mmol). The reaction mixture was stirred at rt under argon for 2h. Then the solvent of the reaction was removed in vacuo and the residue was dissolved in EtOAc (15 mL), and washed with a saturated solution of NaHCO₃, water and brine. The organic layer was dried over MgSO₄, filtrated and concentrated in vacuo. The crude product was purified by flash column chromatography (silica gel, DCM/MeOH 0 to 2%) to give 129 mg (0.13 mmol, 57% yield) of the title compound. ¹H NMR (DMSO-*d₆*) δ 8.44 (1H, d), 8.39 (1H, d), 7.55 (1H, d), 7.38 (11H, m), 5.70 (1H, s), 4.88 (1H, d), 4.48 (2H, m), 4.44-4.19 (6H, m), 3.97 (1H, m), 3.73 (4H, m), 3.62 (6H, s), 3.60 (3H, s), 2.88-2.73 (4H, m), 2.20 (2H, t), 2.08 (1H, m), 1.79 (4H, m), 1.37 (9H, s), 1.21 (6H, t).

### Intermediate 113

### 1-OBn-4,6-0-Benzylidene-MurNAc-Ala-D-GluOMe-γ-mesoLAN(Boc, OMe)-GlyOMe

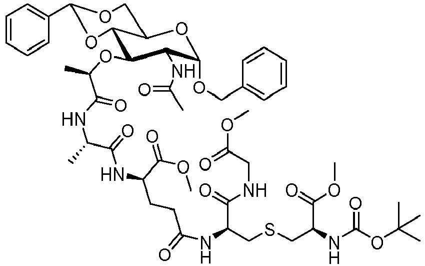

Benzyl-2-acetamido-4,6-0-dibenzylidene-2-deoxy-3-0-[(R)-1-carboxyethyl]-α-D-glucopyranoside (44 mg, 0.09 mmol was suspended in dry DMF (5 mL), then were added compound 109 (56 mg, 0.09 mmol) HATU (39 mg, 0.10 mmol) and NMM (10 µL, 0.10 mmol). The reaction mixture was stirred at rt under argon for 2h. Then the solvent of the reaction was removed in vacuo and the residue was dissolved in EtOAc (15 mL), and was filtrated, washed with Et₂O and dried in vacuo to give 72 mg (0.06 mmol, 72% yield) of the title compound. ¹H NMR (DMSO-*d₆*) δ 8.46 (2H, d), 8.18 (2H, d), 7.35 (1H, d), 7.32 (11H, m), 5.70 (1H, s), 4.87 (1H, d), 4.48 (3H, m), 4.38-4.28 (5H, m), 4.01 (1H, m), 3.85 (2H, d), 3.72 (4H, m), 3.62 (3H, s), 3.61 (3H, s), 3.60 (3H, s), 2.83-2.72 (4H, m), 2.17 (2H, t), 2.08 (2H, m), 1.79 (3H, s), 1.37 (9H, s), 1.21 (6H, t).

### Intermediate 113

### 1-OBn-4,6-0-Benzylidene-MurNAc-Ala-D-GluOMe-γ-mesoLAN(Boc, OMe)-D-AlaOMe

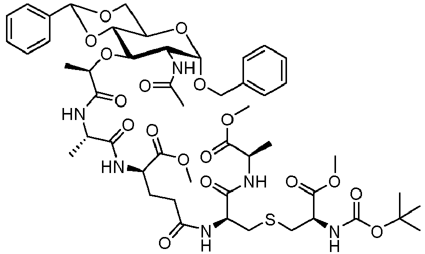

Benzyl-2-acetamido-4,6-0-dibenzylidene-2-deoxy-3-0-[(R)-1-carboxyethyl]-α-D-glucopyranoside (65 mg, 0.14 mmol was suspended in dry DMF (5 mL), then were added compound 110 (98 mg, 0.16 mmol) HATU (58 mg, 0.15 mmol) and NMM (42 µL, 0.41 mmol). The reaction mixture was stirred at rt under argon for 2h. Then the solvent of the reaction was removed in vacuo and the residue was dissolved in EtOAc (15 mL), and was filtrated, washed with Et₂O and dried in vacuo to give 95 mg (0.09 mmol, 65% yield) of the title compound. ¹H NMR (DMSO-*d₆*) δ 8.55 (1H, d), 8.45 (1H, d),8.15 (2H, d), 7.55 (1H, d), 7.30 (11H, m), 5.70 (1H, s), 4.87 (1H, d), 4.70 (1H, m),4.50 (2H, m), 4.30-4.17 (6H, m), 3.98 (1H, m), 3.85 (2H, d), 3.89 (4H, m), 3.61 (9H, s), 3.06-2.74 (4H, m), 2.27 (2H, t), 2.08 (2H, m), 1.79 (3H, s), 1.37 (9H, s), 1.22 (9H, m).

### Intermediate 115

### Tr-SerOMe

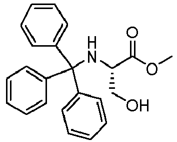

To a suspension of L-Serine methyl ester hydrochloride (7.5 g, 48.0 mmol) in DCM (100 ml) at 0°C TEA (13.8 ml, 96.0 mmol) was added dropwise followed by triphenylmethyl chloride (13.44 g, 48.0 mmol) in DCM (30 ml). After stirring at 4 °C for 4 h, the mixture was allowed to warm to rt and the stirring was maintained overnight. The white precipitate formed was filtered off and the filtrate evaporated *in vacuo* to yield a white solid, which was dissolved in EtOAc (100 ml) and washed with saturated NaHCO₃, 10% citric acid and water. The organic layer was dried over Na₂SO₄, filtered and concentrated *in vacuo* to give 17.09 g (47.3 mmol, 98% yield) of the title compound as a white solid which was used for the next step without any further purification. ¹H NMR (DMSO-*d₆*) δ 7.44-7.17 (15H, m), 4.93 (1H, t), 3.59 (1H, m), 3.44 (1H, m), 3.21 (1H, m), 3.13 (3H, s), 2.80 (1H, d).

### Intermediate 116

### Tr-AziOMe

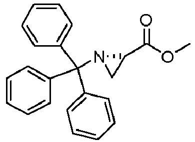

TEA (7.11 ml, 48.69 mmol) was added dropwise over 10 min to a stirred solution of intermediate 115 (8.00 g, 22.13 mmol) in THF (200 ml) at 0 °C, followed by dropwise addition of methanesulfonyl chloride (1.88 ml, 24.34 mmol) and the solution was stirred at 0°C for 30 min and at reflux overnight. After completion of the reaction, solvent was removed under reduced pressure. The resulting residue was dissolved in EtOAc (200 mL) and washed with 10% citric acid, water, saturated Na₂CO₃ and brine. The organic layer was dried over Na₂SO₄, filtered and concentrated *in vacuo.* The crude product was purified by flash chromatography (silica gel, petroleum ether/ EtOAc 0 to 2%) to provide 6.05 g (17.6 mmol, 79% yield) of the title compound. ¹H NMR (DMSO-*d₆*) δ 7.36-7.19 (15H, m), 3.69 (3H, s), 2.24 (1H, d), 1.71 (1H, m), 1.32 (1H, m).

### Intermediate 117

### pNZ-AziOMe

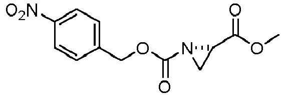

Trifluoroacetic acid (2.72 ml, 35.53 mmol) was added dropwise over 10 min to a solution of intermediate 116 (6.10 g, 17.8 mmol) in dry DCM (100 ml) and dry MeOH (791 µl) at 0°C. The solution was stirred for 30min at 0°C. Then TEA (12.86 mL, 91.48 mmol) was added dropwise over a period of 10min followed by succinimidyl 4-nitrobenzyl carbonate (5.22 g, 17.76 mmol) at 0°C. The resulting mixture was warmed to rt and stirred vigorously for 18h. After completion of the reaction, the mixture was washed with citric acid, water and brine, the organic layer was dried over Na₂SO₄, filtered and concentrated *in vacuo.* The crude product was further purified by flash chromatography (silica gel, DCM/MeOH 0 to 0.5%) to give 3.45 g (12.3 mmol, 69%) of the title compound as a colorless oil. ¹H NMR (CDCl₃) δ 8.25 (2H, d), 7.55 (2H, d), 5.27 (2H, m), 3.78 (3H, s), 3.17 (1H, m), 2.65 (1H, m), 2.55 (1H, m).

### Intermediate 118

Pht-D-SerOMe

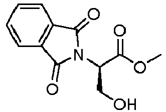

To a solution of D-serine methyl ester hydrochloride (4.00 g, 25.71 mmol) in dry toluene (40 mL) were added phthalic anhydride (3.81 g, 25.71 mmol) and TEA (3.61 mL, 25.71 mmol). The reaction mixture was refluxed under Dean-Stark condition for 2 h. Volatiles were evaporated under reduced pressure and the residue was dissolved in EtOAc, washed with 10% citric acid, water, saturated NaHCO₃ and brine. Organic layer was dried over Na₂SO₄, filtered and concentrated. The residue was purified by flash column chromatography (silica gel, DCM/MeOH 0 to 1%) to provide 4.14 g (16.60 mmol, 64% yield) of the title compound. ¹H NMR (CDCl₃) δ 7.91 (2H, m), 7.79 (2H, m), 5.06 (1H, m), 4.23 (2H, m), 3.81 (3H, s).

### Intermediate 119

### pNZ-mesoOxaDAP(Pht, OMe)OMe

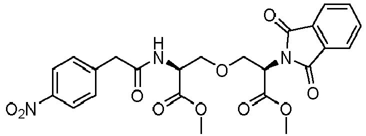

To a stirred solution of intermediate 117 (2.08 g, 7.41 mmol) and oxygen nucleophile 118 (3.69 g, 14.83 mmol) in dry toluene (40 mL) BF₃.OEt₂ (0.974 mL, 3.71 mmol) was added dropwise at rt. The resulting reaction mixture was refluxed at 110°C for 4h. After completion of the reaction, the solvent was removed under reduced pressure and the crude product was purified by flash column chromatography (silica gel, Petroleum ether/EtOAc 8/2 to 1/1) to provide 1.92 g (3.63 mmol, 49% yield) of the title compound. ¹H NMR (CDCl₃) δ 8.24 (2H, d), 7.89 (2H, m), 7.77 (2H, m), 7.54 (2H, d), 5.75 (1H, d), 5.23 (2H, m), 5.11 (1H, m), 4.44 (1H, m), 4.15 (2H, m), 3.88 (1H, m), 3.81 (1H, m), 3.77 (3H, s), 3.51 (3H, s).

### Intermediate 120

### H-mesoOxaDAP(Pht, OMe)OMe

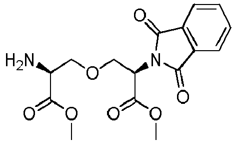

Intermediate 119 (3.04 g, 5.74 mmol) was dissolved in a mixture of MeOH/THF (5/1) (30 ml), followed by addition of 10% Pd/C (306 mg) and stirred for 2 h at rt under hydrogen atmosphere. The reaction mixture was filtered through celite and the filtrate was concentrated *in vacuo.* The crude product was purified by flash column chromatography (silica gel, DCM/MeOH 0 to 2%) to give 1.70 g (4.85 mmol, 84% yield) of the title compound. ¹H NMR (CDCl₃) δ 7.90 (2H, m), 7.77 (2H, m), 5.16 (1H, m), 4.17 (1H, m), 3.84 (1H, m), 3.77 (1H, m), 3.68 (3H, s), 3.52 (3H, s), 3.46 (1H, m).

### Intermediate 121

### Boc-D-GluOBn-γ-mesoOxaDAP(Pht, OMe)OMe

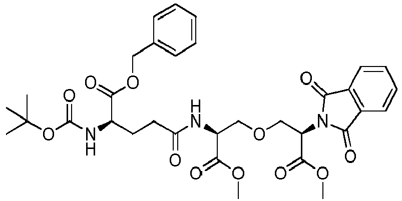

To a solution of intermediate 120 (1.69 g, 4.83 mmol) in dry DCM (50 mL) were added Boc-D-GluOBn (1.62 g, 4.83 mmol) and PyBOP (2.76 g, 5.31 mmol), the pH was adjusted to 10 with NMM. The reaction mixture was stirred at rt under argon for 18h. Then the reaction was diluted with DCM (100 mL), and washed twice with a saturated solution of ammonium chloride. The organic layer was dried over MgSO₄, filtrated and concentrated in vacuo. The crude product was purified by flash column chromatography (silica gel, DCM/MeOH 0 to 1%) to give 2.70 g (4.03 mmol, 83% yield) of the title compound. ¹H NMR (CDCl₃) δ 7.90 (2H, m), 7.77 (2H, m), 7.38 (5H, m), 6.60 (1H, m), 5.45 (1H, m), 5.18 (2H, m), 5.10 (1H, m), 4.65 (1H, m), 4.38 (1H, m), 4.12 (2H, m), 3.90 (1H, m), 3.77 (3H, s), 3.71 (1H, m), 3.49 (3H, s), 2.34 (2H, m), 2.22 (1H, m), 2.05 (1H, m), 1.43 (9H, s).

### Intermediate 122

### Boc-D-GluOBn-γ-mesoOxaDAP(OMe)OMe

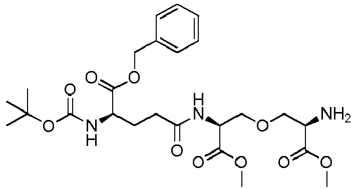

Intermediate 121 (200 mg, 0.30 mmol) was dissolved in MeOH (5 ml), followed by addition of hydrazine acetate (44 mg, 0.48 mmol) and the mixture was stirred under reflux for 4h. The reaction mixture was concentrated *in vacuo.* The residue was triturated with DCM, filtered and the filtrate was concentrated in vacuo. The crude product was purified by flash column chromatography (silica gel, DCM/MeOH 0 to 3%) to give 160 mg (0.30 mmol, 100% yield) of the title compound. ¹H NMR (CDCl₃) δ 7.36 (5H, m), 5.21 (2H, s), 4.74 (2H, m), 4.28 (1H, m), 4.00 (2H, m), 3.82-3.72 (5H, m), 3.65 (3H, s), 2.40-3.32 (3H, m), 2.13 (1H, m), 1.45 (9H, s).

### Intermediate 123

### Boc-D-Glu-γ-mesoOxaDAP(OMe)OMe

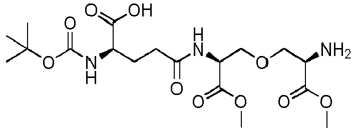

Intermediate 122 (160 mg, 0.30 mmol) was dissolved in a mixture of MeOH/THF (1/1) (10 ml), followed by addition of 10% Pd/C (33 mg) and stirred for 2 h at rt under hydrogen atmosphere. The reaction mixture was filtered through celite and the filtrate was concentrated *in vacuo.* The crude product was triturated with Et₂O to give 80 mg (0.18 mmol, 57% yield) of the title compound which was used for the next step without any further purification. ¹H NMR (DMSO-*d₆*) δ 8.45 (1H, m), 6.82 (1H, m), 4.50 (1H, m), 3.98 (2H, m), 3.75 (2H, m), 3.72 (2H, m), 3.62 (3H, s), 3.55 (3H, s), 2.21 (2H, m), 1.90 (1H, m), 1.75 (1H, m), 1.34 (9H, s).

### Intermediate 124

### TFA.D-GluOBn-γ-mesoOxaDAP(Pht, OMe)OMe

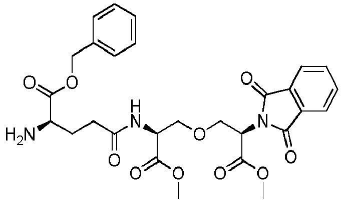

Intermediate 121 (597 mg, 0.89 mmol) was dissolved in TFA/DCM mixture (1/1) (20 mL). After stirring for 2h at rt, the solvents were removed under *vacuo;* the residue was coevaporated 3 times with toluene. The crude compound was triturated with Et₂O to give 590 mg (0.86 mmol, 97% yield) of the title compound which was used for the next step without any further purification. ¹H NMR (CDCl₃) δ 7.88 (2H, m), 7.75 (2H, m), 7.50 (1H, d), 7.34 (5H, m), 5.27 (2H, m), 5.14 (1H, m), 4.57 (1H, m), 4.35 (1H, m), 4.08 (2H, m), 3.90 (1H, m), 3.76 (4H, m), 3.41 (3H, s), 2.63 (2H, m), 2.45 (1H, m), 2.33 (1H, m).

### Intermediate 125

### C₁₄-D-GluOBn-γ-mesoOxaDAP(Pht, OMe)OMe

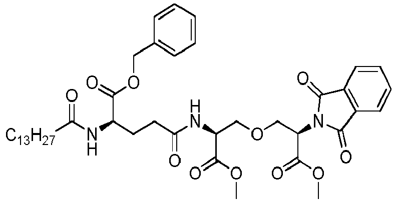

To a solution of intermediate 123 (200 mg, 0.29 mmol) in dry DCM (10 mL) under argon atmosphere was added TEA (61 µL, 0.44 mmol). The mixture was cooled to 0°C and a solution of myristoyl chloride (75 mg, 0.30 mmol) in dry DCM (1 mL) was added dropwise. The solution was allowed to warm to rt and stirred overnight. The reaction was diluted in DCM (30 mL), washed with NaHCO₃, 1N HCl, water, and brine. The organic layer was dried over MgSO₄, filtered and concentrated *in vacuo.* The crude product was purified by flash column chromatography (silica gel, DCM/MeOH 0 to 2%) to provide 170 mg (0.22 mmol, 75% yield) of the title compound. ¹H NMR (CDCl₃) δ 7.89 (2H, m), 7.77 (2H, m), 7.37 (5H, m), 6.78 (1H, d), 6.67 (1H, m), 5.19 (2H, s), 5.11 (1H, m), 4.66 (2H, m), 4.30-3.90 (3H, m), 3.75 (4H, m), 3.49 (3H, s), 2.35 (2H, m), 2.23 (3H, m), 2.13 (1H, m), 1.63 (2H, m), 1.26 (20H, s), 0.90 (3H, t).

### Intermediate 126

### C₁₄-D-GluOBn-γ-mesoOxaDAP(OMe)OMe

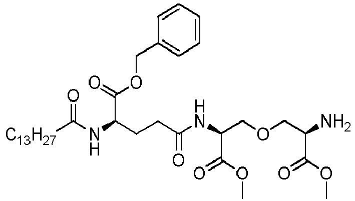

Intermediate 125 (159 mg, 0.20 mmol) was dissolved in MeOH (8 ml), followed by addition of hydrazine acetate (28 mg, 0.30 mmol) and the mixture was stirred under reflux for 4h. The reaction mixture was concentrated *in vacuo.* The residue was triturated with DCM, filtered and the filtrate was concentrated in vacuo. The crude product was purified by flash column chromatography (silica gel, DCM/MeOH 0 to 3%) to give 79 mg (0.12 mmol, 60% yield) of the title compound. ¹H NMR (CDCl₃) δ 7.37 (5H, m), 5.20 (2H, s), 4.75 (2H, m), 4.28 (1H, m), 4.00 (2H, m), 3.82-3.72 (5H, m), 3.67 (3H, s), 2.40-2.20 (5H, m), 2.03 (1H, m), 1.63 (2H, m), 1.28 (20H, s), 0.90 (3H, t).

### Intermediate 127

### C₁₄-D-Glu-γ-mesoOxaDAP(OMe)OMe

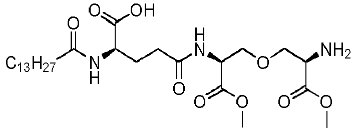

Intermediate 126 (75 mg, 0.11 mmol) was dissolved in MeOH (5 ml), followed by addition of 10% Pd/C (12 mg) and stirred for 1h at rt under hydrogen atmosphere. The reaction mixture was filtered through celite and the filtrate was concentrated *in vacuo.* The crude product was triturated with Et₂O to give 63 mg (0.11 mmol, 97% yield) of the title compound which was used for the next step without any further purification. ¹H NMR (CDCl₃) δ 4.67 (2H, m), 4.18 (1H, m), 4.00 (2H, m), 3.80-3.68 (5H, m), 3.57 (3H, s), 2.40-2.20 (5H, m), 2.00 (1H, m), 1.64 (2H, m), 1.28 (20H, s), 0.88 (3H, t).

### Intermediate 128

### Boc-Ala-D-GluOBn-γ-mesoOxaDAP(Pht, OMe)OMe

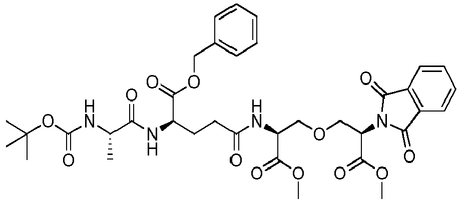

To a solution of intermediate 124 (400 mg, 0.58 mmol) in dry DCM (10 mL) were added Boc-AlaOH (111 mg, 0.58 mmol) and PyBOP (335 mg, 0.64 mmol), the pH was adjusted to 10 with NMM. The reaction mixture was stirred at rt under argon for 18h. Then the reaction was diluted with DCM (50 mL), and washed twice with a saturated solution of ammonium chloride. The organic layer was dried over MgSO₄, filtrated and concentrated *in vacuo.* The crude product was purified by flash column chromatography (silica gel, DCM/MeOH 0 to 2%) to give 344 mg (0.46 mmol, 79% yield) of the title compound. ¹H NMR (CDCl₃) δ 7.89 (2H, m), 7.76 (2H, m), 7.36 (5H, m), 7.05 (1H, m), 5.18 (2H, m), 5.11 (1H, m), 4.65 (1H, m), 4.52 (1H, m), 4.30-3.90 (4H, m), 3.77 (4H, m), 3.46 (3H, s), 2.31 (2H, m), 2.22 (1H, m), 2.05 (1H, m), 1.46 (9H, s), 1.37 (3H, d).

### Intermediate 129

### Boc-Ala-D-GluOBn-γ-mesoOxaDAP(OMe)OMe

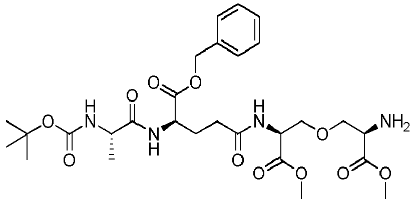

Intermediate 128 (173 mg, 0.23 mmol) was dissolved in MeOH (5 ml), followed by addition of hydrazine acetate (34 mg, 0.37 mmol) and the mixture was stirred under reflux for 4h. The reaction mixture was concentrated *in vacuo.* The residue was triturated with DCM, filtered and the filtrate was concentrated in vacuo. The crude product was purified by flash column chromatography (silica gel, DCM/MeOH 0 to 4%) to give 124 mg (0.20 mmol, 87% yield) of the title compound. ¹H NMR (DMSO-*d₆*) δ 7.89 (1H, d), 7.76 (1H, d), 7.36 (5H, m), 7.05 (1H, t), 5.15 (2H, m), 5.11 (1H, m), 4.65 (1H, m), 4.52 (1H, m), 4.30-3.90 (4H, m), 3.62 (6H, s), 2.31 (2H, m), 2.22 (1H, m), 2.05 (1H, m), 1.46 (9H, s), 1.37 (3H, d).

### Intermediate 130

### Boc-Ala-D-Glu-γ-mesoOxaDAP(OMe)OMe

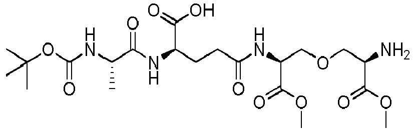

Intermediate 129 (114 mg, 0.19 mmol) was dissolved in a mixture of MeOH/THF (1/1) (7 ml), followed by addition of 10% Pd/C (20 mg) and stirred for 2 h at rt under hydrogen atmosphere. The reaction mixture was filtered through celite and the filtrate was concentrated *in vacuo.* The crude product was triturated with Et₂O to give 96 mg (0.18 mmol, 99% yield) of the title compound which was used for the next step without any further purification.

### Intermediate 131

### TFA.Ala-D-GluOBn-γ-mesoOxaDAP(Pht, OMe)OMe

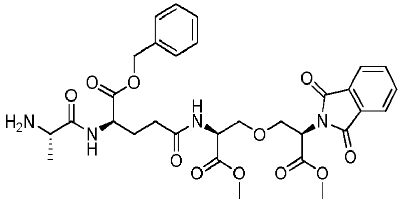

Intermediate 128 (168 mg, 0.23 mmol) was dissolved in TFA/DCM mixture (1/1) (10 mL). After stirring for 3h at rt, the solvents were removed under *vacuo;* the residue was coevaporated 3 times with toluene. The crude compound was triturated with Et₂O to give 171 mg (0.22 mmol, 99% yield) of the title compound which was used for the next step without any further purification. ¹H NMR (CDCl₃) δ 8.20 (1H, m), 7.86 (2H, m), 7.75 (2H, m), 7.51 (1H, m), 7.36 (5H, m), 5.15 (3H, m), 4.62 (1H, m), 4.46 (1H, m), 4.28 (1H, m), 4.11 (2H, m), 3.90 (1H, m), 3.75 (4H, m), 3.34 (3H, s), 2.28 (2H, m), 2.12 (1H, m), 2.05 (1H, m), 1.27 (3H, d).

### Intermediate 132

### C₁₄-Ala-D-GluOBn-γ-mesoOxaDAP(Pht, OMe)OMe

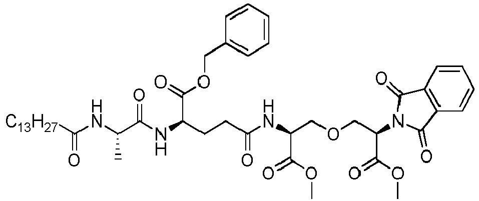

To a solution of intermediate 131 (160 mg, 0.21 mmol) in dry DCM (10 mL) under argon atmosphere was added TEA (44 µL, 0.32 mmol). The mixture was cooled to 0°C and a solution of myristoyl chloride (55 mg, 0.22 mmol) in dry DCM (1 mL) was added dropwise. The solution was allowed to warm to rt and stirred overnight. The reaction was diluted in DCM (30 mL), washed with NaHCO₃, 1N HCl, water, and brine. The organic layer was dried over MgSO₄, filtered and concentrated in vacuo. The crude product was purified by flash column chromatography (silica gel, DCM/MeOH 0 to 2%) to provide 144 mg (0.17 mmol, 80% yield) of the title compound. ¹H NMR (CDCl₃) δ 7.85 (2H, m), 7.76 (2H, m), 7.45 (1H, m), 7.34 (5H, m), 7.03 (1H, m), 6.013 (1H, m), 5.18 (2H, m), 5.11 (1H, m), 4.67-4.49 (3H, m), 4.22-3.93 (3H, m), 3.77 (4H, m), 3.46 (3H, s), 2.34-2.14 (5H, m), 1.60 (2H, m), 1.38-1.24 (23H, m), 0.90 (3H, t).

### Intermediate 133:

### C₁₄-Ala-D-GluOBn-γ-mesoOxaDAP(OMe)OMe

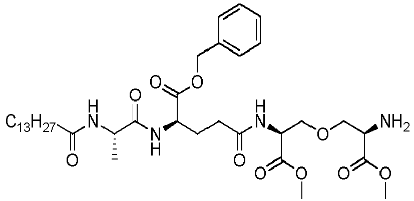

Intermediate 132 (139 mg, 0.16 mmol) was dissolved in MeOH (5 ml), followed by addition of hydrazine acetate (22 mg, 0.24 mmol) and the mixture was stirred under reflux for 4h. The reaction mixture was concentrated *in vacuo.* The residue was triturated with DCM, filtered and the filtrate was concentrated in vacuo. The crude product was purified by flash column chromatography (silica gel, DCM/MeOH 0 to 4%) to give 150 mg (0.15 mmol, 92% yield) of the title compound. ¹H NMR (CDCl₃) δ 8.10-7.86 (2H, m), 7.36 (5H, m), 6.74 (1H, m), 6.62 (1H, m), 5.11 (2H, m), 4.81-4.49 (3H, m), 4.15-3.88 (3H, m), 3.67-3.64 (8H, m), 2.35-2.02 (5H, m), 1.60 (2H, m), 1.45-1.24 (23H, m), 0.87 (3H, t).

### Intermediate 134

### C₁₄-Ala-D-Glu-γ-mesoOxaDAP(OMe)OMe

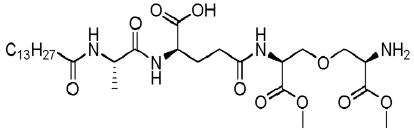

Intermediate 133 (108 mg, 0.15 mmol) was dissolved in a mixture of MeOH/THF (1/1) (5 ml), followed by addition of 10% Pd/C (16 mg) and stirred for 2h at rt under hydrogen atmosphere. The reaction mixture was filtered through celite and the filtrate was concentrated *in vacuo.* The crude product was triturated with Et₂O to give 53 mg (0.084 mmol, 55% yield) of the title compound which was used for the next step without any further purification. ¹H NMR (CDCl₃) δ 8.10-7.77 (2H, m), 6.84 (1H, m), 6.72 (1H, m), 4.83-4.45 (3H, m), 4.15-3.89 (3H, m), 3.70-3.65 (8H, m), 2.35-2.02 (5H, m), 1.61 (2H, m), 1.45-1.24 (23H, m), 0.88 (3H, t).

### EXAMPLES

### Example A

General protocol for cleavage of the benzyl and benzyloxycarbonyl group:

To a solution of the protected derivative (0.18 mmol) in dry DCM (5 mL) was added a mixture of 1,2-ethanedithiol (EDT) (0.5 mL), thioanisole (1.17 mL) and TFA (7.5 mL), the mixture was cooled to 0°C. Trimethylsilyl bromide (1.32 mL) was added dropwise within 1h to the mixture. The solution was allowed to warm to rt and stirred overnight. Then the mixture was triturated with water and DCM, the precipitate was filtrated, washed with EtOH, EtOAc and Et₂O. All compounds are obtained as trifluoroacetate salt.

### Example B

### General protocol for cleavage of the methyl ester and tert-butyloxycarbonyl group:

To a solution of protected intermediate in dioxane (5 mL) was added a solution of lithium hydroxyde (1 M), the mixture was stirred at rt overnight or at 50°C for 2H00. Then the mixture was acidified (pH 4) by addition of Amberlite. The mixture was filtered and the resine was washed with MeOH, then the solvent was removed in vacuo and the residue was directly dissolved in TFA (5 mL). The mixture was stirred for 2H00 at RT. Then the solvents were removed under vacuo; the residue was coevaporated 3 times with toluene. The crude compound was triturated with Et₂O to provide the title compound as trifluoroacetate salt.

### Example C

### General protocol for cleavage of the methyl ester:

The protected intermediate was dissolved in 6 N HCl and was refluxed for 5 h. After extraction with Et₂O, the aqueous layer was concentrated to yield a yellow oil. The crude product was purified by ion-exchange chromatography (Biorad AG 50W-X8 hydrogen form resin) by loading with distilled H₂O, flushing 3 column volume with de-ionized water, followed by elution with NH₄OH (2N) than the fraction was lyophilized. All compounds are obtained as ammonium salt.

### Example 1

### TFA N⁵-((S)-2-(((R)-2-amino-2-carboxyethyl)thio)-1-carboxyethyl)-D-glutamine

### (D-Glu-γ-mesoLANOH (iE-LAN))

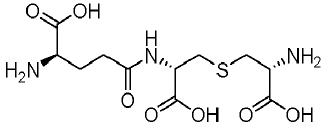

Example 1 was prepared from intermediate 13 using a similar procedure to that described in Example A, to provide 35 mg (0.08 mmol, 64% yield) of the the title compound. ¹H NMR (D₂O) δ 4.26 (1H, m), 3.37 (1H, m), 3.65 (1H, m), 3.03-2.76 (4H, m), 2.34 (2H, m), 2.01 (2H, m). MS (+)-ES [M+H]⁺ 338 m/z.

### Example 2

### TFA N⁵-((S)-2-(((R)-2-amino-2-carboxyethyl)thio)-1-carboxyethyl)-N²-heptanoyl-D-glutamine

### [C₇-D-Glu-γ-mesoLANOH (C₇-iE-LAN)]

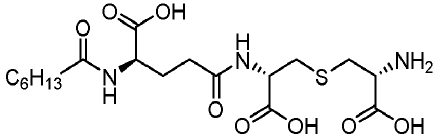

Example 2 was prepared from intermediate 20 using a similar procedure to that described in Example B, to provide 33 mg (0.074 mmol, 55% yield) of the title compound. ¹H NMR (DMSO-*d₆*) δ 4.00 (2H, m), 3.45 (3H, m), 3.17 (2H, m), 2.81 (4H, m), 2.09 (4H, m), 1.82 (2H, m), 1.46 (2H, m), 1.27 (7H, m), 0.88 (3H, t). MS (+)-ES [M+H]⁺ 450 m/z.

### Example 3

### TFA N⁵-((S)-2-(((R)-2-amino-2-carboxyethyl)thio)-1-carboxyethyl)-N²-dodecanoyl-D-glutamine

### [C₁₂-D-Glu-γ-mesoLANOH (C₁₂-iE-LAN)]

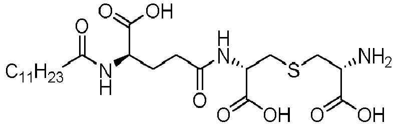

Example 3 was prepared from intermediate 14 using a similar procedure to that described in Example A, to provide 35 mg (0.067 mmol, 80% yield) of the title compound. ¹H NMR (DMSO-*d₆*) δ 4.47 (1H, m), 4.38 (1H, m), 4.25 (1H, m), 4.07 (1H, m), 3.55-3.05 (4H, m), 2.13 (2H, m), 2.02 (2H, m), 1.96 (1H, m), 1.75 (1H, m), 1.40 (2H, m), 1.16 (16H, m), 0.77 (3H, t). MS (+)-ES [M+H]⁺ 520 m/z.

### Example 4

### TFA N⁵-((S)-2-(((R)-2-amino-2-carboxyethyl)thio)-1-carboxyethyl)-N²-tetradecanoyl-D-glutamine

### [.C₁₄-D-Glu-γ-mesoLANOH (C₁₄-iE-LAN)]

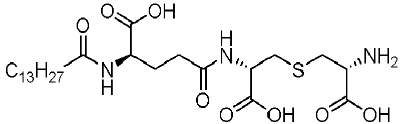

Example 4 was prepared from intermediate 17 using a similar procedure to that described in Example A, to provide 45 mg (0.082 mmol, 55% yield) of the title compound. ¹H NMR (DMSO-*d₆*) δ 4.25 (1H, m), 4.14 (1H, m), 3.37 (1H, m), 2.97-2.66 (4H, m), 2.10-2.00 (4H, m), 1.84 (1H, m), 1.77 (1H, m), 1.39 (2H, m), 1.16 (20H, m), 0.78 (3H, t). MS (+)-ES [M+H]⁺ 548 m/z.

### Example 5

### TFA N²-(L-alanyl)-N⁵-((S)-2-(((R)-2-amino-2-carboxyethyl)thio)-1-carboxyethyl)-D-glutamine

### [Ala-D-Glu-γ-mesoLANOH (A-iE-LAN)]

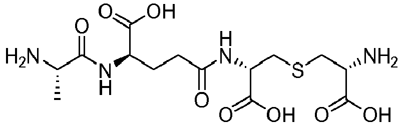

Example 5 was prepared from intermediate 22 using a similar procedure to that described in Example A, to provide 22 mg (0.042 mmol, 36% yield) of the title compound. ¹H NMR (D₂O) δ 4.07 (1H, m), 4.08 (1H, m), 3.95 (1H, q), 3.77 (1H, m), 3.05-2.76 (4H, m), 2.24 (2H, m), 2.01 (1H, m), 1.86 (1H, m), 1.02 (3H, d). MS (+)-ES [M+H]⁺ 409 m/z.

### Example 6

### TFA N²-(L-leucyl)-N⁵-((S)-2-(((R)-2-amino-2-carboxyethyl)thio)-1-carboxyethyl)-D-glutamine

### [Leu-D-Glu-γ-mesoLANOH (L-iE-LAN)]

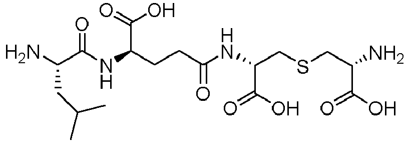

Example 6 was prepared from intermediate 31 using a similar procedure to that described in Example B, to provide 10 mg (0.014 mmol, 13% yield) of the title compound. ¹H NMR (D₂O) δ 4.15 (1H, m), 3.93 (3H, m), 3.27-2.98 (4H, m), 2.32 (2H, m), 2.10 (1H, m), 1.90 (1H, m), 1.62 (2H, m), 0.88 (6H, m). MS (+)-ES [M+H]⁺ 451 m/z.

### Example 7

### TFA N²-(L-alloisoleucyl)-N⁵-((S)-2-(((R)-2-amino-2-carboxyethyl)thio)-1-carboxyethyl)-D-glutamine

### [.Ile-D-Glu-γ-mesoLANOH (I-iE-LAN)]

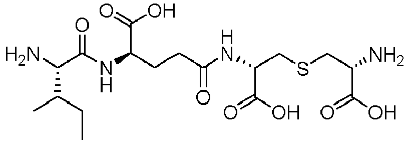

Example 7 was prepared from intermediate 32 using a similar procedure to that described in Example B, to provide 25 mg (0.036 mmol, 34% yield) of the title compound. ¹H NMR (D₂O) δ 4.35 (1H, m), 4.16 (1H, m), 3.86 (2H, m), 3.30-2.88 (4H, m), 2.33 (2H, m), 2.06 (1H, m), 1.91 (1H, m), 1.48 (1H, m), 1.25 (1H, m), 0.92 (6H, m). MS (+)-ES [M+H]⁺ 451 m/z.

### Example 8

### TFA N²-(L-valyl)-N⁵-((S)-2-(((R)-2-amino-2-carboxyethyl)thio)-1-carboxyethyl)-D-glutamine

### [Val-D-Glu -γ-mesoLANOH -(V-iE-LAN)]

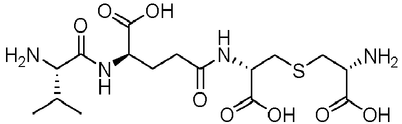

Example 8 was prepared from intermediate 33 using a similar procedure to that described in Example B, to provide 55 mg (0.082 mmol, 48% yield) of the title compound. ¹H NMR (D₂O) δ 4.32 (1H, m), 4.15 (1H, m), 3.84 (1H, m), 3.72 (1H, m), 3.08-2.83 (4H, m), 2.33 (2H, m), 2.13 (1H, m), 2.05 (1H, m), 1.48 (1H, m), 0.97 (6H, d). MS (+)-ES [M+H]⁺ 437 m/z.

### Example 9

### TFA N²-(L-phenylalanyl)-N⁵-((S)-2-(((R)-2-amino-2-carboxyethyl)thio)-1-carboxyethyl)-D-glutamine

### [Phe-D-Glu-γ-mesoLANOH (F-iE-LAN)]

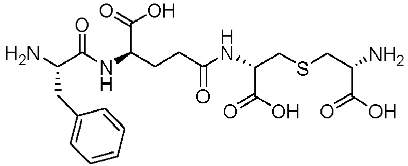

Example 9 was prepared from intermediate 34 using a similar procedure to that described in Example B, to provide 58 mg (0.082 mmol, 44% yield) of the title compound. ¹H NMR (D₂O) δ 7.35 (3H, m), 7.23 (2H, m), 4.30 (1H, m), 4.14 (1H, m), 3.97 (1H, m), 3.84 (1H, m), 3.14-2.84 (4H, m), 1.82 (2H, m), 1.61 (2H, m). MS (+)-ES [M+H]⁺ 485 m/z.

### Example 10

### N⁵-((S)-2-(((R)-2-amino-2-carboxyethyl)thio)-1-carboxyethyl)-N²-(tetradecanoyl-L-leucyl)-D-glutamine

### [C₁₄Leu-D-Glu-γ-mesoLANOH (C₁₄-L-iE-LAN)]

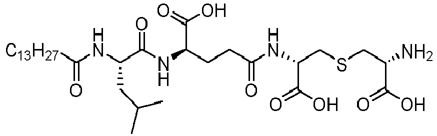

Example 10 was prepared from intermediate 47 using a similar procedure to that described in Example B, to provide 42 mg (0.055 mmol, 46% yield) of the title compound. ¹H NMR (D₂O) δ 4.32 (2H, m), 4.14 (1H, m), 3.90 (1H, m), 3.28-2.85 (4H, m), 2.27 (4H, m), 2.03 (1H, m), 1.90 (1H, m), 1.62 (4H, m), 1.16 (20H, m), 0.87 (9H, m). MS (+)-ES [M+H]⁺ 661 m/z.

### Example 11

### N⁵-((S)-2-(((R)-2-amino-2-carboxyethyl)thio)-1-carboxyethyl)-N²-(tetradecanoyl-L-alloisoleucyl)-D-glutamine

### [C₁₄-Ile-D-Glu-γ-mesoLANOH (C₁₄-I-iE-LAN)]

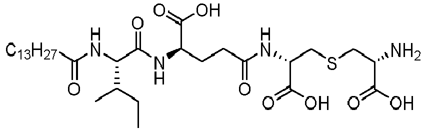

Example 11 was prepared from intermediate 48 using a similar procedure to that described in Example B, to provide 82 mg (0.106 mmol, 89% yield) of the title compound. ¹H NMR (D₂O) δ 4.32 (1H, m), 4.25 (1H, m), 4.15 (1H, m), 3.87 (1H, m), 3.13-2.85 (4H, m), 2.29 (4H, m), 1.93 (2H, m), 1.54 (2H, m), 1.41 (2H, m), 1.16 (22H, m), 0.81 (9H, m). MS (+)-ES [M+H]⁺ 659 m/z.

### Example 12

### TFA N⁵-((S)-2-(((R)-2-amino-2-carboxyethyl)thio)-1-carboxyethyl)-N²-(tetradecanoyl-L-valyl)-D-glutamine

### [C₁₄-Val-D-Glu-γ-mesoLANOH (C₁₄-V-iE-LAN)]

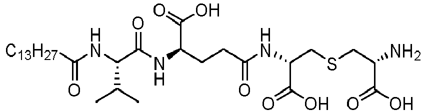

Example 12 was prepared from intermediate 49 using a similar procedure to that described in Example B, to provide 58 mg (0.077 mmol, 64% yield) of the title compound. ¹H NMR (D₂O) δ 4.32 (1H, m), 4.14 (2H, m), 3.85 (1H, m), 3.12-2.86 (4H, m), 2.35 (4H, m), 2.07 (2H, m), 1.94 (1H, m), 1.57 (3H, m), 1.16 (20H, m), 0.97 (6H, m), 0.76 (3H, t). MS (+)-ES [M+H]⁺ 647 m/z.

### Example 13

### TFA N⁵-((S)-2-(((R)-2-amino-2-carboxyethyl)thio)-1-carboxyethyl)-N²-(tetradecanoyl-L-phenylalanyl)-D-glutamine

### [C₁₄-Phe-D-Glu-γ-mesoLANOH (C₁₄-F-iE-LAN)]

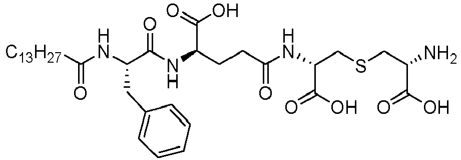

Example 13 was prepared from intermediate 50 using a similar procedure to that described in Example B, to provide 49 mg (0.061 mmol, 54% yield) of the title compound¹H NMR (MeOD-*d₁*) δ 7.25 (1H, m), 4.74 (1H, m), 4.47 (2H, m), 4.32 (1H, m), 3.77 (1H, m), 3.30-2.88 (4H, m), 2.20 (4H, m), 2.07 (1H, m), 2.00 (1H, m), 1.48 (2H, m), 1.16 (22H, m), 0.90 (3H, t). MS (+)-ES [M+H]⁺ 695 m/z.

### Example 14

### TFA.N⁵-((S)-3-(((R)-2-amino-2-carboxyethyl)thio)-1-(((R)-1-carboxyethyl)amino)-1-oxopropan-2-yl)-D-glutamine

### [.D-Glu-γ-mesoLAN-D-AlaOH (iE-LAN-D-A)]

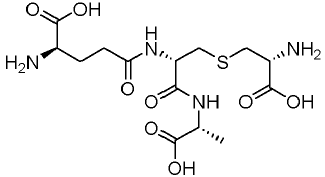

Example 14 was prepared from intermediate 64 using a similar procedure to that described in Example A, to provide 55 mg (0.13 mmol, 62% yield) of the title compound. ¹H NMR (D₂O) δ 4.22 (1H, m), 3.89 (1H, m), 3.74 (1H, m), 3.26 (1H, m), 3.09-2.83 (4H, m), 2.83 (2H, m), 2.43 (2H, m), 1.32 (3H, d). MS (+)-ES [M+H]⁺ 409 m/z.

### Example 15

### TFA.N⁵-((S)-3-(((R)-2-amino-2-carboxyethyl)thio)-1-(((R)-1-carboxyethyl)amino)-1-oxopropan-2-yl)-N²-heptanoyl-D-glutamine

### [C₇-D-Glu-γ-mesoLAN-D-AlaOH (C₇-iE-LAN-D-A)]

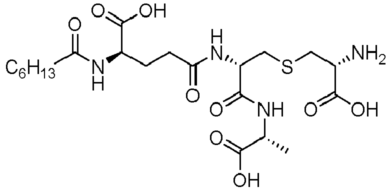

Example 15 was prepared from intermediate 65 using a similar procedure to that described in Example A, to provide 47 mg (0.091 mmol, 50% yield) of the title compound. ¹H NMR (DMSO-*d₆*) 8.32 (2H, m), 8.16 (2H, m), 8.06 (1H, d), 4.52 (1H, m), 4.20 (3H, m), 3.17-2.67 (4H, m), 2.25 (2H, m), 2.10 (2H, t), 1.99 (1H, m), 1.75 (1H, m), 1.46 (2H, m), 1.26 (8H, m), 0.86 (3H, t). MS (+)-ES [M+H]⁺ 521 m/z.

### Example 16

### TFA N⁵-((S)-3-(((R)-2-amino-2-carboxyethyl)thio)-1-(((R)-1-carboxyethyl)amino)-1-oxopropan-2-yl)-N²-dodecanoyl-D-glutamine

### [C₁₂-D-Glu-γ-mesoLAN-D-AlaOH (C₁₂-iE-LAN-D-A)]

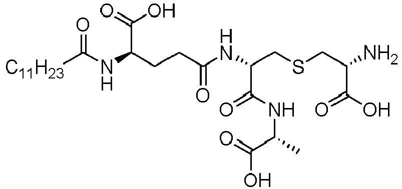

Example 16 was prepared from intermediate 66 using a similar procedure to that described in Example A, to provide 29 mg (0.049 mmol, 65% yield) of the title compound. ¹H NMR (DMSO-*d₆*) 8.29 (2H, m), 8.14 (2H, m), 8.03 (1H, d), 4.19 (1H, m), 4.01 (3H, m), 3.87-2.73 (4H, m), 2.13 (2H, m), 2.04 (2H, m), 1.89 (1H, m), 1.72 (1H, m), 1.41 (2H, m), 1.16 (16H, m), 0.78 (3H, t). MS (+)-ES [M+H]⁺ 591 m/z.

### Example 17

### TFA N⁵-((S)-3-(((R)-2-amino-2-carboxyethyl)thio)-1-(((R)-1-carboxyethyl)amino)-1-oxopropan-2-yl)-N²-tetradecanoyl-D-glutamine

### [C₁₄-D-Glu-γ-mesoLAN-D-AlaOH (C₁₄-iE-LAN-D-A)]

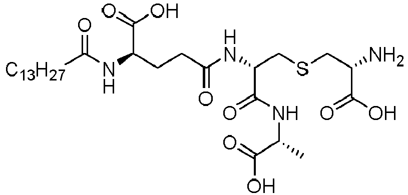

Example 17 was prepared from intermediate 67 using a similar procedure to that described in Example A, to provide 68 mg (0.11 mmol, 58% yield) of the title compound. ¹H NMR (D₂O) δ 4.52 (1H, m), 4.08 (2H, m), 3.77 (1H, m), 3.10-2.86 (4H, m), 2.31 (2H, t), 2.20 (2H, t), 2.14 (1H, m), 1.79 (1H, m), 1.51 (2H, m), 1.18 (20H, m), 0.79 (3H, t). MS (+)-ES [M+H]⁺ 619 m/z.

### Example 18

### TFA N²-(L-alanyl)-N⁵-((S)-3-(((R)-2-amino-2-carboxyethyl)thio)-1-(((R)-1-carboxyethyl)amino)-1-oxopropan-2-yl)-D-glutamine

### [Ala-D-Glu-γ-mesoLAN-D-AlaOH (A-iE-LAN-D-A)]

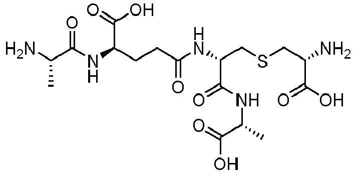

Example 18 was prepared from intermediate 69 using a similar procedure to that described in Example A, to provide 84 mg (0.17 mmol, 87% yield) of the title compound. ¹H NMR (D₂O) δ 4.54 (1H, m), 4.32 (2H, m), 4.18 (1H, m), 4.05 (1H, m), 3.17-2.66 (4H, m), 2.38 (2H, m), 2.16 (1H, m), 1.97 (1H, m), 1.48 (3H, d), 1.35 (3H, d). MS (+)-ES [M+H]⁺ 480 m/z.

### Example 19

### TFA N⁵-((S)-3-(((R)-2-amino-2-carboxyethyl)thio)-1-(((R)-1-carboxyethyl)amino)-1-oxopropan-2-yl)-N²-(heptanoyl-L-alanyl)-D-glutamine

### [C₇-Ala-D-Glu-γ-mesoLAN-D-AlaOH (C₇-A-iE-LAN-D-A)]

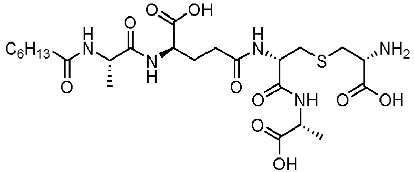

Example 19 was prepared from intermediate 72 using a similar procedure to that described in Example B, to provide 46 mg (0.077 mmol, 38% yield) of the title compound. ¹H NMR (DMSO-*d₆*) 4.27 (2H, m), 3.90 (2H, m), 3.45 (1H, m), 3.02-2.85 (4H, m), 2.14 (3H, m), 1.90 (1H, t), 1.48 (1H, m), 1.18 (12H, m), 0.85 (3H, t). MS (+)-ES [M+H]⁺ 592 m/z.

### Example 20

### TFA N⁵-((S)-3-(((R)-2-amino-2-carboxyethyl)thio)-1-(((R)-1-carboxyethyl)amino)-1-oxopropan-2-yl)-N²-(dodecanoyl-L-alanyl)-D-glutamine

### [C₁₂-Ala-D-Glu-γ-mesoLAN-D-AlaOH (C₁₂-A-iE-LAN-D-A)]

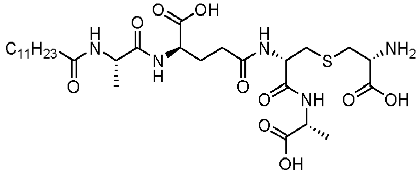

Example 120 was prepared from intermediate 73 using a similar procedure to that described in Example A, to provide 45 mg (0.068 mmol, 59% yield) of the title compound. ¹H NMR (DMSO-*d₆*) δ 8.52 (1H, m), 8.37 (1H, m), 8.25 (1H, m), 8.17 (1H, m), 8.00 (1H, m), 6.95 (1H, m), 4.55 (2H, m), 4.30 (1H, m), 4.22 (2H, m), 3.13-2.78 (4H, m), 2.25 (2H, m), 2.15 (2H, m), 1.99 (1H, m), 1.82 (1H, m), 1.47 (2H, m), 1.29-1.19 (22H, m), 0.85 (3H, t). MS (+)-ES [M+H]⁺ 662 m/z.

### Example 21

**TFA.*N⁵*-((*S*)-3-(((*R*)-2-amino-2-carboxyethyl)thio)-1-(((*R*)-1-carboxyethyl)amino)-1-oxopropan-2-yl)-*N²*-(tetradecanoyl-*L*-alanyl)-*D-*glutamine**

### [C₁₄-Ala-D-Glu-γ-mesoLAN-D-AlaOH (C₁₄-A-iE-LAN-D-A)]

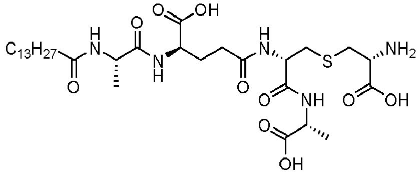

Example 21 was prepared from intermediate 74 using a similar procedure to that described in Example A, to provide 68 mg (0.11 mmol, 58% yield) of the title compound. ¹H NMR (DMSO-*d₆*) 8.55 (1H, m), 8.36 (1H, m), 8.23 (1H, m), 8.15 (1H, m), 7.98 (1H, m), 6.98 (1H, m), 4.58 (2H, m), 4.35 (1H, m), 4.20 (2H, m), 3.12-2.78 (4H, m), 2.20 (2H, m), 2.10 (2H, m), 1.99 (1H, m), 1.81 (1H, m), 1.46 (2H, m), 1.29-1.19 (26H, m), 0.88 (3H, t). MS (+)-ES [M+H]⁺ 690 m/z.

### Example 22

### TFA N⁵-((S)-3-(((R)-2-amino-2-carboxyethyl)thio)-1-((carboxymethyl)amino)-1-oxopropan-2-yl)-D-glutamine

### [D-Glu-γ-mesoLAN-GlyOH (iE-LAN-G)]

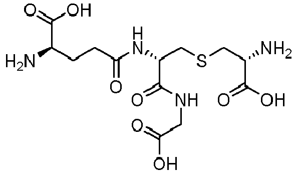

Example 22 was prepared from intermediate 92 using a similar procedure to that described in Example B, to provide 28 mg (0.07 mmol, 36% yield) of the title compound. ¹H NMR (DMSO-*d₆*) δ 4.06 (1H, m), 3.89 (1H, m), 3.74 (3H, m), 3.17-2.89 (4H, m), 2.45 (2H, m), 2.33 (1H, m), 2.09 (3H, m). MS (+)-ES [M+H]⁺ 395 m/z.

### Example 23

### TFA N⁵-((S)-3-(((R)-2-amino-2-carboxyethyl)thio)-1-((carboxymethyl)amino)-1-oxopropan-2-yl)-N²-heptanoyl-D-glutamine

### [C₇-D-Glu-γ-mesoLAN-GlyOH (C₇-iE-LAN-G)]

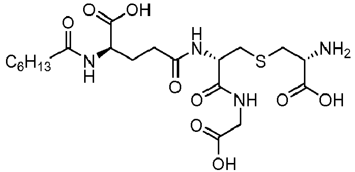

Example 23 was prepared from intermediate 93 using a similar procedure to that described in Example B, to provide 54 mg (0.11 mmol, 52% yield) of the title compound. ¹H NMR (DMSO-*d₆*) δ 4.07 (2H, m), 3.44 (3H, m), 3.17-2.83 (4H, m), 2.12 (3H, m), 1.98 (1H, m), 1.45 (2H, m), 1.26 (8H, m), 0.87 (3H, t). MS (+)-ES [M+H]⁺ 507 m/z.

### Example 24

### TFA.N⁵-((S)-3-(((R)-2-amino-2-carboxyethyl)thio)-1-((carboxymethyl)amino)-1-oxopropan-2-yl)-N²-dodecanoyl-D-glutamine

### [C₁₂-D-Glu-γ-mesoLAN-GlyOH (C₁₂-iE-LAN-G)]

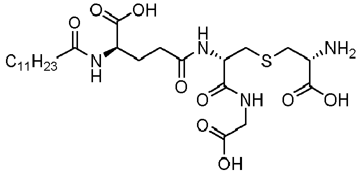

Example 24 was prepared from intermediate 94 using a similar procedure to that described in Example B, to provide 67 mg (0.12 mmol, 45% yield) of the title compound. ¹H NMR (DMSO-*d₆*) δ 4.07 (2H, m), 3.44 (3H, m), 3.02-2.82 (4H, m), 2.12 (3H, m), 1.86 (1H, m), 1.46 (2H, m), 1.26 (16H, m), 0.87 (3H, t). MS (+)-ES [M+H]⁺ 577 m/z.

### Example 25

### TFA.N⁵-((S)-3-(((R)-2-amino-2-carboxyethyl)thio)-1-((carboxymethyl)amino)-1-oxopropan-2-yl)-N²-tetradecanoyl-D-glutamine

### [C₁₄-D-Glu-γ-mesoLAN-GlyOH (C₁₄-iE-LAN-G)]

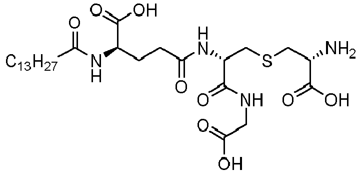

Example 25 was prepared from intermediate 95 using a similar procedure to that described in Example B, to provide 46 mg (0.07 mmol, 39% yield) of the title compound.. ¹H NMR (DMSO-*d₆*) δ 4.02 (2H, m), 3.47 (3H, m), 2.89-2.53 (4H, m), 2.12 (3H, m), 1.85 (1H, m), 1.48 (2H, m), 1.26 (20H, m), 0.83 (3H, t). MS (+)-ES [M+H]⁺ 605 m/z.

### Example 26

### TFA.N²-(L-alanyl)-N⁵-((S)-3-(((R)-2-amino-2-carboxyethyl)thio)-1-((carboxymethyl)amino)-1-oxopropan-2-yl)-D-glutamine

### [Ala-D-Glu-γ-mesoLAN-GlyOH (A-iE-LAN-G)]

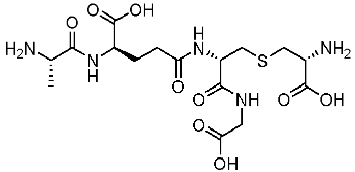

Example 26 was prepared from intermediate 99 using a similar procedure to that described in Example A, to provide 55 mg (0.09 mmol, 99% yield) of the title compound. ¹H NMR (DMSO-*d₆*) δ 4.41 (1H, m), 4.17 (1H, m), 3.82 (2H, m), 3.65 (2H, m), 3.02-2.82 (4H, m), 2.15 (2H, m), 2.01 (1H, m), 1.95 (1H, m), 1.29 (3H, d). MS (+)-ES [M+H]⁺ 466 m/z.

### Example 27

### TFA.N⁵-((S)-3-(((R)-2-amino-2-carboxyethyl)thio)-1-((carboxymethyl)amino)-1-oxopropan-2-yl)-N²-(heptanoyl-L-alanyl)-D-glutamine

### [C₇-Ala-D-Glu-γ-mesoLAN-GlyOH (C₇-A-iE-LAN-G)]

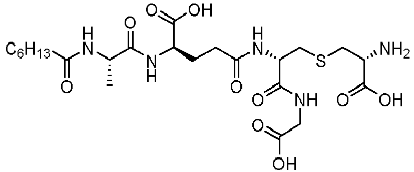

Example 27 was prepared from intermediate 100 using a similar procedure to that described in Example B, to provide 47 mg (0.08 mmol, 39% yield) of the title compound. ¹H NMR (DMSO-*d₆*) δ 4.42 (1H, m), 4.26 (1H, m), 4.07 (1H, m), 3.88 (2H, m), 3.02-2.84 (4H, m), 2.11 (3H, m), 1.88 (1H, m), 1.48 (2H, m), 1.20 (7H, m), 1.16 (3H, d), 0.85 (3H, t). MS (+)-ES [M+H]⁺ 578 m/z.

### Example 28

### TFA N⁵-((S)-3-(((R)-2-amino-2-carboxyethyl)thio)-1-((carboxymethyl)amino)-1-oxopropan-2-yl)-N²-(dodecanoyl-L-alanyl)-D-glutamine

### [C₁₂-Ala-D-Glu-γ-mesoLAN-GlyOH (C₁₂-A-iE-LAN-G)]

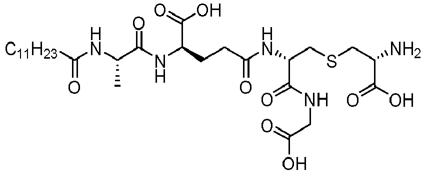

Example 28 was prepared from intermediate 101 using a similar procedure to that described in Example A, to provide 38 mg (0.05 mmol, 80% yield) of the title compound. ¹H NMR (DMSO-*d₆*) δ 4.42 (1H, m), 4.24 (1H, m), 4.07 (1H, m), 3.62 (2H, m), 2.91-2.66 (4H, m), 2.19 (2H, m), 2.06 (2H, m), 1.94 (1H, m), 1.73 (1H, m), 1.39 (3H, m) 1.20 (18H, m), 0.79 (3H, t). MS (+)-ES [M+H]⁺ 648 m/z.

### Example 29

### TFA N⁵-((S)-3-(((R)-2-amino-2-carboxyethyl)thio)-1-((carboxymethyl)amino)-1-oxopropan-2-yl)-N²-(tetradecanoyl-L-alanyl)-D-glutamine

### [C₁₄-Ala-D-Glu-γ-mesoLAN-GlyOH (C₁₄-A-iE-LAN-G)]

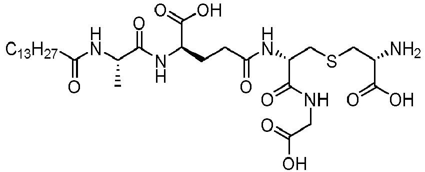

Example 29 was prepared from intermediate 102 using a similar procedure to that described in Example A, to provide 28 mg (0.05 mmol, 65% yield) of the title compound. ¹H NMR (DMSO-*d₆*) δ 4.39 (1H, m), 4.18 (1H, m), 4.10 (1H, m), 3.69 (2H, m), 2.79-2.50 (4H, m), 2.21 (2H, m), 2.06 (2H, m), 1.92 (1H, m), 1.78 (1H, m), 1.31 (2H, m) 1.20 (20H, m), 0.74 (3H, t). MS (+)-ES [M+H]⁺ 676 m/z.

### Example 30

### TFA N⁵-((S)-3-(((R)-2-amino-2-carboxyethyl)thio)-1-((carboxymethyl)amino)-1-oxopropan-2-yl)-N²-(((R)-2-hydroxypropanoyl)-L-alanyl)-D-glutamine

### [Lac-Ala-D-Glu-γ-mesoLAN-GlyOH (Lac-A-iE-LAN-Gly)]

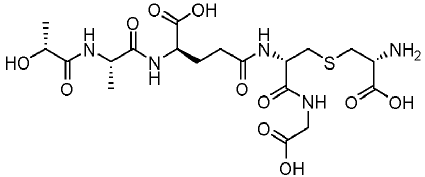

Example 30 was prepared from intermediate 109 using a similar procedure to that described in Example B, to provide 48 mg (0.08 mmol, 81% yield) of the title compound. ¹H NMR (DMSO-*d₆*) δ 8.38-8.19 (4H, m), 7.63 (1H, d), 4.52 (1H, m), 4.38 (1H, m), 4.16 (2H, m), 3.98 (1H, m), 3.77 (2H, d), 3.16-2.96 (4H, m), 2.24 (2H, m), 2.01 (1H, m), 1.77 (1H, m), 1.22 (6H, t). MS (+)-ES [M+H]⁺ 538 m/z.

### Example 31

### TFA N²-(((R)-2-(((2S,3R,4R,5S,6R)-3-acetamido-2-(benzyloxy)-5-hydroxy-6-(hydroxymethyl)tetrahydro-2H-pyran-4-yl)oxy)propanoyl)-L-alanyl)-N⁵-((S)-2-(((R)-2-amino-2-carboxyethyl)thio)-1-carboxyethyl)-D-glutamine

### [1-OBn-MurNAc-Ala-D-Glu-γ-mesoLANOH (BnMurNAc-A-iE-LAN)]

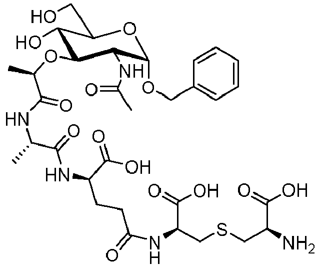

Example 31 was prepared from intermediate 110 using a similar procedure to that described in Example B, to provide 49 mg (0.06 mmol, 71% yield) of the title compound. ¹H NMR (DMSO-*d₆*) δ 8.38-8.11 (4H, m), 7.73 (1H, d), 7.35 (5H, m), 4.78 (2H, m), 4.45-4.17 (4H, m), 3.78 (1H, m), 3.63 (2H, m), 3.18-2.72 (4H, m), 2.27 (2H, m), 1.83 (1H, m), 1.78 (3H, s), 1.77 (1H, m), 1.21 (6H, m). MS (+)-ES [M+H]+ 774 m/z.

### Example 32

### TFA N²-(((R)-2-(((2S,3R,4R,5S,6R)-3-acetamido-2-(benzyloxy)-5-hydroxy-6-(hydroxymethyl)tetrahydro-2H-pyran-4-yl)oxy)propanoyl)-L-alanyl)-N⁵-((S)-3-(((R)-2-amino-2-carboxyethyl)thio)-1-((carboxymethyl)amino)-1-oxopropan-2-yl)-D-glutamine

### [1-OBn-MurNAc-Ala-D-Glu-γ-mesoLAN-GlyOH (BnMurNAc-A-iE-LAN-G)]

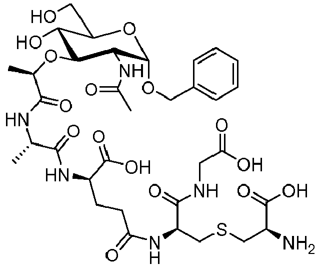

Example 32 was prepared from intermediate 111 using a similar procedure to that described in Example B, to provide 45 mg (0.05 mmol, 81% yield) of the title compound.. ¹H NMR (DMSO-*d₆*) δ 8.35-8.14 (4H, m), 7.63 (1H, d), 7.35 (5H, m), 4.81-4.10 (8H, m), 3.78 (3H, m), 3.12-2.72 (4H, m), 2.26 (2H, m), 2.08 (1H, m), 1.78 (3H, s), 1.77 (1H, m), 1.23 (6H, m). MS (+)-ES [M+H]⁺ 831 m/z.

### Example 33

### TFA N₂-(((R)-2-(((2S,3R,4R,5S,6R)-3-acetamido-2-(benzyloxy)-5-hydroxy-6-(hydroxymethyl)tetrahydro-2H-pyran-4-yl)oxy)propanoyl)-L-alanyl)-N⁵-((S)-3-(((R)-2-amino-2-carboxyethyl)thio)-1-(((R)-1-carboxyethyl)amino)-1-oxopropan-2-yl)-D-glutamine

### [1-OBn-MurNAc-Ala-D-Glu-γ-mesoLAN-D-AlaOH (BnMurNAc-A-iE-LAN-D-Ala)]

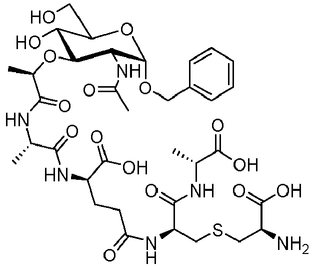

Example 33 was prepared from intermediate 114 using a similar procedure to that described in Example B, to provide 36 mg (0.04 mmol, 48% yield) of the title compound.. ¹H NMR (DMSO-*d₆*) δ 8.30-7.80 (5H, m), 7.36 (5H, m), 4.78-4.67 (2H, m), 4.42-4.27 (6H, m), 3.80 (3H, m), 3.12-2.72 (4H, m), 2.15 (2H, m), 2.00 (1H, m), 1.78 (3H, s), 1.77 (1H, m), 1.23 (9H, m). MS (+)-ES [M+H]⁺ 845 m/z.

### Example 34

### N⁵-((S)-2-((R)-2-amino-2-carboxyethoxy)-1-carboxyethyl)-D-glutamine

### [H-D-Glu-γ-mesoOxaDAP.NH₄⁺ (iE-OxaDAP)]

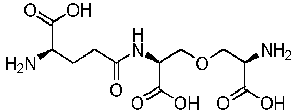

Example 34 was prepared from intermediate 123 using a similar procedure to that described in Example C, to provide 45 mg (0.05 mmol, 81% yield) of the title compound. ¹H NMR (D₂O) δ 4.27 (1H, m), 3.27 (1H, m), 3.55 (1H, m), 3.03-2.77 (4H, m), 2.30 (2H, m), 2.01 (1H, m), 1.98 (1H, m). MS (+)-ES [M+H]⁺ 322 m/z.

### Example 35

### N⁵-((S)-2-((R)-2-amino-2-carboxyethoxy)-1-carboxyethyl)-N²-tetradecanoyl-D-glutamine

### [C₁₄-D-Glu-γ-mesoOxaDAP.NH₄⁺ (C₁₄-iE-OxaDAP)]

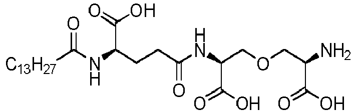

Example 34 was prepared from intermediate 127 using a similar procedure to that described in Example C, to provide 45 mg (0.05 mmol, 81% yield) of the title compound. ¹H NMR (DMSO-*d₆*) δ 4.55 (1H, m), 4.24 (1H, m), 3.77 (3H, m), 2.97-2.66 (4H, m), 2.20 (2H, m), 2.11 (2H, m), 1.98 (1H, m), 1.77 (1H, m), 1.50 (2H, m), 1.24 (20H, m), 0.84 (3H, t). MS (+)-ES [M+H]⁺ 532 m/z.

### Example 36

### N²-(L-alanyl)-N⁵-((S)-2-((R)-2-amino-2-carboxyethoxy)-1-carboxyethyl)-D-glutamine

### [H-Ala-D-Glu-γ-mesoOxaDAP.NH₄⁺ (A-iE-OxaDAP)]

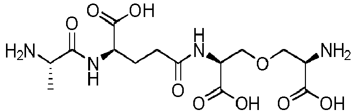

Example 36 was prepared from intermediate 130 using a similar procedure to that described in Example C, to provide 30 mg (0.076 mmol, 42% yield) of the title compound. ¹H NMR (D₂O) δ 4.55 (1H, m), 4.22 (2H, m), 4.17 (1H, m), 4.10 (1H, m), 3.17-2.86 (4H, m), 2.28 (2H, m), 2.10 (1H, m), 1.98 (1H, m), 1.46 (3H, d), 1.34 (3H, d). MS (+)-ES [M+H]⁺ 393 m/z.

### Example 37

### N⁵-((S)-2-((R)-2-amino-2-carboxyethoxy)-1-carboxyethyl)-N²-(tetradecanoyl-L-alanyl)-D-glutamine

### [C₁₄-Ala-D-Glu-γ-mesoOxaDAP.NH₄⁺ (C₁₄-A-iE-OxaDAP)]

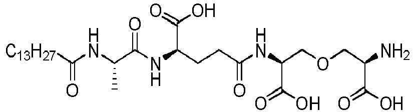

Example 37 was prepared from intermediate 134 using a similar procedure to that described in Example C, to provide 35 mg (0.06 mmol, 69% yield) of the title compound. ¹H NMR (D₂O) δ 8.28-8.00 (3H, m), 4.41 (2H, m), 3.90-3.55 (5H, m), 2.20 (2H, m), 2.11 (2H, m), 2.03 (1H, m), 1.96 (1H, m), 1.46 (2H, m), 1.21 (20H, m), 0.86 (3H, t). MS (+)-ES [M+H]⁺ 661 m/z.

### BIOLOGICAL DATA

The biological activities of exemplified compounds of formula (i) were assessed in the following assays:

### NOD1 functional agonist assay protocol

HEK293 cells were stably transfected with plasmid for the overexpression of the NOD1 genes and a reporter plasmid expressing a secreted embryonic alkaline phosphatase (SEAP) gene under the control of a promoter inducible by the transcription factors NF-κB and AP-1. These cells are referred herein as 293hNOD1-SEAP (for cells overexpressing the human NOD1 gene) and 293mNOD1-SEAP (for cells overexpressing the murine NOD1 gene). Upon NOD1 stimulation, these cells induce the activiation of NF-κB and AP-1 and subsequently the secretion of SEAP. The reporter protein is easily detectable and measurable when using QUANTI-Blue™ (InvivoGen, San Diego, CA), a medium that turns purple/blue in the presence of SEAP.

The biological activity of the compounds of the invention tested using 293hNOD1 and 293mNOD1 cells. SEAP expression was measured using the commercially available SEAP detection kit (HEK-Blue™ Detection, InvivoGen). The SEAP activity in cells was quantified using the microplate reader (FLUOStar OPTIMA, BMG laboratories), the absorbance values were read using filter with excitation at 620 nm and emission at 655 nm. The results provided in Table 2 were expressed as the concentration were a significant mean optical density (OD) can be observed (above 3*O.D of negative control). As a positive control, the NOD1 pathway was stimulated with commercially available iE-DAP (InvivoGen) and C₁₂-iE-DAP (an acylated derivative of DAP; InvivoGen). As a negative control endotoxin-free water was used. Additionally, 293 cells expressing the SEAP gene but not the NOD1 gene were used as a control.

**Table 2**

| **Examples** | **mNod1 (µg/mL)** | **hNod1 (µg/mL)** |
|---|---|---|
| **1** | 10 | 1 |
| **2** | 1 | 0.1 |
| **3** | 0.1 | 0.01 |
| **4** | 0.001 | 0.0001 |
| **5** | 1 | 1 |
| **6** | 1 | 0.1 |
| **7** | 1 | 0.1 |
| **8** | 1 | 0.1 |
| **9** | 10 | 10 |
| **10** | 0.1 | 0.001 |
| **11** | 0.1 | 0.001 |
| **12** | 0.1 | 0.001 |
| **13** | 1 | 0.001 |
| **14** | 10 | 1 |
| **15** | 0.01 | 0.01 |
| **16** | 0.001 | 0.01 |
| **17** | 0.001 | 0.01 |
| **18** | 1 | 1 |
| **19** | 0.1 | 0.1 |
| **20** | 0.01 | 0.01 |
| **21** | 0.001 | 0.01 |
| **22** | 10 | 1 |
| **23** | 1 | 1 |
| **24** | 0.01 | 0.01 |
| **25** | 0.01 | 0.01 |
| **26** | 0.1 | 10 |
| **27** | 1 | 1 |
| **28** | 0.001 | 0.001 |
| **29** | 0.001 | 0.0001 |
| **30** | 10 | 10 |
| **31** | 1 | 1 |
| **32** | 0.1 | 0.1 |
| **33** | 1 | 1 |
| **34** | 10 | 10 |
| **35** | 0.01 | 0.01 |
| **36** | 100 | 100 |
| **37** | 0.1 | 0.1 |

### NOD2 functional agonist assay protocol

HEK293 cells were stably transfected with plasmid for the overexpression of the NOD2 genes and a reporter plasmid expressing a secreted embryonic alkaline phosphatase (SEAP) gene under the control of a promoter inducible by the transcription factors NF-κB and AP-1. These cells are referred herein as 293hNOD2-SEAP. Upon NOD2 stimulation, these cells induce the activiation of NF-κB and AP-1 and subsequently the secretion of SEAP. The reporter protein is easily detectable and measurable when using QUANTI-Blue™ (InvivoGen, San Diego, CA), a medium that turns purple/blue in the presence of SEAP.

The biological activity of the compounds of the invtested using 293hNOD2 cells. SEAP expression was measured using the commercially available SEAP detection kit (HEK-Blue™ Detection, InvivoGen). The SEAP activity in cells was quantified using the microplate reader (FLUOStar OPTIMA, BMG laboratories), the absorbance values were read using filter with excitation at 620 nm and emission at 655 nm. The results provided in Table 3 were expressed as the concentration were a significant mean optical density (OD) can be observed (above 3*O.D of negative control) as the mean optical density. As a positive control, the NOD2 pathway was stimulated with commercially available MDP (InvivoGen) and M-triDAP (InvivoGen). As a negative control endotoxin-free water was used. Additionally, 293 cells expressing the SEAP gene but not the NOD2 gene were used as a control.

**Table 3**

| **Examples** | **hNod2 (µg/mL)** |
|---|---|
| **31** | 0.1 |
| **32** | 0.1 |
| **33** | 1 |

### PRECLINICAL INFLAMMATORY MODELS

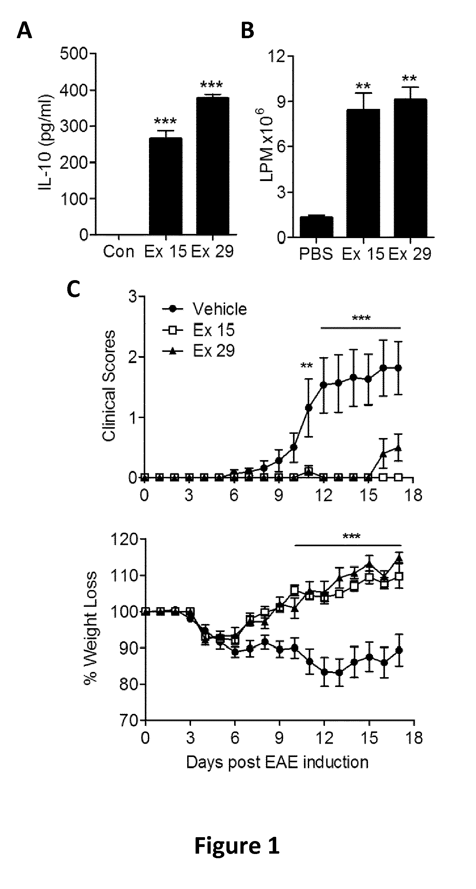

As provided in Figure 1B, compounds of Example 29(Ex.29) and of Example 15 (Ex.15) significantly increased the numbers of LPM in the peritoneal cavity when injected i.p. into mice and induced the production of significant concentrations of IL-10 by PEC *in vitro* (Figure 1A).

### 1 Experimental autoimmune encephalomyelitis (EAE) model

EAE is induced in C57BL/6 mice by immunization with an emulsion of MOG₃₅₋₅₅ or MOG₁₋₁₂₅ in complete Freund's adjuvant (CFA), followed by administration of pertussis toxin (PT) in PBS, first on the day of immunization and then again the following day.

As illustrated in Figure 1C, the treatment of mice with compounds of Example 29(Ex.29) and of Example 15 (Ex.15) significantly attenuated the clinical course of EAE and prevented associated weight loss.

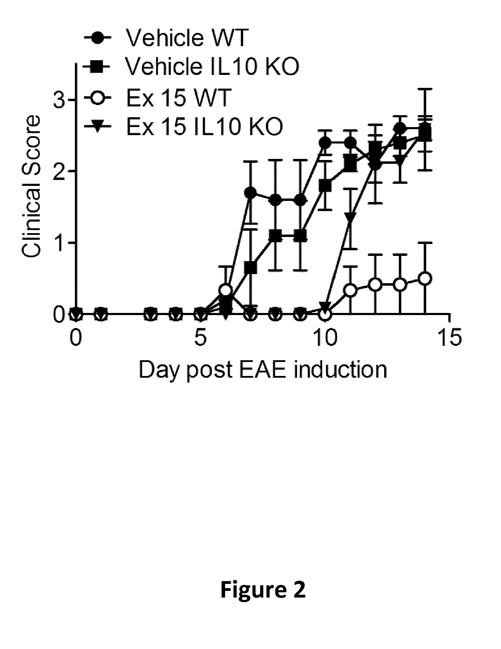

As provided in Figure 2, the treatment with compound of Example 15(Ex.15) significantly attenuated EAE in WT mice, with only one of 6 mice had any symptoms of EAE. In contrast, 100% of compound of Example 15(Ex.15) -treated IL-10^{-/-}mice developed EAE, albeit slightly later onset than untreated WT or IL-10^{-/-} mice.

### 2. Dextran sulfate sodium (DSS)-induced colitis mouse model.

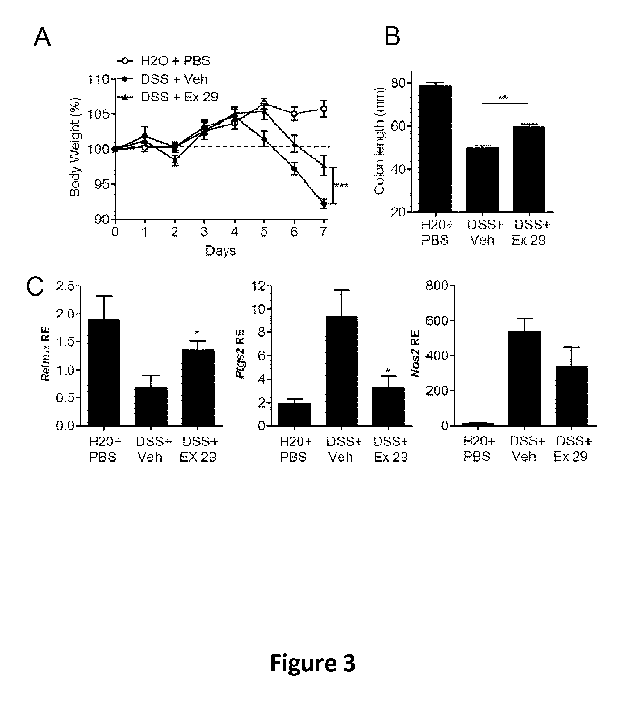

As provides in Figure 3, mice were given water or 2% DSS in their drinking water for 7 days and were treated i.p. with either PBS, vehicle or 25 µg compound of Example 29(Ex.29) on days 0, 1, 2, 4 and 6 of the experiment. (3A) Body weights were recorded daily. (3B) Mice were sacrificed on day 7 and colon lengths were measured (mean ± SE; n=6). (3C) Total mRNA were extracted from the colons on day 7 and the relative expression (RE) of the M2 macrophage markers *Relma,* and the M1 macrophage markers *Ptgs2* (Cox2) and *Nos2* were quantified by qPCR (mean ± SE; n=6). *, P<0.05; and **, P<0.01 versus DSS + vehicle treated mice.

### 3. DSS-induced inflammation in the colon model

Mice were given water or 2% DSS in their drinking water for 7 days and were treated i.p. with either PBS, vehicle or 25 µg compound of Example 29(Ex.29) on days 0, 1, 2, 4 and 6 of the experiment. Mice were sacrificed on day 7 and colon sections were prepared and stained with H&E.

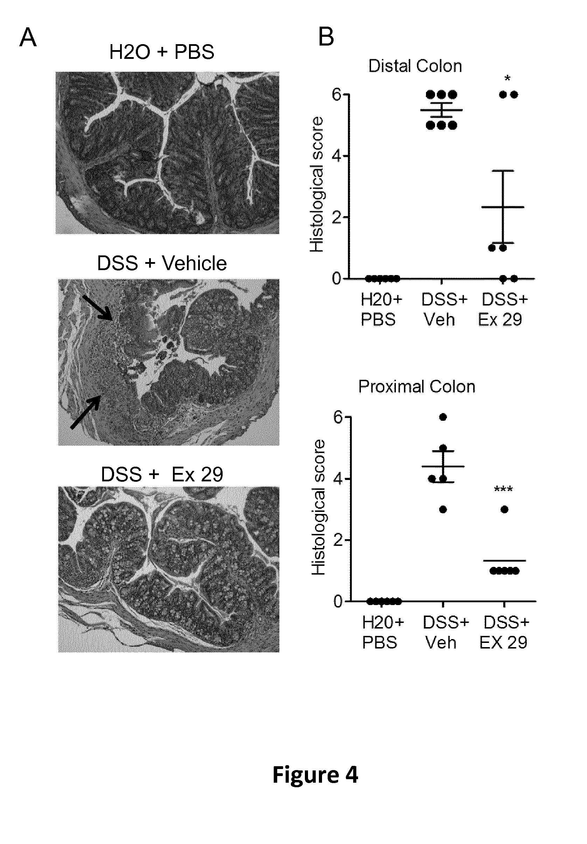

Figure 4(A) shows a representative histology from individual mice. Arrows indicate areas of severe inflammatory pathology. Figure 4(B) shows inflammatory scores for proximal and distal colon for each group of mice (mean ± SE; n=6). *, P<0.05; and ***, P<0.001 versus DSS + vehicle treated mice.

## Claims

1. A compound of formula (I) or a pharmaceutically acceptable salt thereof, 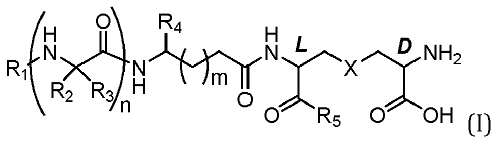 wherein
• **R₁** is hydrogen, C₁-C₂₀ alkyl, C₂-C₂₀ alkenyl or C₂-C₂₀ alkynyl each of which groups are optionally substituted by one or more of C₁-C₄ alkyamino, C₁-C₄ alkoxy or R₁ is (C₁-C₂₀ alkyl)-W-(C₂-C₂₀ alkyenyl)-, wherein W is -O-, -S-, -NH-, -C(O)- or -S(O)₂-, or R₁ is a group (II)
-Z-R₆ (II)
in which Z is -C(O)-,-C(S)- or -S(O)₂-, R₆ is C₁-C₂₀ alkyl, C₂-C₂₀ alkenyl; C₂-C₂₀ alkynyl, C₃-C₇ cycloalkyl, phenyl or benzyl each of which may be optionally substituted by one or more of halogen, C₁-C₄ alkyl or C₁-C₄ alkoxy; or R₁ is a Muramic acid derivative of formula (III): 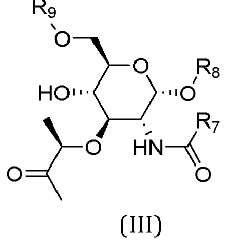 in which:
• **R₇** is C₁-C₄ alkyl optionally substituted by hydroxyl;
• **R₈** is hydrogen or benzyl group;
• **R₉** is hydrogen, C₁-C₂₀ alkyl, C₁-C₂₀ alkenyl, C₁-C₂₀ alkynyl each of which may be optionally substituted by C₁-C₄ alkyamino or C₁-C₄ alkoxy; or R₉ is a group:
-Z-R₁₀ (IV)
in which Z is is -C(O)-,-C(S)- or -S(O)₂-, **R₁₀** is a group selected from C₁-C₂₀ alkyl, C₂-C₂₀ alkenyl; C₂-C₂₀ alkynyl, C₃-C₇ cycloalkyl, phenyl or benzyl; each of which may be optionally substituted by one or more of hydroxy, halogen, C₁-C₄ alkyl or C₁-C₄ alkoxy;
• **R₂ and R₃** are hydrogen or are independently selected from hydrogen, benzyl which may be optionally substituted by hydroxy or C₁-C₆ alkyl group which may be optionally substituted by one or more of hydroxy, carboxy, amino, -C(O)NH₂, -SH, -SCH₃,-NHC(=NH)NH₂ or of a heterocyclyl group;
• **R₄** is hydrogen, hydroxyl, amino, -COOR₁₁, or -CONR₁₁R₁₂ wherein R₁₁ and R₁₂ are hydrogen or are independently selected from hydrogen, or C₁-C₂₀ alkyl optionally substituted with hydroxyl or amino group;
• **R₈** is hydroxy, amino, -O-C₁-C₂₀ alkyl, -NH-C₁-C₂₀ alkyl, optionally substituted by hydroxyl or R₅ is a group of formula (V), 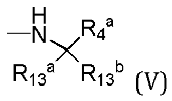 wherein
o **R₁₃^{a} and R₁₃^{b}** are hydrogen or are independently selected from hydrogen, benzyl which may be optionally substituted by hydroxy or C₁-C₆ alkyl group which may be optionally substituted by one or more of hydroxy, carboxy, amino, -C(O)NH₂, -SH, -SCH₃,-NHC(=NH)NH₂ or of a heterocyclyl group;
o **R₄^{a}** is are hydrogen, hydroxyl, amino, -COOR₁₁, or -CONR₁₁R₁₂ wherein R₁₁ and R₁₂ are hydrogen or are independently selected from hydrogen, or C₁-C₂₀ alkyl optionally substituted with hydroxyl or amino group;
• m is an integer from 1 to 3;
• n is an integer from 0 to 2;
• X is sulfur, oxygen or -NH-;
• L or D represents the absolute configuration of the chiral carbon atom;
with the proviso that when X is sulfur, m and n are 1, R₂ is hydrogen, R₃ is methyl, R₄ is -COOH or -CONH2 and R₅ is hydroxy, R₁ is not a dodecanoyl group.

2. A compound of formula (I) or a pharmaceutically acceptable salt thereof according to claim 1, wherein n is 0 and X is sulphur.

3. A compound of formula (I) or a pharmaceutically acceptable salt thereof according to claim to claim 1, wherein n is 1 and X is sulphur.

4. A compound of formula (I) or a pharmaceutically acceptable salt thereof according to claim 1, wherein X is oxigen.

5. A compound of formula (I) or a pharmaceutically acceptable salt thereof according to claim 1, wherein n is 0 and m is 1, X is sulphur and R₅ is hydroxy, a D-alanyl or a glycyl residue.

6. A compound of formula (I) or a pharmaceutically acceptable salt thereof according to claim 1, wherein n and m are 1, X is sulphur and R₅ is hydroxy, a D-alanyl or a glycyl residue.

7. A compound of formula (I) or a pharmaceutically acceptable salt thereof according to claim 1, wherein m is 1, X is oxygen and R₅ is hydroxy, a D-alanyl or a glycyl residue.

8. A compound of formula (Ia) or a pharmaceutically acceptable salt thereof according to claim 1, 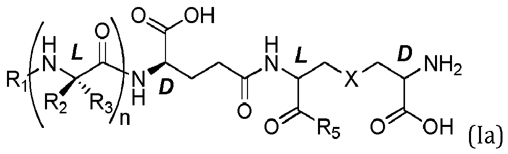 wherein
• **R₁** is hydrogen, an acyl group selected from a heptanoyl, dodecanoyl tetradecanoyl or 2-hydroxypropanoyl group or R₁ is a Muramic acid derivative of formula (III) 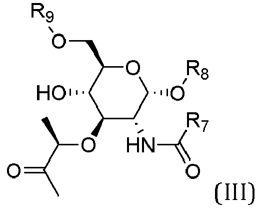 in which:
• **R₇** is C₁-C₄ alkyl;
• **R₈** is hydrogen or benzyl group;
• **R₉** is hydrogen,
• **R₂ and R₃** are independently selected from hydrogen, benzyl which may be optionally substituted by hydroxy or C₁-C₆ alkyl group which may be optionally substituted by one or more of hydroxy, carboxy, amino, -C(O)NH₂, -SH, -SCH₃,-NHC(=NH)NH₂ or of a heterocyclyl group;
• **R₈** is hydroxyl, a D-alanyl or a glycyl residue;
• **n** is an integer from 0 or 1;
• **X** is sulphur or oxygen;
• **L or D** represents the absolute configuation of the chiral center;
with the proviso that when X is sulfur, m and n are 1, R₂ is hydrogen, R₃ is methyl, and R₅ is hydroxyl, R₁ is not a dodecanoyl group.

9. A compound of formula (Ic) or a pharmaceutically acceptable salt thereof according to claim 1, 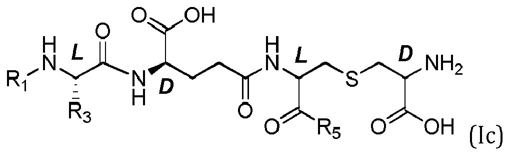 wherein
• **R₁** is hydrogen, an acyl group selected from a heptanoyl, dodecanoyl tetradecanoyl or-2-hydroxypropanoyl or R₁ is a Muramic acid derivative of formula (III): 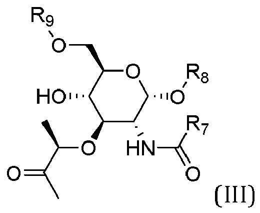 in which:
• **R₇** is C₁-C₄ alkyl;
• **R₈** is hydrogen or benzyl group;
• **R₉** is hydrogen,
• **R₃** is hydrogen, benzyl which may be optionally substituted by hydroxy or C₁-C₆ alkyl group which may be optionally substituted by one or more of hydroxy, carboxy or amino;
• **R₅** is hydroxyl, a D-alanyl or a glycyl residue;
• **L or D** represents the absolute configuation of the the chiral carbon atom;
with the proviso that when R₃ is methyl and R₅ is hydroxy, R₁ is not a dodecanoyl group.

10. A compound of formula (Id) or a pharmaceutically acceptable salt thereof according to claim 1, 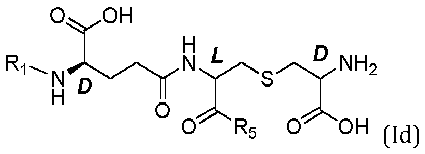 wherein
• **R₁** is hydrogen or an acyl group selected from a heptanoyl, dodecanoyl tetradecanoyl or -2-hydroxypropanoyl or R₁ is a Muramic acid derivative of formula (III): 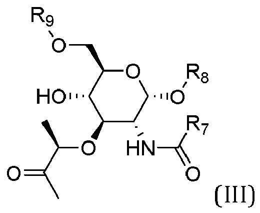 in which:
• **R₇** is C₁-C₄ alkyl;
• **R₈** is hydrogen or benzyl group;
• **R₉** is hydrogen,
• **R₃** is hydrogen, benzyl which may be optionally substituted by hydroxy or C₁-C₆ alkyl group which may be optionally substituted by one or more of hydroxy, carboxy, or amino;
• **R₅** is hydroxyl, a D-alanyl or a glycyl residue;
• **L or D** represents the absolute configuation of the the chiral carbon atom

11. A compound or a pharmaceutically acceptable salt thereof selected from:
• N5-((S)-2-(((R)-2-amino-2-carboxyethyl)thio)-1-carboxyethyl)-D-glutamine;
• N5-((S)-2-(((R)-2-amino-2-carboxyethyl)thio)-1-carboxyethyl)-N2-heptanoyl-D-glutamine;
• N5-((S)-2-(((R)-2-amino-2-carboxyethyl)thio)-1-carboxyethyl)-N2-dodecanoyl-D-glutamine;
• N5-((S)-2-(((R)-2-amino-2-carboxyethyl)thio)-1-carboxyethyl)-N2-tetradecanoyl-D-glutamine;
• N2-(L-alanyl)-N5-((S)-2-(((R)-2-amino-2-carboxyethyl)thio)-1-carboxyethyl)-D-glutamine;
• N2-(L-leucyl)-N5-((S)-2-(((R)-2-amino-2-carboxyethyl)thio)-1-carboxyethyl)-D-glutamine;
• N2-(L-alloisoleucyl)-N5-((S)-2-(((R)-2-amino-2-carboxyethyl)thio)-1-carboxyethyl)-D-glutamine;
• N2-(L-valyl)-N5-((S)-2-(((R)-2-amino-2-carboxyethyl)thio)-1-carboxyethyl)-D-glutamine;
• N2-(L-phenylalanyl)-N5-((S)-2-(((R)-2-amino-2-carboxyethyl)thio)-1-carboxyethyl)-D-glutamine;
• N5-((S)-2-(((R)-2-amino-2-carboxyethyl)thio)-1-carboxyethyl)-N2-(tetradecanoyl-L-leucyl)-D-glutamine;
• N5-((S)-2-(((R)-2-amino-2-carboxyethyl)thio)-1-carboxyethyl)-N2-(tetradecanoyl-L-alloisoleucyl)-D-glutamine;
• N5-((S)-2-(((R)-2-amino-2-carboxyethyl)thio)-1-carboxyethyl)-N2-(tetradecanoyl-L-valyl)-D-glutamine;
• N5-((S)-2-(((R)-2-amino-2-carboxyethyl)thio)-1-carboxyethyl)-N2-(tetradecanoyl-L-phenylalanyl)-D-glutamine;
• N5-((S)-3-(((R)-2-amino-2-carboxyethyl)thio)-1-(((R)-1-carboxyethyl)amino)-1-oxopropan-2-yl)-D-glutamine;
• N5-((S)-3-(((R)-2-amino-2-carboxyethyl)thio)-1-(((R)-1-carboxyethyl)amino)-1-oxopropan-2-yl)-N2-heptanoyl-D-glutamine;
• N5-((S)-3-(((R)-2-amino-2-carboxyethyl)thio)-1-(((R)-1-carboxyethyl)amino)-1-oxopropan-2-yl)-N2-dodecanoyl-D-glutamine;
• N5-((S)-3-(((R)-2-amino-2-carboxyethyl)thio)-1-(((R)-1-carboxyethyl)amino)-1-oxopropan-2-yl)-N2-tetradecanoyl-D-glutamine;
• N2-(L-alanyl)-N5-((S)-3-(((R)-2-amino-2-carboxyethyl)thio)-1-(((R)-1-carboxyethyl)amino)-1-oxopropan-2-yl)-D-glutamine;
• N5-((S)-3-(((R)-2-amino-2-carboxyethyl)thio)-1-(((R)-1-carboxyethyl)amino)-1-oxopropan-2-yl)-N2-(heptanoyl-L-alanyl)-D-glutamine;
• N5-((S)-3-(((R)-2-amino-2-carboxyethyl)thio)-1-(((R)-1-carboxyethyl)amino)-1-oxopropan-2-yl)-N2-(dodecanoyl-L-alanyl)-D-glutamine;
• N5-((S)-3-(((R)-2-amino-2-carboxyethyl)thio)-1-(((R)-1-carboxyethyl)amino)-1-oxopropan-2-yl)-N2-(tetradecanoyl-L-alanyl)-D-glutamine;
• N5-((S)-3-(((R)-2-amino-2-carboxyethyl)thio)-1-((carboxymethyl)amino)-1-oxopropan-2-yl)-D-glutamine;
• N5-((S)-3-(((R)-2-amino-2-carboxyethyl)thio)-1-((carboxymethyl)amino)-1-oxopropan-2-yl)-N2-heptanoyl-D-glutamine;
• N5-((S)-3-(((R)-2-amino-2-carboxyethyl)thio)-1-((carboxymethyl)amino)-1-oxopropan-2-yl)-N2-dodecanoyl-D-glutamine;
• N5-((S)-3-(((R)-2-amino-2-carboxyethyl)thio)-1-((carboxymethyl)amino)-1-oxopropan-2-yl)-N2-tetradecanoyl-D-glutamine;
• N2-(L-alanyl)-N5-((S)-3-(((R)-2-amino-2-carboxyethyl)thio)-1-((carboxymethyl)amino)-1-oxopropan-2-yl)-D-glutamine;
• N5-((S)-3-(((R)-2-amino-2-carboxyethyl)thio)-1-((carboxymethyl)amino)-1-oxopropan-2-yl)-N2-(heptanoyl-L-alanyl)-D-glutamine;
• N5-((S)-3-(((R)-2-amino-2-carboxyethyl)thio)-1-((carboxymethyl)amino)-1-oxopropan-2-yl)-N2-(dodecanoyl-L-alanyl)-D-glutamine;
• N5-((S)-3-(((R)-2-amino-2-carboxyethyl)thio)-1-((carboxymethyl)amino)-1-oxopropan-2-yl)-N2-(tetradecanoyl-L-alanyl)-D-glutamine;
• N5-((S)-3-(((R)-2-amino-2-carboxyethyl)thio)-1-((carboxymethyl)amino)-1-oxopropan-2-yl)-N2-(((R)-2-hydroxypropanoyl)-L-alanyl)-D-glutamine;
• N2-(((R)-2-(((2S,3R,4R,5S,6R)-3-acetamido-2-(benzyloxy)-5-hydroxy-6-(hydroxymethyl)tetrahydro-2H-pyran-4-yl)oxy)propanoyl)-L-alanyl)-N5-((S)-2-(((R)-2-amino-2-carboxyethyl)thio)-1-carboxyethyl)-D-glutamine;
• N2-(((R)-2-(((2S,3R,4R,5S,6R)-3-acetamido-2-(benzyloxy)-5-hydroxy-6-(hydroxymethyl)tetrahydro-2H-pyran-4-yl)oxy)propanoyl)-L-alanyl)-N5-((S)-3-(((R)-2-amino-2-carboxyethyl)thio)-1-((carboxymethyl)amino)-1-oxopropan-2-yl)-D-glutamine;
• N2-(((R)-2-(((2S,3R,4R,5S,6R)-3-acetamido-2-(benzyloxy)-5-hydroxy-6-(hydroxymethyl)tetrahydro-2H-pyran-4-yl)oxy)propanoyl)-L-alanyl)-N5-((S)-3-(((R)-2-amino-2-carboxyethyl)thio)-1-(((R)-1-carboxyethyl)amino)-1-oxopropan-2-yl)-D-glutamine
• N5-((S)-2-((R)-2-amino-2-carboxyethoxy)-1-carboxyethyl)-D-glutamine; N5-((S)-2-((R)-2-amino-2-carboxyethoxy)-1-carboxyethyl)-N2-tetradecanoyl-D-glutamine;
• N2-(L-alanyl)-N5-((S)-2-((R)-2-amino-2-carboxyethoxy)-1-carboxyethyl)-D-glutamine;
• N5-((S)-2-((R)-2-amino-2-carboxyethoxy)-1-carboxyethyl)-N2-(tetradecanoyl-L-alanyl)-D-glutamine; or pharmaceutically acceptable salts thereof.

12. A compound or a pharmaceutically acceptable salt thereof, as defined in any one of claims 1 to 11, for use in therapy.

13. A compound or a pharmaceutically acceptable salt thereof, as defined in any one of claims 1 to 11, for use in treatment of conditions for which agonism of NOD1 or NODland NOD2 receptors is beneficial.

14. A compound as claimed in any one of claims 1 to 11 or a pharmaceutically acceptable salt or solvate thereof for use in the treatment of patients with inflammatory and/or autoimmune diseases

15. A pharmaceutical composition comprising a) a compound defined by any one of claims 1 to 11 or a pharmaceutically acceptable salt thereof and b) one or more pharmaceutically acceptable exicipents.
